# EUROPEAN PATENT SPECIFICATION

(11) **EP 1 442 025 B1**
(45) Date of publication and mention of the grant of the patent: **07.03.2007**
(21) Application number: 02801692.1
(22) Date of filing: 15.10.2002
(51) Int. Cl.: C07D 239/91, C07D 495/04, C07D 513/04, A61K 31/519, A61P 3/04

(54) **PYRIMIDINONES AS MELANIN CONCENTRATING HORMONE RECEPTOR 1**
PYRIMIDINONE ALS MELANIN CONCENTRATING HORMONE RECEPTOR 1
PYRIMIDINONES EN TANT QUE RECEPTEUR 1 DE L'HORMONE DE CONCENTRATION DE LA MELANINE

(30) Priority: 15.10.2001 GB 0124627
(43) Date of publication of application: 04.08.2004
(73) Proprietor: SMITHKLINE BEECHAM PLC, Brentford, Middlesex TW8 9GS (GB)
(72) Inventor: CARPENTER, Andrew J., GlaxoSmithKline, Research Triangle Park, NC 27709 (US); C0OPER, Joel P., GlaxoSmithKline, Research Triangle Park, NC 27709 (US); HANDLON, Anthony, L., GlaxoSmithKline, Research Triangle Park, NC 27709 (US); HERTZOG, Donald L., GlaxoSmithKline, Research Triangle Park, NC 27709 (US); HYMAN, Clifton E., GlaxoSmithKline, Research Triangle Park, NC 27709 (US); GUO, Yu, C., GlaxoSmithKline, Research Triangle Park, NC 27709 (US); SPEAKE, Jason D., GlaxoSmithKline, Research Triangle Park, NC 27709 (US); WITTY, David Richard, GlaxoSmithKline, Harlow, Essex CM 19 5AW (GB)
(74) Representative: Learoyd, Stephanie Anne
(86) International application number: PCT/US2002/032739
(87) International publication number: WO 2003/033476

(56) References cited:
- WO-A-01/21577
- WO-A-97/41097

## Description

This invention relates to novel pyrimidinones which are antagonists at the melanin-concentrating hormone receptor 1 (MCHR1), also referred to as 11 CBy, to pharmaceutical compositions containing them, to processes for their preparation, and to their use in therapy.

Obesity is a medical condition that is reaching epidemic proportions among humans in a number of countries throughout the world. It is a condition that is also associated with or induces other diseases or conditions that disrupt life activities and lifestyles. Obesity is recognized as a serious risk factor for other diseases and conditions such as diabetes, hypertension, and arteriosclerosis. It is also known that increased body weight due to obesity can place a burden on joints, such as knee joints, causing arthritis, pain, and stiffness.

Because overeating and obesity have become such a problem in the general population, many individuals are now interested in losing weight, reducing weight, and/or maintaining a healthy body weight and desirable lifestyle.

WO01/21577 (Takeda) relates to a compound of the formula 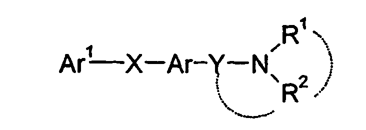 wherein Ar¹ is a cyclic group which may have substituents, X is a spacer having a main chain of 1 to 6 atoms, Y is a bond or a spacer having a main chain of 1 to 6 atoms, Ar is a monocyclic aromatic ring which may be condensed with a 4 to 8 membered non-aromatic ring, and may have further substituents; R¹ and R² are independently hydrogen or a hydrocarbon group which may have substituents; R¹ and R² together with the adjacent nitrogen atom may form a nitrogen containing hetero ring which may have substituents; R² may form a spiro ring together with Ar; or R² together with the adjacent nitrogen atom may form a nitrogen containing hetero ring which may have substituents; or a salt thereof; and which compounds are antagonists of a melanin-concentrating hormone. Such compounds are suggested as being useful for preventing or treating obesity.

In particular, it is known that melanin-concentrating hormone ("MCH") originates in the hypothalamus and has orexigenic action (see Nature, Vol. 396, p. 670, (1998), for example). There is an on-going need for the development of a melanin-concentrating hormone antagonist useful in the treatment of obesity and other associated or related diseases and conditions.

Accordingly, we have now found a novel group of pyrimidinones that exhibit a useful profile of activity as antagonists of the melanin-concentrating hormone receptor (MCHR1) disclosed in Nature, Vol. 400, p. 261-265 (1999).

### SUMMARY OF THE INVENTION

The present invention provides a compound of formula (Ia) comprising: 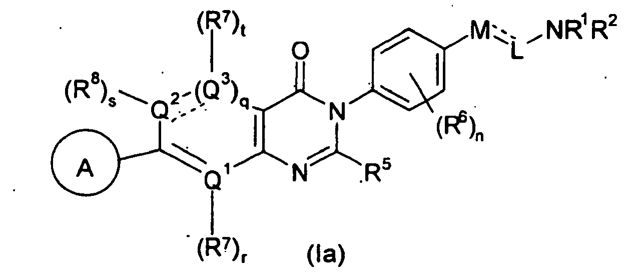 a pharmaceutically acceptable salt or solvate wherein:
Ⓐ is aryl or heteroaryl, optionally substituted by one to four C₁₋₆ straight or branched alkyl, alkenyl, halo, amino, alkylamino, dialkylamino, hydroxy, C₁₋₆ alkoxy, cyano, or alkylthio groups;
Q¹, Q², Q³ are C; and q, r, s, and t are 1;
   or
Q¹ is N, Q² is S and q, r and S are 0;
   or
Q¹ is C; Q² is S; q and S are 0; and r is 1;
Each corresponding R⁷ is independently selected from the group consisting of hydrogen, C₁₋₆ straight or branched alkyl, C₃₋₆ cycloalkyl, C₁₋₆ alkoxy, amino, C₁₋₆ alkylamino, C₁₋₆ dialkylamino, hydroxy, cyano, C₁₋₆ alkylthio, and halo;
R⁵ is selected from the group consisting of hydrogen, C₁₋₆ straight or branched alkyl, C₃₋₆ cycloalkyl, and C₁₋₃ alkylthio;
R⁶ is selected from the group consisting of hydrogen and methoxy; and n is 1;
M is selected from the group consisting of O, S, S(O)₂, S(O)₂NR, N-R, C(O), C(R)₂, N-C(O)R, and N-S(O)₂R, wherein R is selected from the group consisting of hydrogen, phenyl, heteroaryl, C₁₋₆ straight or branched alkyl, and C₃₋₆ cycloalkyl;
L is C₂₋₃ alkyl, C₂₋₃ alkenyl, or -C(O)(CH₂)-;
(i) R¹ and R² each independently are selected from the group consisting of hydrogen, C₁₋₆ straight or branched alkyl, C₃₋₆ cycloalkyl, and a 5- or 6-membered heterocycle wherein said alkyl, said cycloalkyl and said heterocycle are optionally substituted by phenyl, one to four C₁₋₃ alkyl, hydroxy, oxo (i.e., =O), alkoxy or halo;
or (ii) R¹ and R² may be selected from the group consisting of aryl and a 5- or 6-membered heteroaryl containing 1, 2, or 3 heteroatoms selected from N, O, and S, wherein said aryl and said heteroaryl are optionally substituted 1, 2, or 3 times with a substituent selected from halo, C₁₋₆ straight or branched alkyl, C₃₋₆ cycloalkyl, C₁₋₆ alkenyl, C₃₋₆ cycloalkenyl, hydroxy, C₁₋₆ alkoxy, oxo (i.e., =O), amino, C₁₋₆ alkylamino, C₁₋₆ dialkylamino, C₁₋₆ alkylthio, C₁₋₆ alkylsulfinyl, and phenyl;
or (iii) R¹ and R² together with the nitrogen atom to which they are bonded form a 4-8 membered heterocyclic ring or a 7-11 membered bicyclic heterocyclic ring, each of said 4-8 membered heterocyclic ring and said 7-11 membered bicyclic heterocyclic ring contain 1, 2 or 3 heteroatoms selected from the group consisting of N, O, and S, and wherein either said heterocyclic ring or said bicyclic heterocyclic ring may be optionally substituted by phenyl, one to four C₁₋₃ alkyl, hydroxy, C₁₋₃ alkoxy, oxo (i.e., =O), or halo;
or (iv) R¹ and R² may be independently linked either to the group L or linked to the group M when M is selected from the group consisting of S(O)₂NR, N-R, C(R)₂, N-C(O)R, and N-S(O)₂R, and wherein R is C₁₋₆ straight or branched alkyl, to form a 3-7 membered cyclic group which may be optionally substituted by phenyl, one to four C₁₋₃ alkyl, hydroxy, alkoxy, oxo (i.e., =O), or halo.

In another aspect of the invention, there is provided a pharmaceutical composition for use in the treatment, prophylaxis or both of one or more conditions or indications set forth herein comprising a compound of formula (Ia), or a physiologically acceptable salt or solvate, and a pharmaceutically acceptable carrier.

In a further embodiment of the invention, there are provided processes for the preparation a compound of formula (1a).

### Detailed Description of the Invention

As used herein, "a compound of the invention" or "a compound of formula (Ia)" means a compound of formula (Ia) or a pharmaceutically acceptable salt or solvate thereof.

As used herein, unless otherwise specified, the term "alkyl" and "alkylene" refer to straight or branched hydrocarbon chains containing 1 to 6 carbon atoms. Examples of "alkyl" as used herein include, but are not limited to, methyl, ethyl, n-propyl, n-butyl, n-pentyl, isobutyl, isopropyl, tert-butyl, and hexyl. Examples of "alkylene" as used herein include, but are not limited to, methylene, ethylene, propylene, butylene, and isobutylene. "Alkyl" also includes substituted alkyl. The alkyl groups may optionally be substituted with hydroxy, alkoxy, halo, amino, thio, and cyano. Halo, alkoxy, and hydroxy are particularly preferred.

As used herein, unless otherwise specified, the term "cycloalkyl" refers to a non-aromatic carbocyclic ring having from 3 to 8 carbon atoms (unless otherwise specified) and no carbon-carbon double bonds. "Cycloalkyl" includes by way of example cyclopropyl, cyclobutyl, cyclopentyl, cyclohexyl, cycloheptyl, and cyclooctyl. "Cycloalkyl" also includes substituted cycloalkyl. The cycloalkyl may be optionally substituted with substituents selected from the group consisting of hydroxy, cyano, halo, alkoxy, and alkyl. Halo, hydroxy, and alkoxy are preferred.

As used herein, unless otherwise specified, the term "alkenyl" refers to straight or branched hydrocarbon chains containing 2 to 8 carbon atoms and at least one and up to three carbon-carbon double bonds. Examples of "alkenyl" as used herein include, but are not limited to, ethenyl and propenyl. "Alkenyl" also includes substituted alkenyl. The alkenyl groups may be optionally substituted with alkyl, halo, hydroxy, alkoxy, and cyano. Halo, hydroxy, and alkoxy are preferred.

As used herein, unless otherwise specified, the term "cycloalkenyl" refers to a non-aromatic carbocyclic ring having from 3 to 8 carbon atoms (unless otherwise specified) and up to 3 carbon-carbon double bonds. "Cycloalkenyl" includes by way of example, cyclobutenyl, cyclopentenyl, and cyclohexenyl. "Cycloalkenyl" also includes substituted cycloalkenyl. The ring may be optionally substituted with at least one substituent selected from the group consisting of cyano, halo, hydroxy, NH₂, -N₃, -CN, -O-C₁₋₃alkyl, -NH(C₁₋₃ alkyl), -N(C₁₋₃alkyl)₂ and C₁₋₃alkyl (including haloalkyl).

As used herein, the terms "halo" or "halogen" refer to fluorine, chlorine, bromine, and iodine. Preferred among these are chlorine (or "chloro") and fluorine (or "fluoro").

Unless otherwise specified, the term, "aryl" refers to monocyclic carbocyclic groups and fused bicyclic carbocylic groups having from 6 to 12 carbon atoms and having at least one aromatic ring. Examples of particular aryl groups include but are not limited to phenyl and naphthyl. "Aryl" also includes substituted aryl, especially substituted phenyl. Aryl rings may be optionally substituted with substituents selected from the group consisting of halo, alkyl (including haloalkyl), alkenyl, cycloalkyl, cycloalkenyl, alkoxy, amino, hydroxy, hydroxyalkyl, aminoalkyl, carboxy, carboxamide, sulfonamide, heteroaryl (abbreviated as "Het"), amidine, cyano, nitro, and azido. Preferred aryl groups according to the invention include but are not limited to phenyl and substituted phenyl. Preferred substituted phenyl is a phenyl containing one or more halo groups, particularly chloro and fluoro groups.

The term "heterocyclic", unless otherwise specified, refers to monocyclic saturated or unsaturated non-aromatic groups and fused bicyclic non-aromatic groups, having the specified number of members (e.g., carbon and heteroatoms N and/or O and/or S) in a single ring and containing 1, 2, 3, or 4 hereroatoms selected from N, O and S. Examples of particular heterocyclic groups include but are not limited to tetrahydrofuran, dihydropyran, tetrahydropyran, pyran, oxetane, thietane, 1,4-dioxane, 1,3-dioxane, 1,3-dioxalane, piperidine, piperazine, tetrahydropyrimidine, pyrrolidine, morpholine, thiomorpholine, thiazolidine, oxazolidine, tetrahydrothiopyran, tetrahydrothiopyran, tetrahydrothiophene, and the like. "Heterocyclic" also includes substituted heterocyclic. The heterocyclic group may be optionally substituted with substituents selected from the group consisting of halo, alkyl (including haloalkyls), alkenyl, cycloalkyl, cycloalkenyl, perfluoroalkyl, alkoxy, amino, hydroxy, alkylhydroxy, alkylamine, carboxy, carboxamide, sulfonamide, Het, amidine, cyano, nitro, and azido. Preferred heterocyclic groups according to the invention include, but are not limited to, substituted and unsubstituted tetrahydrofuran, pyrrolidine, piperidine, morpholine, thiomorpholine, and piperazine. Piperidine, morpholine, piperazine, and pyrrolidine are particularly preferred, with pyrrolidine being most preferred.

The term "heteroaryl", unless otherwise specified, refers to aromatic monocyclic groups and aromatic fused bicyclic groups having the specified number of members (e.g., carbon and heteroatoms N and/or O and/or S) and containing 1, 2, 3, or 4 heteroatoms selected from N, O, and S. Examples of particular heteroaryl groups include but are not limited to furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, indole, and indazole. "Heteroaryl" also includes substituted heteroaryl. The heteroaryl group may be optionally substituted with substituents selected from the group consisting of halo, alkyl (including perhaloalkyl, e.g., perfluoroalkyl), alkenyl, cycloalkyl, cycloalkenyl, alkoxy, amino, hydroxy, alkylhydroxy, alkylamine, carboxy, carboxamide, sulfonamide, Het, amidine, cyano, nitro, and azido. Preferred heteroaryl groups according to the invention include, but are not limited to, substituted an unsubstituted pyridine, furan, thiophene, pyrrole, imidazole, oxadiazole, pyrazole, oxazole, thiazole, and pyrimidine. Pyridine, oxadiazole, and thiazole are most preferred.

As used herein, the term "optionally" means that the subsequently described event(s) may or may not occur, and includes both event(s) that occur and events that do not occur.

Formula (Ia) of the invention is set forth in detail as follows.
Ⓐ is aryl or heteroaryl, optionally substituted by one to four C₁₋₆ straight or branched alkyl, alkenyl, halo, amino, alkylamino, dialkylamino, hydroxy, C₁₋₆ alkoxy, cyano or alkylthio groups. Preferred among these substituted groups are halo, C₁₋₃ alkyl, and C₁₋₃ alkoxy. Most preferred are fluoro, chloro, and methoxy. In a preferred embodiment said aryl is substituted with a halo group, q is 0, Q¹ is carbon, and R⁷ is hydrogen or halo. For example, aryl is 4-chlorophenyl and R⁵ and R⁷ are each hydrogen.

In formula (Ia), q is 0 or 1. When q is 1 the dashed line between Q² and Q³ in formula (Ia) is a double bond. When q is 0 there is no dashed line, and the bond between Q² and Q³ is a single bond. When q is 0 then Q² is N, S, or O. And when q is 1, Q² is C or N. When q is 1 and Q² is N, then s is 0 and there is no R⁸ substituent.

Q¹ and Q³ are each independently carbon (C) or nitrogen (N). In one embodiment, Q¹, Q², and Q³ are carbon and q, r, s, and t are 1. In another embodiment, Q¹ is carbon, Q² is sulfur, q and s are 0, and r is 1.

In the formula, r and t are each independently 0 or 1. When r and t are each independently 0, then there is no R⁷ substituent. When r and t are each independently 1, Q¹ and Q³ are each independently bonded by the group R⁷. Each R⁷ is the same or different and is independently selected from hydrogen, C₁₋₆ straight or branched alkyl, C₃₋₆ cycloalkyl, C₁₋₆ alkoxy, amino, alkylamino, dialkylamio, hydroxy, cyano, alkylthio, and halo.

In formula (Ia), s is 0 or 1. When Q² is S or O, then s is 0 and there is no R⁸ group. When Q² is C, then s is 1 and R⁸ is selected from the group consisting of hydrogen, C₁₋₆ straight or branched alkyl, C₃₋₆ cycloalkyl, C₁₋₆ alkoxy, amino, alkylamino, dialkylamino, hydroxy, cyano, alkylthio, and halo. When Q² is C, preferably R⁸ is hydrogen or a C₁₋₃ alkyl; most preferably R⁸ is hydrogen or methyl.

When Q² is N, and s is 1, R⁸ is selected from the group consisting of hydrogen, C₁₋₆ straight or branched alkyl, C₃₋₆ cycloalkyl, C₁₋₆ alkoxy, amino, alkylamino, dialkyl amino, hydroxy, cyano, alkylthio, and halo. When Q² is N, preferably R⁸ is hydrogen or a C₁₋₃ alkyl; most preferably R⁸ is hydrogen or methyl.

When either or both Q¹ and Q³ are C, then R⁷ is selected from the group consisting of hydrogen, C₁₋₆ straight or branched alkyl, C₃₋₆ cycloalkyl, C₁₋₆ alkoxy, amino, alkylamino, dialkylamino, hydroxy, cyano, alkylthio, and halo. Preferably, when either or both Q¹ and Q³ are C, R⁷ is hydrogen or C₁₋₃ alkyl; most preferably R⁷ is hydrogen or methyl.

In formula (1a), R⁵ is selected from the group consisting of hydrogen, C₁₋₆ straight or branched alkyl, and C₃₋₆ cycloalkyl. Preferably, R⁵ is hydrogen or a C₁₋₃ alkyl; most preferably R⁵ is hydrogen or methyl.

R⁶ is selected from the group consisting of hydrogen and methoxy, and n is 1.

In the formula (Ia), M is selected from the group consisting of O, S, S(O)₂, S(O)₂NR, N-R, C(O), C(R)₂, N-C(O)R, and N-S(O)₂R, wherein R is selected from the group consisting of hydrogen, phenyl, heteroaryl, C₁₋₆ straight or branched alkyl, and C₃₋₆ cycloalkyl. Preferably M is selected from the group consisting of O, S, S(O)₂NR, N-R, N-C(O)R, and N-S(O)₂R; most preferably M is selected from the group consisting of O, N-R, and N-C(O)R. Preferably R is selected from the group consisting of hydrogen, phenyl, C₁₋₆ straight or branched alkyl, and C₃₋₆ cycloalkyl; most preferably R is selected from the group consisting of hydrogen, C₁₋₆ straight or branched alkyl, and C₃₋₆ cycloalkyl.

L of formula (la) is C₂₋₃ alkyl, C₂₋₃ alkenyl, or C(O)(CH₂)-. Preferably, L is C₂₋₃ alkyl or C₂₋₃ alkenyl; most preferably L is C₂₋₃ alkyl. C(O)(CH₂)- is only present when M is N.

In (i) R¹ and R² in formula (Ia) are each independently selected from the group consisting of hydrogen, C₁₋₆ straight or branched alkyl, C₃₋₆ cycloalkyl, phenyl, and 5- or 6-membered heterocycle, wherein said alkyl, said cycloalkyl, and said heterocycle are optionally substituted by phenyl, one to four C₁₋₃ alkyl, hydroxy, oxo, alkoxy, or halo. Preferably, R¹ and R² are selected from the group consisting of hydrogen, C₁₋₆ straight or branched alkyl, and C₃₋₆ cycloalkyl. Most preferably, R¹ and R² are selected from the group consisting of hydrogen, C₁₋₃ alkyl, and C₃₋₆ cycloalkyl.

Or, in (ii) R¹ and R² may be selected from the group consisting of aryl and a 5- or 6-membered heteroaryl containing 1, 2, or 3 heteroatoms selected from N, O, and S, wherein said aryl and said heteroaryl are optionally substituted 1, 2, or 3 times with a substituent selected from the group consisting of halo, C₁₋₆ straight or branched alkyl, C₃₋₆ cycloalkyl, C₁₋₆ alkenyl, C₃₋₆ cycloalkenyl, hydroxy, C₁₋₆ alkoxy, oxo, amino, C₁₋₆ alkylamino, C₁₋₆ dialkylamino, C₁₋₆ alkylthio, C₁₋₆ alkylsulfinyl, and phenyl. Preferably, when either R¹ or R² is aryl or heteroaryl, the other remaining R¹ or R² is hydrogen, C₁₋₆ alkyl, or a C₃₋₆ cycloalkyl.

Additionally, in (iii) R¹ and R² together with the nitrogen atom to which they are bonded can form a 4-8 membered heterocyclic ring or a 7-11 membered bicyclic heterocyclic ring. The 4-8 membered heterocyclic ring and/or the 7-11 membered bicyclic heterocyclic ring may contain 1, 2, or 3 heteroatoms selected from the group consisting of N, O, and S. And either the heterocyclic ring or the bicyclic heterocyclic ring may be optionally substituted by phenyl, one to four C₁₋₃ alkyl, hydroxy, C₁₋₃ alkoxy, oxo, or halo. Here neither group R¹ or R² is linked back to M or L. Preferably, R¹ and R² together form a 5- or 6-membered heterocyclic ring or an 8- to 11-membered bicylic heterocyclic ring, having 1 or 2 heteroatoms selected from the group N, O, and S wherein said heterocyclic ring and said bicyclic heterocyclic ring may be optionally substituted up to two times with a substituent selected from the group consisting of oxo and halo.

Also additionally, in (iv) R¹ and R² may be independently linked either to the group L or linked to the group M when M is selected from the group consisting of S(O)₂NR, N-R, C(R)₂, N-C(O)R, and N-S(O)₂R, (where R is C₁₋₆ straight or branched alkyl), to form a 3-7 membered cyclic group. The 3-7 membered cyclic group may be optionally substituted by phenyl, one to four C₁₋₃ alkyl, hydroxy, alkoxy, oxo, or halo. Preferably, either or both R¹ and R² are linked to M when M is selected from the group consisting of S(O)₂NR, N-R, C(R)₂, N-C(O)R, and N-S(O)₂R, (wherein R is C₁₋₆ straight or branched alkyl), to form a 4-7 membered ring. Most preferably a 5-7 membered ring is formed. The 5-7 membered ring or cyclic group may be optionally substituted by phenyl, one to four C₁₋₃ alkyl, hydroxy, alkoxy, oxo, or halo. Here, either the R¹ group or the R² group, or both R¹ and R² are linked back to the group L. Preferably, only one of the groups R¹ and R² are linked back to the group L.

In one embodiement when L is C₂-C₃ alkyl or C₂-C₃ alkenyl, in (i), R¹ and R² are selected from the group consisting of hydrogen, C₁-C₃ straight or branched alkyl, C₃-C₆ cycloalkyl substituted with a substituent selected from the group consisting of halo, alkyl, hydroxy, oxo, and alkoxy. Or, when L is C₂-C₃ alkyl or C₂-C₃ alkenyl, in (iii), R¹ and R² together with the nitrogen atom to which they are bonded form a 4-6 membered heterocyclic ring wherein said heterocyclic ring is optionally substituted with a substituent selected from the group consisting of one to four C₁-C₃ alkyl, hydroxy, alkoxy, oxo, and halo.

In another embodiment, when L is a C₂-C₃ alkyl, in (i), R¹ and R² are each independently selected from the group consisting of hydrogen and C₃-C₆ cycloalkyl substituted with a substituent selected from the group consisting of oxo and halo. Or, when L is a C₂-C₃ alkyl, in (iii), R¹ and R² together with the nitrogen atom to which they are bonded form a 5- or 6- membered heterocyclic that is optionally substituted with a substituent selected from the group consisting of one to two oxo and halo. In a further embodiment L is CH₂CH₂ and, in (iii), R¹ and R² together with the nitrogen atom to which they are bonded form a pyrrolidine ring substituted at the 3-position with a fluorine atom.

In still another embodiment M is O, N-R or N-C(O)R, where R is hydrogen or C₁-C₆ straight or branched alkyl, and R⁶ is selected from the group consisting of hydrogen, C₁-C₆ straight or branched alkyl, C₁-C₃ alkoxy, trihaloalkyl, trihaloalkoxy, cyano, and halo. Preferably, M is O or N-R where R is hydrogen and R⁶ is selected from the group consisting of hydrogen, C₁-C₂ straight or branched alkyl, C₁-C₂ alkoxy, or halo. Most preferably in this embodiment, M is O and R⁶ is methoxy.

Most preferred compounds according to this invention are selected from the group consisting of 6-(4-chlorophenyl)-3-{3-methoxy-4-[2-(3-oxopyrrolidin-1-yl)ethoxy]phenyl)thieno[3,2-*d*] pyrimidin-4(3*H*)-one and 6-(4-chlorophenyl)-3-{4-[2-(3-fluoropyrrolidin-1-yl)ethoxy]-3-methoxyphenyl}thieno[3,2-d]pyrimidin-4(3H)-one.

Certain compounds of formula (la) may exist in stereoisomeric forms (e.g., they may contain one or more asymmetric carbon atoms or may exhibit cis-trans isomerism). The individual stereoisomers (enantiomers and diastereomers) and mixtures of these are included within the scope of the present invention. The present invention also covers the individual isomers of the compounds represented by formula (Ia) as mixtures with isomers thereof in which one or more chiral centers are inverted. Certain compounds of formula (1a) may be prepared as regioisomers. The present invention covers both the mixture of regioisomers as well as individual compounds. Likewise, it

| Example No. | Structure | Name |
|---|---|---|
| H1 | 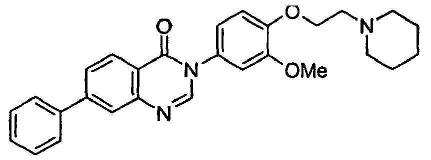 | 3-{3-methoxy-4-[2-(1-piperidinyl)ethoxy]phenyl}-7-phenyl-4(3*H*)-quinazolinone |
| H2 | 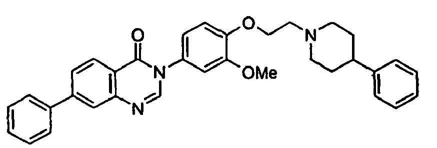 | 3-{3-methoxy-4-[2-(4-phenyl-1-piperidinyl)ethoxy]phenyl}-7-phenyl-4(3*H*)-quinazolinone |
| H3 | 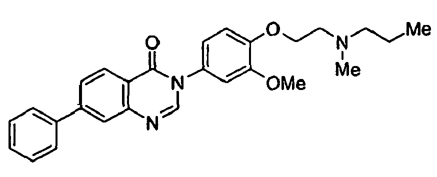 | 3-(3-methoxy-4-{2-[methyl(propyl)amino]ethoxy}p henyl)-7-phenyl-4(3*H*)-quinazolinone |
| H4 | 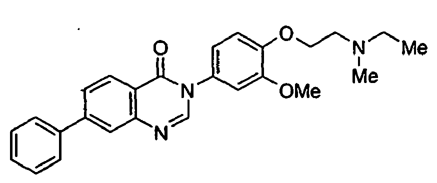 | 3-(4-{2-[ethyl(methyl)amino]ethoxy}-3-methoxyphenyl)-7-phenyl-4(3*H*)-quinazolinone |
| H5 | 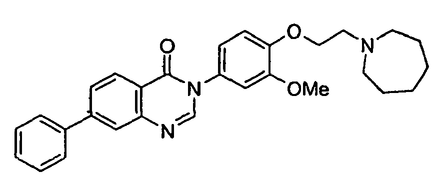 | 3-{4-[2-(1-azepanyl)ethoxy]-3-methoxyphenyl}-7-phenyl-4(3*H*)-quinazolinone |
| H6 | 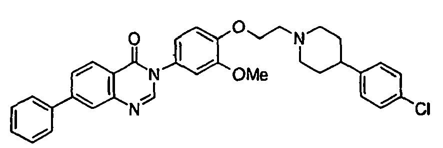 | 3-(4-{2-[4-(4-chlorophenyl)-1-piperidinyl]ethoxy}-3-methoxyphenyl)-7-phenyl-4(3*H*)-quinazolinone |
| H7 | 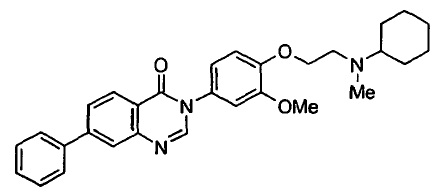 | 3-(4-{2-[cyclohexyl(methyl)amino]etho xy}-3-methoxyphenyl)-7-phenyl-4(3*H*)-quinazolinone |
| H8 | 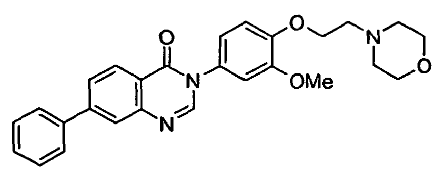 | 3-{3-methoxy-4-[2-(4-morpholinyl)ethoxy]phenyl}-7-phenyl-4(3*H*)-quinazolinone |
| H9 | 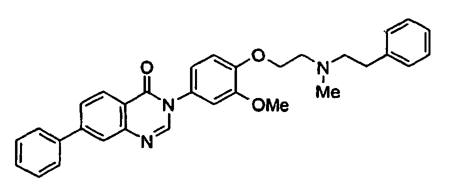 | 3-(3-methoxy-4-(2-[methyl(2-phenylethyl)amino]ethoxy}phe nyl)-7-phenyl-4(3*H*)-quinazolinone |
| H10 | 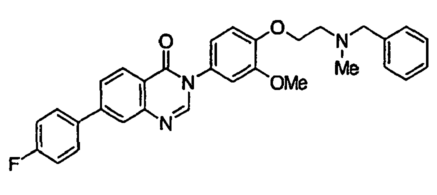 | 3-(4-{2-[benzyl(methyl)amino]ethoxy}-3-methoxyphenyl)-7-(4-fluorophenyl)-4(3*H*)-quinazolinone |
| H11 | 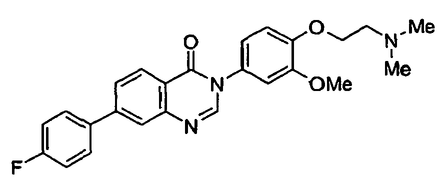 | 3-(4-[2-(dimethylamino)ethoxy]-3-methoxyphenyl}-7-(4-fluorophenyl)-4(3*H*)-quinazolinone |
| H12 | 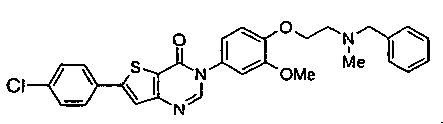 | 3-(4-{2-[benryl(methyl)amino]ethoxy}-3-methoxyphenyl)-6-(4-chlorophenyl)thieno[3,2-*d*]pyrimidin-4(3*H*)-one |
| H13 | 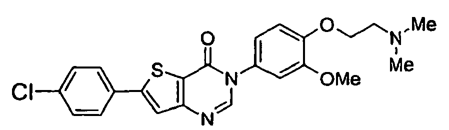 | 6-(4-chlorophenyl)-3-(4-[2-(dimethylamino)ethoxy]-3-methoxyphenyl)thieno[3,2-*d*]pyrimidin-4(3*H*)-one |
| H14 | 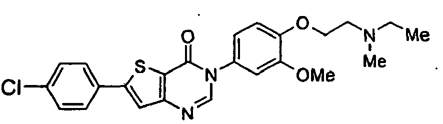 | 6-(4-chlorophenyl)-3-(4-{2-[ethyl(methyl)amino]ethoxy}-3-methoxyphenyl)thieno[3,2-*d*]pyrimidin-4(3*H*)-one |
| H15 | 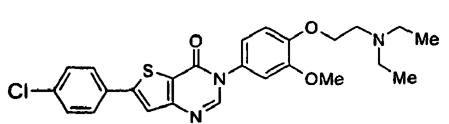 | 6-(4-chlorophenyl)-3-{4-[2-(diethylamino)ethoxy]-3-methoxyphenyl}thieno[3,2-d]pyrimidin-4(3*H*)-one |
| H16 | 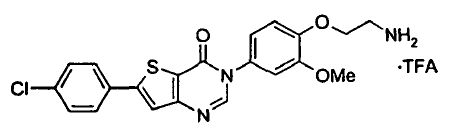 | 3-[4-(2-aminoethoxy)-3-methoxyphenyl]-6-(4-chlorophenyl)thieno[3,2-*d*]pyrimidin-4(3*H*)-one trifluoroacetate salt |
| H17 | 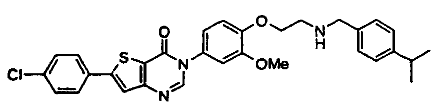 | 6-(4-chlorophenyl)-3-(4-{2-[(4-isopropylbenzyl)amino]ethoxy} -3-methoxyphenyl)thieno[3,2-*d*]pyrimidin-4(3*H*)-one |
| H18 | 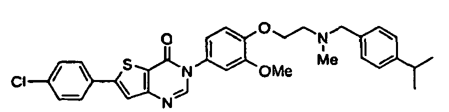 | 6-(4-chlorophenyl)-3-(4-{2-[(4-isopropylbenzyl)(methyl)amino ]ethoxy}-3-methoxyphenyl)thieno[3,2-*d*]pyrimidin-4(3*H*)-one |
| H19 | 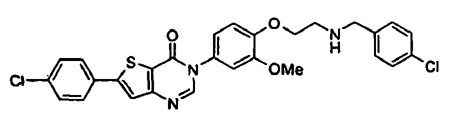 | 3-(4-{2-[(4-chlorobenzyl)amino]ethoxy}-3-methoxyphenyl)-6-(4-chlorophenyl)thienol3,2-*d*]pyrimidin-4(3*H*)-one |
| H20 | 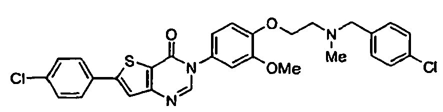 | 3-(4-{2-[(4-chlorobenzyl)(methyl)amino]et hoxy}-3-methoxyphenyl-6-(4-chlorophenyl)thieno[3,2-*d*]pyrimidin-4(3*H*)-one |
| H21 | 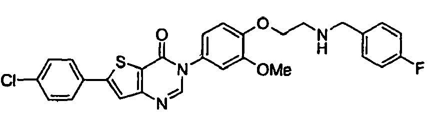 | 6-(4-chlorophenyl)3-(4-{2-[(4-fluorobenzyl)amino]ethoxy}-3-methoxyphenyl)thieno[3,2-*d*]pyrimidin-4(3*H*)-one |
| H22 | 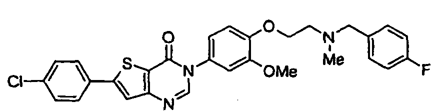 | 6-(4-chlorophenyl)-3-(4-{2-[(4-fluorobenzyl)(methyl)amino]et hoxy}-3-methoxyphenyl)thieno[3,2-*d*]pyrimidin-4(3*H*)-one |
| H23 | 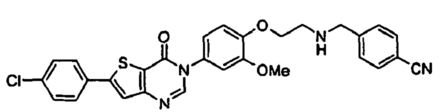 | 4-[({2-[4-(6-(4-chlorophenyl)-4-oxothieno[3,2-*d*]pyrimidin-3(4*H*)-yl)-2-methoxyphenoxy]ethyt}amino) methyl]benzonitrile |
| H24 | 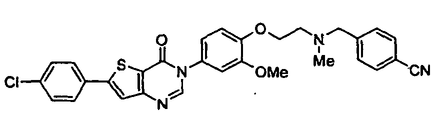 | 4-{[{2-[4-(6-(4-chlorophenyl)-4-oxothieno[3,2-*d*]pyrimidin-3(4H)-yl)-2-methoxyphenoxy]ethyl}(methyl )amino]methyl}benzonitrile |
| H25 | 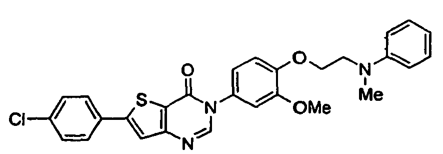 | 6-(4-chlorophenyl)3-{3-methoxy-4-[2-(methylanilino)ethoxy]phenyl} thieno[3,2-*d*]pyrimidin-4(3*H*)-one |
| H26 | 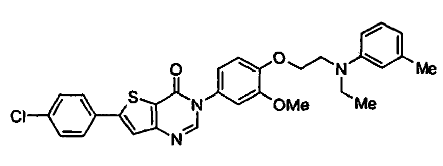 | 6-(4-chlorophenyl)-3-{4-[2-(ethyl-3-methylanilino)ethoxy]-3 -methoxyphenyl}thieno[3,2-*d*]pyrimidin-4(3*H*)-one |
| H27 | 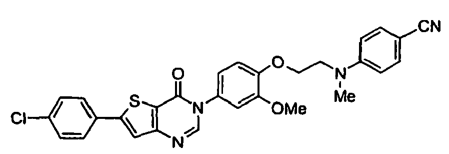 | 4-[{2-[4-(6-(4-chlorophenyl)-4-oxothieno[3,2-*d*]pyrimidin-3(4*H*)-yl)-2-methoxyphenoxy]ethyl}(methyl )amino]benzonitrile |
| H28 | 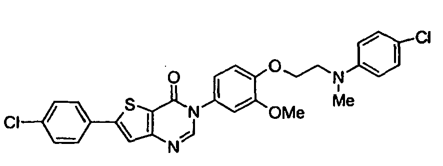 | 3-(4-{2-[4-chloro(methyl)anilino]ethoxy}-3-methoxyphenyl)-6-(4-chlorophenyl)thieno[3,2-*d*]pyrimidin-4(3*H*)-one |
| H29 | 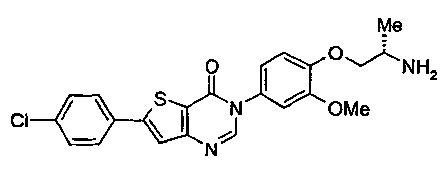 | 3-(4-{[(2S)-2-aminopropyl]oxy}-3-methoxyphenyl)6-(4-chlorophenyl)thieno{3,2-d]pyrimidin-4(3*H*)-one |
| H30 | 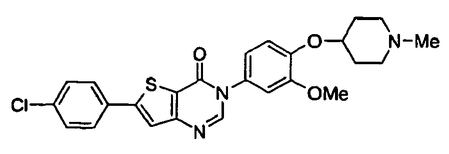 | 6-(4-chlorophenyl)-3-{3-methoxy-4-[(1-methyl-4-piperidinyl)oxy]phenyl}thieno[3 ,2-*d*]pyrimid)n-4(3*H*)-one |
| I1 | 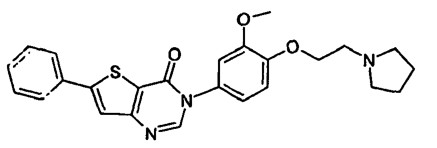 | 3-[3-Methoxy-4-(2-pyrrolidin-1-ylethoxy)phenyl]-6-phenylthieno[3,2-d]pyrimidin-4(3H)-one |
| I2 | 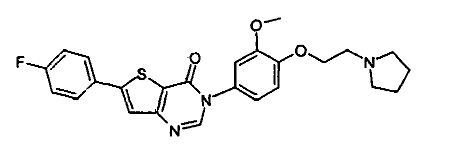 | 6-(4-Fluorophenyl)-3-[3-methoxy-4-(2-pyrrolidin-1-ylethoxy)phenyl]thieno[3,2-d]pyrimidin-4(3H)-one |
| I3 | 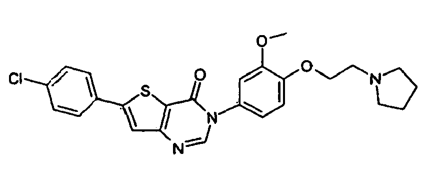 | 6-(4-Chlorophenyl)-3-[3-methoxy-4-(2-pyrrolidin-1-ylethoxy)phenyl]thieno[3,2-d]pyrimidin-4(3H)-one |
| I4 | 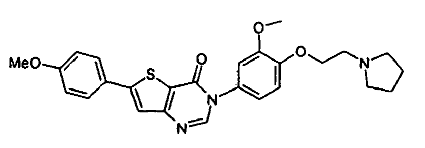 | 6-(4-Methoxyphenyl)-3-[3-methoxy-4-(2-pyrrolidin-1-ylethoxy)phenyl]thieno[3,2-d]pyrimidin-4(3H)-one |
| I5 | 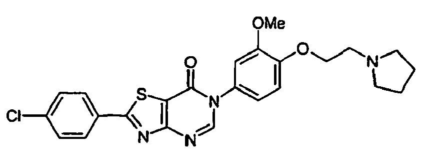 | 2-(4-Chlorophenyl)-6-[3-methoxy-4-(2-pyrrolidin-1ylethoxy)phenyl][1,3]thiazolo[ 4,5-d]pyrimidin-7(6H)-one |
| I6 | 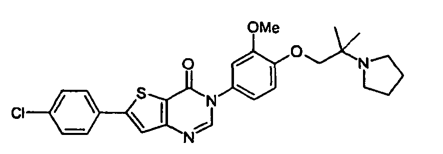 | 6-(4-Chlorophenyl)-3-[3-methoxy-4-(2-methyl-2-pyrrolidin-1-ylpropoxy)phenyl]thieno[3,2-d]pyrimidin-4(3H)-one |
| I7 | 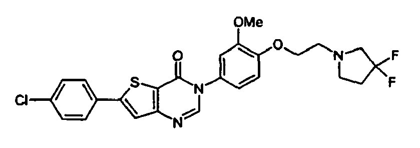 | 6-(4-Chlorophenyl)-3-{4-[2-(3,3-difluoropyrrolidin-1-yl)ethoxy]-3-methoxyphenyl}thieno[3,2-d]pyrimidin-4(3H)-one |
| I8 | 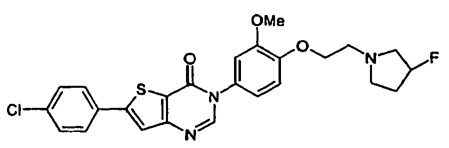 | 6-(4-chlorophenyl)-3-{4-[2-(3-fluoropyrrolidin-1-yl)ethoxy]-3-methoxyphenyl)thieno[3,2-d]pyrimidin-4(3H)-one |
| J1 | 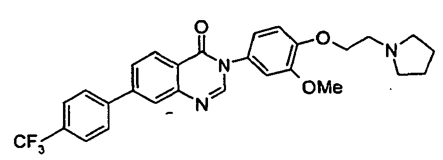 | 3-{3-methoxy-4-[2-(1-pyrrolidinyl)ethoxy]phenyl}-7-[4-(trifluoromethyl)phenyl]-4(3*H*)-quinazolinone |
| J2 | 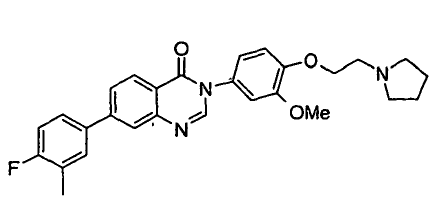 | 7-(4-fluoro-3-methylphenyl)-3-{3-methoxy-4-[2-(1-pyrrolidinyl)ethoxy]phenyl}-4(3*H*)-quinazolinone |
| J3 | 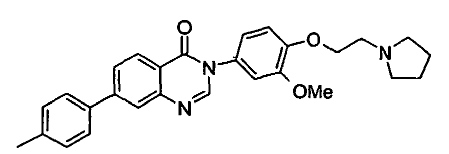 | 3-{3-methoxy-4-[2-(1-pyrrolidinyl)ethoxy]phenyl}-7-(4-methylphenyl)-4(3*H*)-quinazolinone |
| J4 | 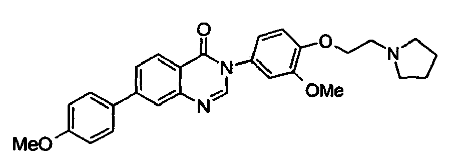 | 7-(4-methoxyphenyl)-3-{3-methoxy-4-[2-(1-pyrrolidinyl)ethoxy]phenyl}-4(3*H*)-quinazolinone |
| J5 | 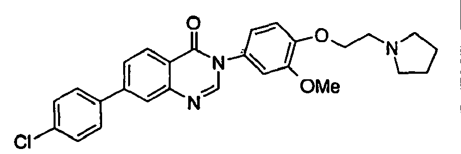 | 7-(4-chlorophenyl)-3-(3-methoxy-4-[2-(1-pyrrolidinyl)ethoxy]phenyl}-4(3*H*)-quinazolinone |
| J6 | 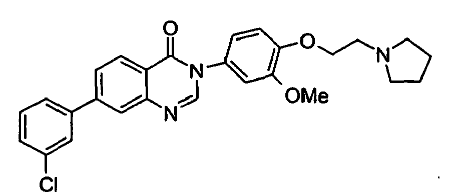 | 7-(3-chlorophenyl)-3-{3-methoxy-4-[2-(1-pyrrolidinyl)ethoxy]phenyl}-4(3*H*)-quinazolinone |
| J7 | 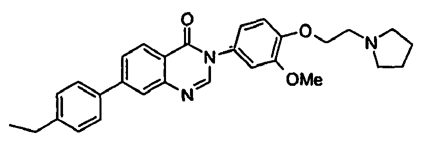 | 7-(4-ethylphenyl)-3-{3-methoxy-4-[2-(1-pyrrolidinyl)ethoxy]phenyl}-4(3*H*)-quinazolinone |
| J8 | 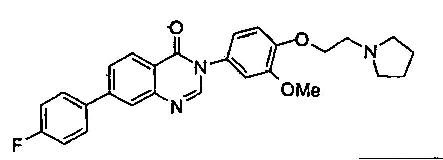 | 7-(4-fluorophenyl)-3-(3-methoxy-4-[2-(1-pyrrolidinyl)ethoxy]phenyl}-4(3*H*)-quinazolinone |
| J9 | 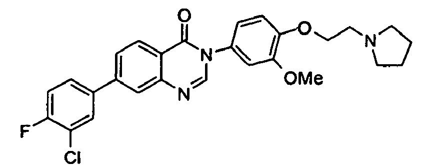 | 7-(3-chloro-4-fluorophenyl)-3-{3-methoxy-4-[2-(1-pyrrolidinyl)ethoxy]phenyl}-4(3*H*)-quinazolinone |
| J10 | 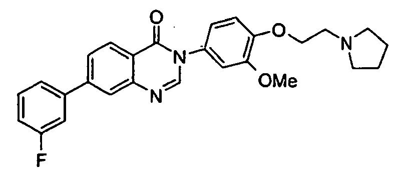 | 7-(3-fluorophenyl)-3-{3-methoxy-4-[2-(1-pyrrolidinyl)ethoxy]phenyl)-4(3*H*)-quinazolinone |
| J11 | 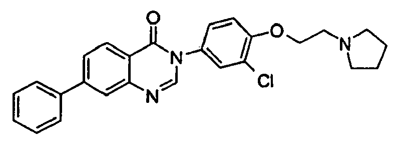 | 3-{3-chloro-4-[2-(1-pyrrolidinyl)ethoxy]phenyl}-7-phenyl-4(3*H*)-quinazolinone |
| J12 | 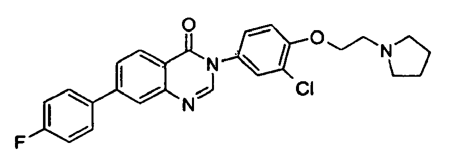 | 3-{3-chloro-4-[2-(1-pyrrolidinyl)ethoxy]phenyl}-7-(4-fluoropheny)-4(3*H*)-quinazolinone |
| J13 | 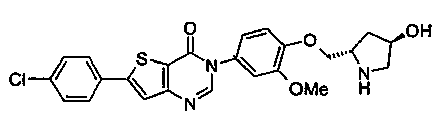 | 6-(4-chlorophenyl)-3-(4-{[(2*S*,4*R*)-4-hydroxypyrrolidinyl]methoxy}-3-methoxyphenyl)thieno[3,2-*d*]pyrimidin-4(3*H*)-one |
| J14 | 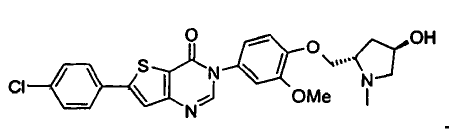 | 6-(4-chlorophenyl)-3-(4-{[(2S,4R)-4-hydroxy-1-methylpyrrolidinyl]methoxy}-3-methoxyphenyl)thieno[3,2-*d*]pyrimidin-4(3*H*)-one |
| J15 | 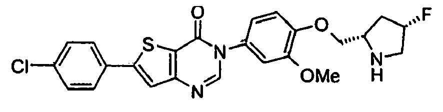 | 6-(4-chlorophenyl)-3-(4-{[(2S,4S)-4-fluoropyrrolidinyl]methoxy}-3-methoxyphenyl)thieno(3,2-*d*]pyrimidin-4(3*H*)-one |
| J16 | 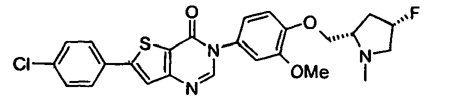 | 6-(4-chlorophenyl)-3-(4-{[(2*S*,4*S*)-4-fluoro-1-methy)pyrrolidiny)]methoxy}-3-methoxyphenyl)thieno[3,2-*d*]pyrimidin-4(3*H*)-one |
| J17 | 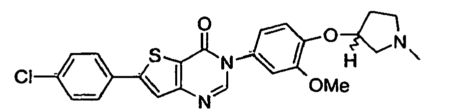 | 6-(4-chlorophenyl)-3-{3-methoxy-4-[(1-methyl-3-pyrrolidinyl)oxy]phenyl}thieno[ 3,2-*d*]pyrimidin-4(3*H*)-one |
| J18 | 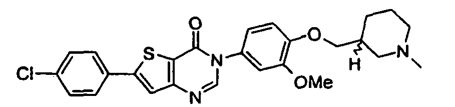 | 6-(4-chlorophenyl)-3-{3-methoxy-4-[(1-methyl-3-piperidinyl)methoxy]phenyl}thi eno[3,2-*d*]pyrimidin-4(3*H*)-one |
| J19 | 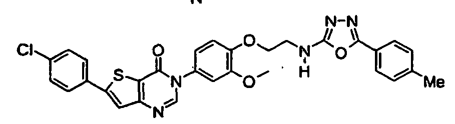 | 6-(4-chlorophenyl)-3-[3-methoxy-4-(2-{[5-(4-methylphenyl)-1,3,4-oxadiazol-2-yl]amino}ethoxy)phenyl]thieno[ 3,2-*d*]pyrimidin-4(3*H*)-one |
| K1 | 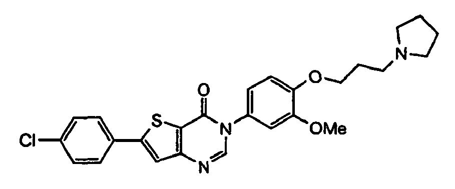 | 6-(4-Chlorophenyl)-3-[3-methoxy-4-(3-pyrrolidin-1-ylpropoxy)phenyl] thieno[3,2-*d*]pyrimidin-4(3*H*)-one (1) |
| K2 | 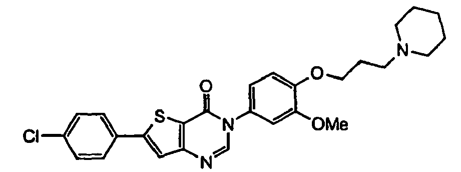 | 6-(4-Chlorophenyl)-3-[3-methoxy-4-(3-piperidin-1-ylpropoxy)phenyl] thieno[3,2-*d*]pyrimidin-4(3*H*)-one |
| K3 | 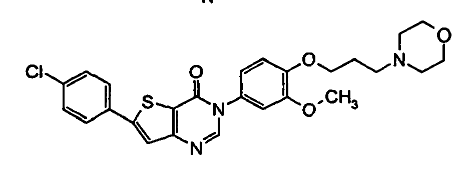 | 6-(4-Chlorophenyl)-3-[3-methoxy-4-(3-morpholin-4-ylpropoxy)phenyl] thieno[3,2-*d*]pyrimidin-4(3*H*)-one |
| K4 | 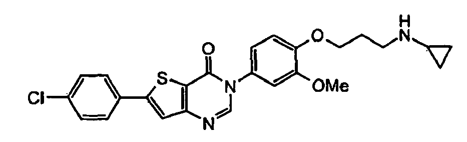 | 6-(4-Chlorophenyl)-3-{4-[3-(cyclopropylamino)propoxy]-3-methoxy- phenyl)thieno[3,2-*d*]pyrimidin-4(3*H*)-one |
| K5 | 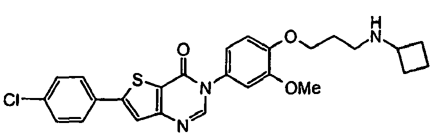 | 6-(4-Chlorophenyl)-3-{4-[3-(cyclobutylamino)propoxy]-3-methoxy-phenyl}thieno[3,2-*d*]pyrimidin-4(3*H*)-one |
| K6 | 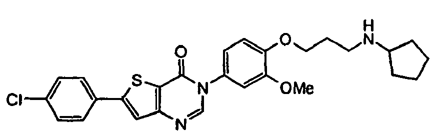 | 6-(4-Chlorophenyl)-3-{4-[3-(cyclopentylamino)propoxy]-3-methoxy-phenyl)thieno[3,2-*d*]pyrimidin-4(3*H*)-one |
| K7 | 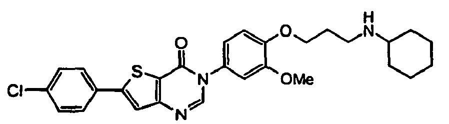 | 6-(4-Chlorophenyl)-3-(4-[3-(cyclohexylamino)propoxy]-3-methoxy-phenyl}thieno[3,2-*d*]pyrimidin-4(3*H*)-one |
| K8 | 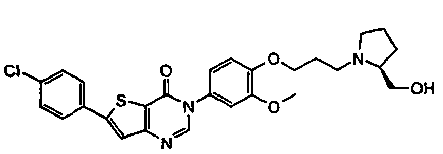 | 6-(4-Chlorophenyl)-3-(4-{3-[(2*S*)-2-(hydroxymethyl)pyrrolidin-1-yl]propoxy}-3-methoxyphenyl) thieno[3,2-*d*]pyrimidin-4(3*H*)-one |
| K9 | 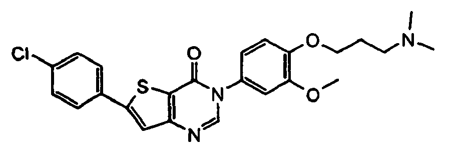 | 6-(4-Chlorophenyl)-3-{4-[3-(dimethylamino)propoxy]-3-methoxy-phenyl}thieno[3,2-*d*]pyrimidin-4(3*H*)-one |
| K10 | 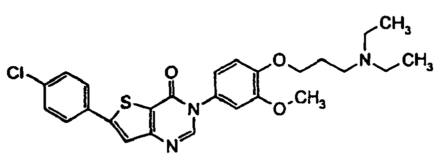 | 6-(4-Chlorophenyl)-3-{4-[3-(diethylamino)propoxy]-3-methoxyphenyl} thieno[3,2-*d*]pyrimidin-4(3*H*)-one |
| K11 | 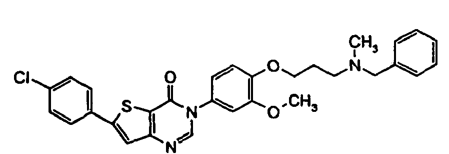 | 3-(4-{3-[benzyl(methyl)amino]propoxy) -3-methoxyphenyl)-6-(4-chlorophenyl)thieno[3,2-*d*]pyrimidin-4(3*H*)-one |
| K12 | 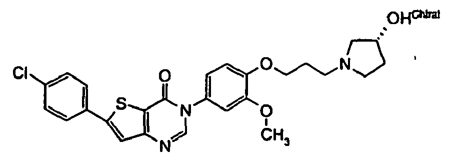 | 6-(4-Chlorophenyl)-3-(4-{3-[(3R)-3-hydroxypyrrolidin-1-yl]propoxy}-3-methoxyphenyl)thieno[3,2-d]pyrimidin-4(3H)-one |
| K13 | 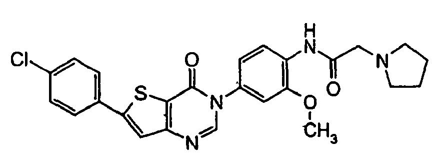 | N-(4-[6-(4-chlorophenyl)-4-oxothieno[3,2-d]pyrimidin-3(4H)-yl]-2-methoxyphenyl}-2-pyrrolidin-1-ylacetamide |
| K14 | 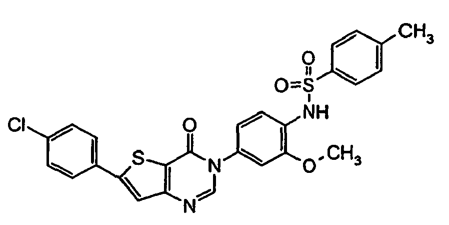 | N-{4-[6-(4-chlorophenyl}-4-oxothieno[3,2-d]pyrimidin-3(4H)-yl]-2-methoxyphenyl}-4-methylbenzenesulfonamide |
| K15 | 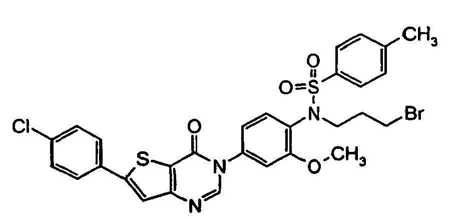 | N-(3-bromopropyl)-N-{4-[6-(4-chlorophenyl)-4-oxothieno[3,2-d]pyrimidin-3(4H)-yl]-2-methoxyphenyl}-4-methylbenzenesulfonamide |
| K16 | 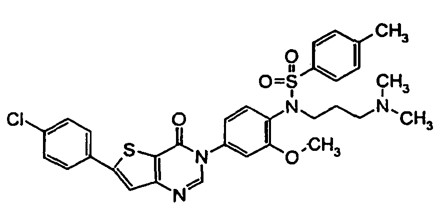 | N-{4-[6-(4-chlorophenyl)-4-oxothieno[3,2-d]pyrimidin-3(4H)-yl]-2-methoxyphenyl}-N-[3-(dimethylamino)propyl]-4-methylbenzene-sulfonamide (14) |
| K17 | 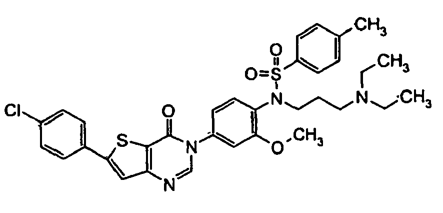 | N-{4-[6-(4-chlorophenyl)-4-oxothieno[3,2-d]pyrimidin-3(4H)-yl]-2-methoxyphenyl}-N-[3-(diethylamino)propyl]-4-methylbenzene-sulfonamide |
| K18 | 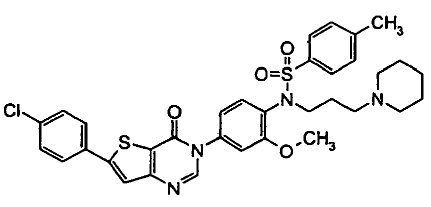 | N-{4-[6-(4-chlorophenyl)-4-oxothieno[3,2-d]pyrimidin-3(4H)-yl]-2-methoxyphenyl}-4-methyl-N-(3-piperidin-1-ylpropyl)benzene-sulfonamide |
| K19 | 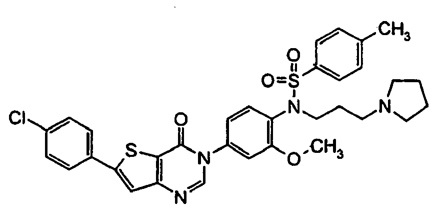 | N-{4-[6-(4-chlorophenyl)-4-oxothieno[3,2-d]pyrimidin-3(4H)-yl]-2-methoxyphenyl}-4-methyl-N-(3-pyrrolidin-1-ylpropyl)benzene-sulfonamide |
| K20 | 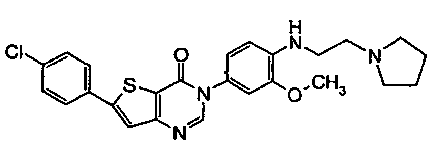 | 6-(4-chlorophenyl)-3-{3-methoxy-4-[(2-pyrrolidin-1-ylethyl)amino] phenyl)thieno[3,2-*d*]pyrimidin-4(3*H*)-one |
| K21 | 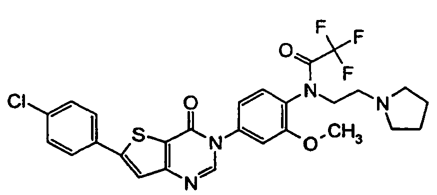 | N-{4-[6-(4-chlorophenyl)-4-oxothieno[3,2-d]pyrimidin-3(4H)-yi]-2-methoxyphenyl)-2,2,2-trifluoro-N-(2-pyrrolidin-1-ylethyl)acetamide |
| K22 | 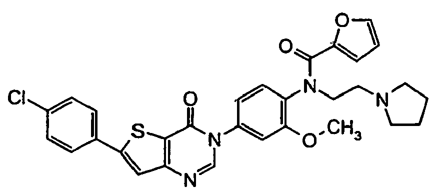 | N-{4-[6-(4-chlorophenyl)-4-oxothieno[3,2-d]pyrimidin-3(4H)-yl]-2-methoxyphenyl}-N-(2-pyrrolidin-1-ylethyl)-2-furamide |
| K23 | 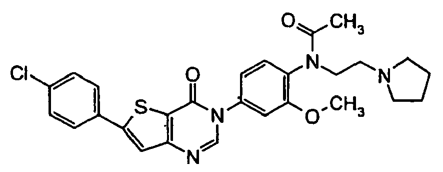 | N-{4-[6-(4-chlorophenyl)-4-oxothieno[3,2-d]pyrimidin-3(4H)-yl]-2-methoxyphenyl}-N-(2-pyrrolidin-1-ylethyl)acetamide |
| K24 | 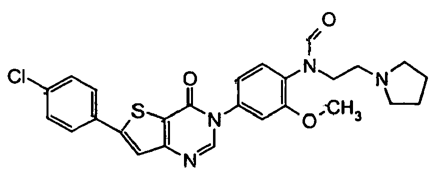 | 4-[6-(4-Chlorophenyl)-4-oxothieno[3,2-*d*]pyrimidin-3(4*H*)-yl]-2-methoxyphenyl(2-pyrrolidin-1-ylethyl)formamide |
| K25 | 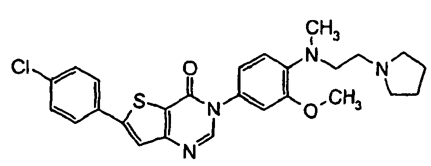 | 6(4-Chlorophenyl)3-{3-methoxy-4-[methyl(2-pyrrolidin-1-ylethyl)-amino]phenyl}thieno[3,2-*d*]pyrimidin-4(3*H*)-one |
| K26 | 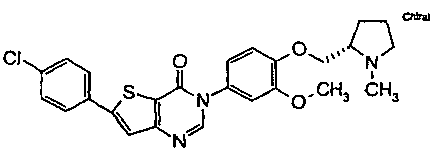 | 6-(4-Chlorophenyl)-3-(3-methoxy-4-{[(2S)-1-methylpyrrolidin-2-yl] methoxy}phenyl)thieno[3,2-*d*]pyrimidin-4(3*H*)-one |
| K27 | 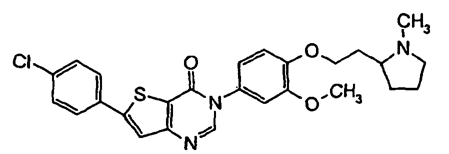 | 6-(4-Chlorophenyl)-3-{3-methoxy-4-[2-(1-methylpyrrolidin-2-yl)ethoxy] phenyl}thieno[3,2-*d*]pyrimidin-4(3*H*)-one |
| K28 | 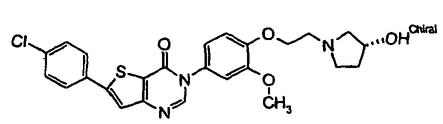 | 6-(4-Chlorophenyl)-3-(4-{2-[(3R)-3-hydroxypyrrolidin-1-yl]ethoxy}-3-methoxyphenyl)thieno[3,2-d]pyrimidin-4(3*H*)-one |
| K29 | 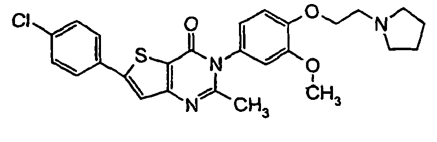 | 6-(4-Chlorophenyl)-3-[3-methoxy-4-(2-pyrrolidin-1-ylethoxy)phenyl]-2-methylthieno[3,2-*d*]pyrimidin-4(3*H*)-one |
| K30 | 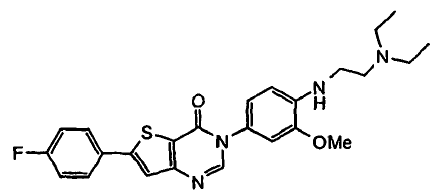 | 3-(4-{[2-(diethylamino)ethyl]amino}-3-methoxyphenyl)6-(4-fluorophenyl) thieno[3,2-*d*]pyrimidin-4(3*H*)-one |
| K31 | 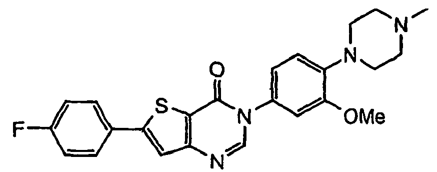 | 6-(4-Fluorophenyl)-3-[3-methoxy-4-(4-methylpiperazin-1-yl)phenyl]thieno[3,2-*d*]pyrimidin-4(3*H*)-one |
| K32 | 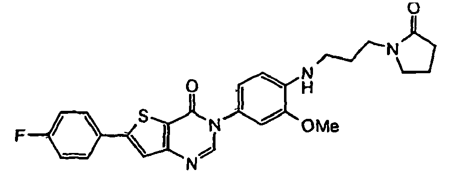 | 6-(4-Fluorophenyl)-3-(3-methoxy-4-{[3-(2-oxopyrrolidin-1-yl)propyl]amino}phenyl) thieno[3,2-*d*]pyrimidin-4(3*H*)-one |
| K33 | 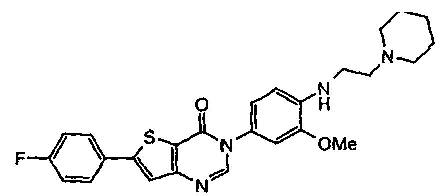 | 6-(4-Fluorophenyl)-3-(3-methoxy-4-[(2-piperidin-1-ylethyl)amino]phenyl} thieno[3,2-*d*]pyrimidin-4(3*H*)-one |
| K34 | 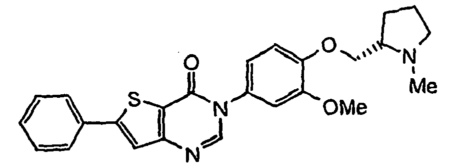 | 3-(3-Methoxy-4-{[(2*R*)-1 -methylpyrrolidin-2-yl]methoxy}phenyl)-6-phenylthieno [3,2-*d*]pyrimidin-4(3*H*)-one |
| K35 | 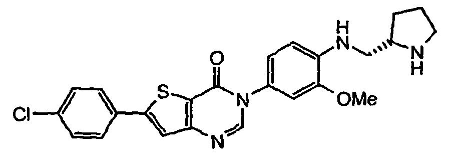 | 6-(4-Chlorophenyl)-3-(3-methoxy-4-{[(2*R*)-pyrrolidin-2-ylmethyl]amino}phenyl) thieno[3,2-d]pyrimidin-4(3H)-one |
| K36 | 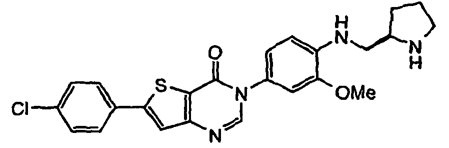 | 6-(4-Chlorophenyl)-3-(3-methoxy-4-{[(2S)-pyrrolidin-2-ylmethyl]amino}phenyl) thieno[3,2-*d*]pyrimidin-4(3*H*)-one |
| K37 | 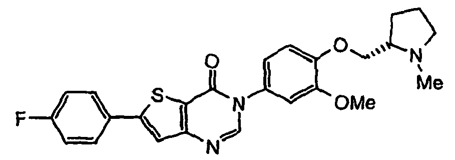 | 6-(4-Fluorophenyl)-3-(3-methoxy-4-{[(2R)-1-methylpyrrolidin-2-yl]methoxy}phenyl) thieno[3,2-*d*]Pyrimidin-4(3*H*)-one |
| L1 | 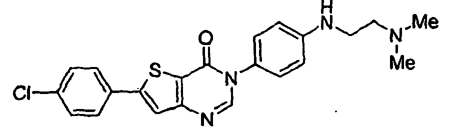 | 6-(4-chlorophenyl)-3-(4-{[2-(dimethylamino)ethyl]amino}ph enyl)thieno[3,2-*d*]pyrimidin-4(3*H*)-one |
| L2 | 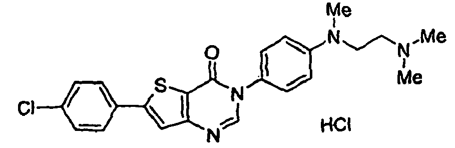 | 6-(4-chlorophenyl)-3-{4-[[2-(dimethylamino)ethyl](methyl)a mino]phenyl}thieno[3,2-*d*]pyrimidin-4(3*H*)-one hydrochloride |
| L3 | 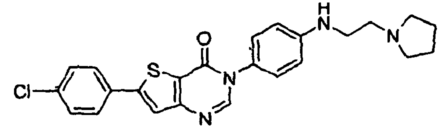 | 6-(4-chlorophenyl)-3-(4-{[2-(1-pyrrolidinyl)ethyl]amino}phenyl )thieno[3,2-*d*]pyrimidin-4(3*H*)-one |
| L4 | 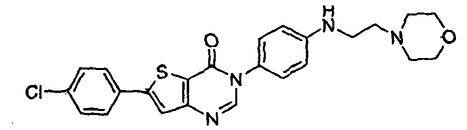 | 6-(4-chlorophenyl)-3-(4-{[2-(4-morpholinyl)ethyl]amino}phenyl l)thieno[3,2-*d*]pyrimidin-4(3*H*)-one |
| L5 | 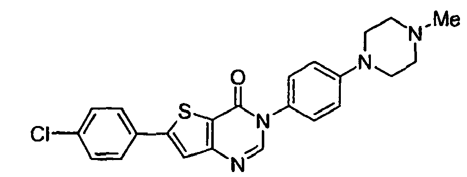 | 6-(4-chlorophenyl)-3-[4-(4-methyl-1-piperazinyl)phenyl]thieno[3,2-*d*]pyrimidin-4(3*H*)-one |
| M1 | 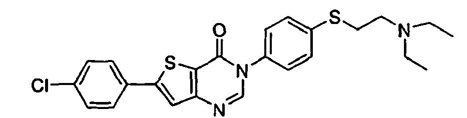 | 6-(4-chlorophenyl)-3-(4-{[2-(diethylamino)ethyl]sulfanyl}ph enyl)thieno[3,2-*d*]pyrimidin-4(3*H*)-one |
| M2 | 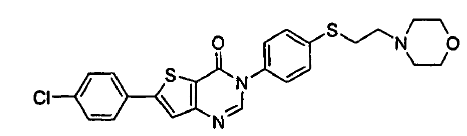 | 6-(4-chlorophenyl)-3-(4-{[2-(4-morpholinyl)ethyl]sulfanyl}phe nyl)thieno[3,2-d]pyrimidin-4(3*H*)-one |
| N1 | 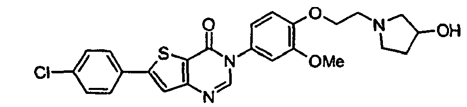 | 6-(4-chlorophenyl)-3-{4-[2-(3-hydroxypyrrolidin-1-yl)ethoxy]-3-methoxyphenyl}thieno[3,2-*d*]pyrimidin-4(3*H*)-one |
| N2 | 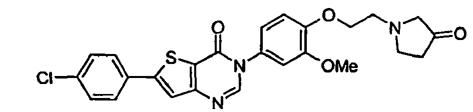 | 6-(4-chlorophenyl)-3-{3-methoxy-4-[2-(3-oxopyrrolidin-1-yl)ethoxy]phenyl}thieno[3,2-*d*]pyrimidin-4(3*H*)-one |
| O1 | 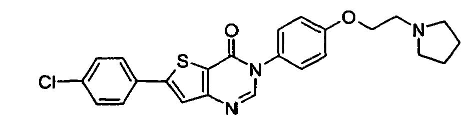 | 6-(4-chlorophenyl)-3-[4-(2-pyrrolidin-1-ylethoxy)phenyl]thieno[3,2-d]pyrimidin-4(3*H*)-one |
| O2 | 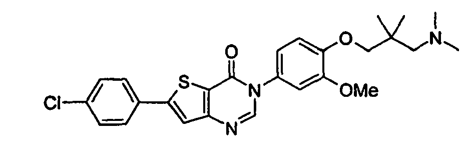 | 6-(4-Chlorophenyl)-3-{4-[3-(dimethylamino)-2,2-dimethylpropoxy]-3-methoxyphenyl}thieno[3,2-d]pyrimidin-4(3*H*)-one |
| O3 | 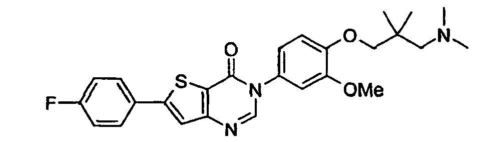 | 6-(4-Fluorophenyl)-3-{4-[3-(dimethylamino)-2,2-dimethylpropoxy]-3-methoxyphenyl)thieno[3,2-*d*]pyrimidin-4(3*H*)-one |
| O4 | 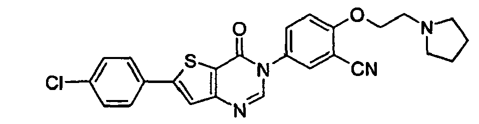 | 5-[6-(4-Chlorophenyl)-4-oxothieno[3,2-*d*]pyrimidin-3(4*H*)-yl]-2-(2-pyrrolidin-1-ylethoxy)benzonitrile |
| O5 | 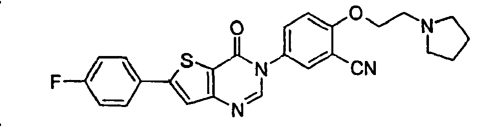 | 5-[6-(4-Fluorophenyl)-4-oxothieno[3,2-*d*]pyrimidin-3(4*H*)-yl]-2-(2-pyrrolidin-1-ylethoxy)benzonitrile |
| O6 | 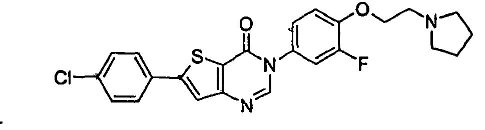 | 6-(4-Chlorophenyl)-3-[3-fluoro-4-(2-pyrrolidin-1-ylethoxy)phenyl]thieno[3,2-*d*]pyrimidin-4(3*H*)-one |

It will be appreciated by those skilled in the art that the compounds of the present invention may also be utilized in the form of a pharmaceutically acceptable salt or solvate thereof (e.g., a prodrug). The pharmaceutically acceptable salts of the compounds of formula (Ia) include conventional salts formed from pharmaceutically acceptable inorganic or organic acids or bases as well as quaternary ammonium salts. More specific examples of suitable acid salts include maleic, hydrochloric, hydrobromic, sulfuric, phosphoric, nitric, perchloric, fumaric, acetic, propionic, succinic, glycolic, formic, lactic, aleic, tartaric, citric, palmoic, malonic, hydroxymaleic, phenylacetic, glutamic, benzoic, salicylic, fumaric, toluenesulfonic, methanesulfonic (mesylate), naphthaliene-2-sulfonic, benzenesulfonic, hydroxynaphthoic, hydroiodic, malic, steroic, tannic, and the like.

Other acids such as oxalic, while not in themselves pharmaceutically acceptable, may be useful in the preparation of salts useful as intermediates in obtaining the compounds of the invention and their pharmaceutically acceptable salts. More specific examples of suitable basic salts include sodium, lithium, potassium, magnesium, aluminum, calcium, zinc, N,N'-dibenzylethylenediamine, chloroprocaine, choline, diethanolamine, ethylenediamine, N-methylglucamine and procaine salts.

The term "solvate" as used herein refers to a complex of variable stoichiometry formed by a solute (a compound of formula (Ia)) and a solvent. Solvents, by way of example, include water, methanol, ethanol, and acetic acid.

Processes for preparing pharmaceutically salts and solvates of the compounds of formula (la) are conventional in the art. See, e.g., Burger's Medicinal Chemistry and Drug Discovery, 5^{th} Edition, Vol.1: Principles and Practice.

Compounds of formula (la) below are conveniently prepared in accordance with the reaction schemes and/or processes outlined or described below.

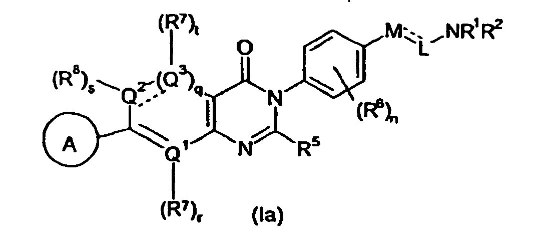

As will be apparent to those skilled in the art, in the processes described below for the preparation of compounds of formula (la), certain intermediates, may be in the form of pharmaceutically salts, solvates or physiologically functional derivatives of the compound. Those terms as applied to any intermediate employed in the process of preparing compounds of formula (Ia) have the same meanings as noted above with respect to compounds of formula (Ia). Processes for preparing pharmaceutically acceptable salts, solvates and physiologically functional derivatives of such intermediates are known in the art and are analogous to the process for preparing pharmaceutically acceptable salts, solvates and physiological functional derivatives of the compounds of formula (Ia). Unless otherwise stated, Ⓐ, R⁸, R⁷, R⁶, R⁵, R², R¹, M, L, Q¹, Q², Q³, q, r, s, t, and n are as defined in formula (Ia).

Thus compounds of formula (Ia) may be prepared by reaction of an aniline of formula (II) below with a compound of formula (III) and wherein Ⓐ, R⁸, R⁷, R⁶, R⁵, R², R¹, M, L, Q¹, Q², Q³, q, r, s, t, and n are as defined in formula (la).

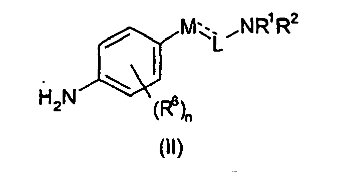

Compounds of formula (la) wherein R⁵ is H can be prepared from formamidine ester (III) by heating with the appropriate aniline (II) in a solvent such as ethanol or decalin and wherein Ⓐ, R⁸, R⁷, R⁶, R⁵, R², R¹, M, L, Q¹, Q², Q³, q, r, s, t, and n are as defined in formula (Ia).

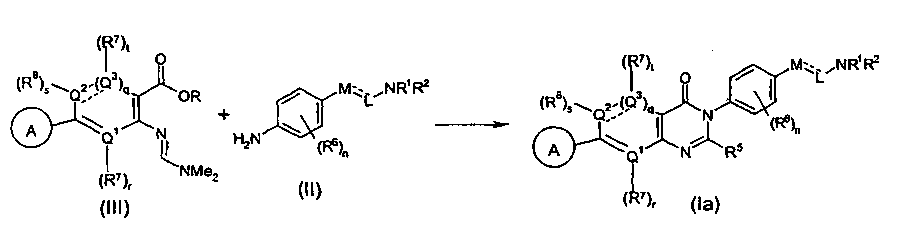

Compounds of formula (la) can also be prepared by an amide coupling of the corresponding amino acid (IV) and the desired aniline (II) in a solvent, such as methylene chloride, with amide coupling agents such as EDC, followed by cyclization in refluxing carboxylic acids, such as formic acid and wherein Ⓐ, R⁸, R⁷, R⁶, R⁵, R², R¹, M, L, Q¹, Q², Q³, q, r, s, t, and n are as defined in formula (Ia).

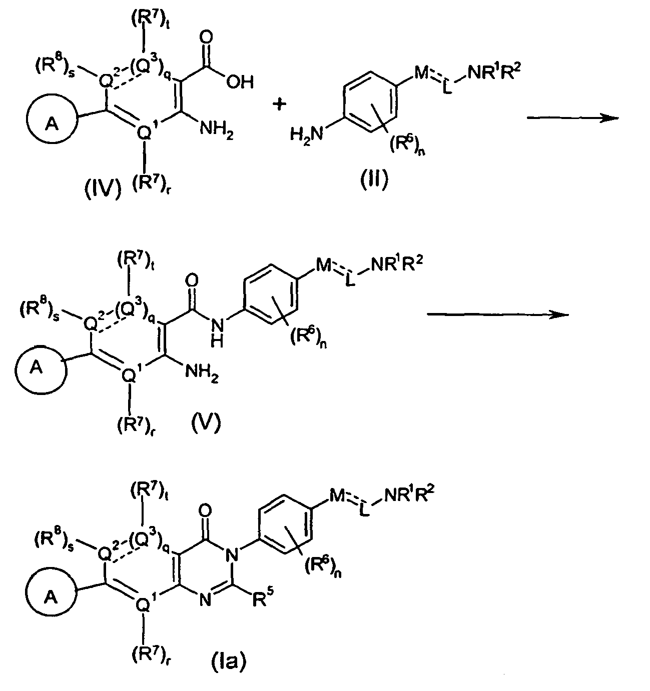

Compounds of formula (Ia) may also be prepared by reaction of a compound of formula (Va) 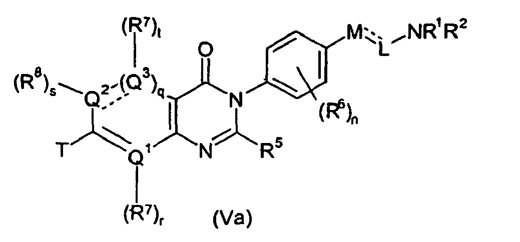 Ⓐ with a compound capable of introducing the group Ⓐ, and wherein Ⓐ, R⁸, R⁷, R⁶, R⁵, R², R¹, M, L, Q¹, Q², Q³, q, r, s, t, and n are as defined in formula (la) and T is a leaving group.

Thus compounds of formula (la) may be prepared from the compound of formula (Va) with the a boronic acid and a palladium catalyst using a Suzuki coupling reaction or with an organostannane reagent and a palladium catalyst using a Stille coupling reaction.

Compounds of formula (1a) wherein R⁵ is hydrogen may also be prepared by reaction of a sulfur-containing compound such as VI with a reductant, such as Raney Nickel, in a solvent such as ethanol and wherein Ⓐ, R⁸, R⁷, R⁶, R⁵, R², R¹, M, L, Q¹, Q², Q³, q, r, s, t, and n are as defined in formula (1a).

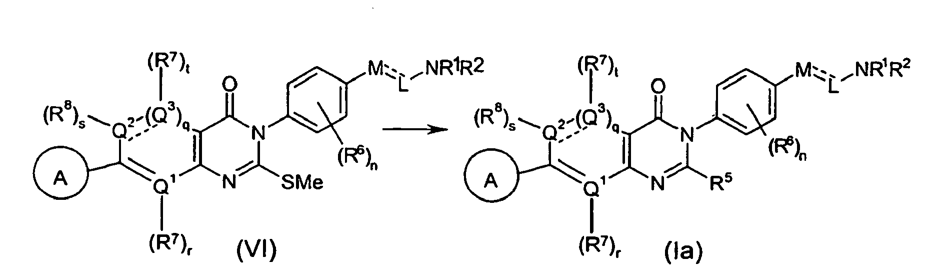

Compounds of formula (II) may be prepared by reduction of the corresponding nitro aromatic (VII) using hydrogen and a catalyst (e.g. 10% Pd on carbon), stannous chloride, or sodium dithionite 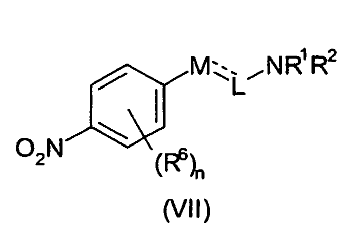 wherein R⁶, M, L, R¹ and R², and n have the meanings defined in formula (la) or a group convertible thereto.

Compounds of formula VII wherein M is O and R⁶, L, R¹, R², and n have the meanings defined in formula (Ia) can be prepared from halo aromatics (VIII) wherein X is chloro or fluoro and an alcohol of formula (IX) in the presence of a suitable base such as cesium carbonate, potassium carbonate or sodium hydride and a polar aprotic solvent such as DMF or DMSO and wherein Ⓐ, R⁸, R⁷, R⁶, R⁵, R², R¹, M, L, Q¹, Q², Q³, q, r, s, t, and n are as defined in formula (la).

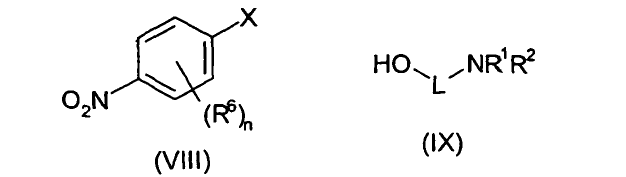

Alternatively, compounds of formula (VII) wherein M is O and R⁶, L, R¹, R², and n have the meanings defined in formula (la) can be prepared from phenols of formula (X) and alcohols of formula (IX) via a Mitsunobu coupling.

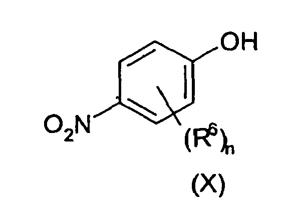

Alternatively, compounds of formula VII wherein M is O or S and R⁶, L, R¹, R², and n have the meanings defined in formula (Ia) can be prepared from phenols of formula (X) and thiophenols of formula (XI) by alkylation with a compound of formula (XII) wherein T is a leaving group such as chloro, bromo, tosylate or mesylate.

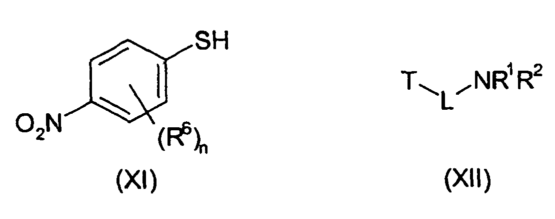

Compounds of formula (VII) wherein M is N and R⁶, L, R¹, R², and n have the meanings defined in formula (Ia) can be prepared from halo aromatics (VIII) wherein X is chloro or fluoro and an amine of formula (XIII) in the presence of a base such as excess amine (XIII) or a trialkylamine.

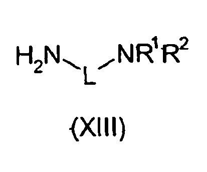

Compounds of formula (VII) wherein M is S(O)₂NR and R⁶, L, R¹, R², and n have the meanings defined in formula (Ia) can be prepared by reaction of amine (XIII) with 4-nitrobenzenesulfonylchloride.

Compounds of formula (Ia) in which M is N-S(O)₂R can be prepared by reaction of compounds of formula (Ia) in which M is NH with sulfonyl chlorides in the presence of a tertiary amine such as triethylamine. Compounds of formula (la) may be prepared by alkylation of an amine of formula (XV) with an alkylating agent of formula (XIV) wherein M is O, T is a leaving group and R¹ and R² have the meanings defined in formula (la).

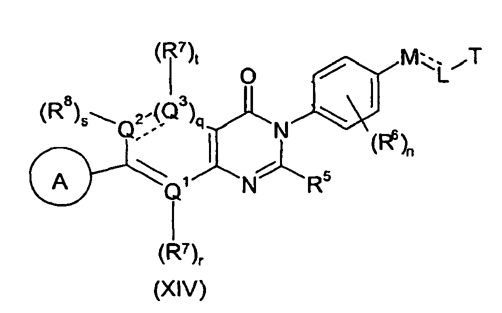

H-NR¹R² (XV

Compounds of formula (Ia) in which M is N(CO)R can be prepared by acylation of aniline of general formula (XVI) by an acylating agent of formula (XVII) and wherein wherein R⁹ is selected from the group consisting of hydrogen, phenyl, heteroaryl, C₁₋₆ straight or branched alkyl, and C₃₋₆ cycloalkyl. 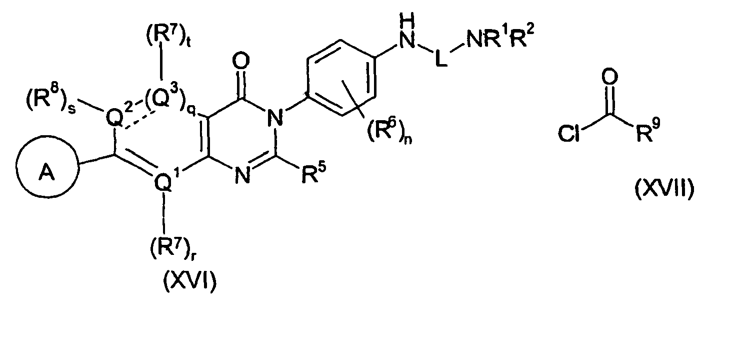 Compounds of formula (Ia) in which M is N can be prepared by reductive alkylation of aniline of general formula (XIX) by an aldehyde of formula (XVIII) in the presence of a borohydride reducing agent or hydrogen and a catalyst and in which the L of formula (XVIII) is CH₂ or CH₂CH₂ and wherein Ⓐ, R⁸, R⁷, R⁶, R⁵, R², R¹, M, Q¹, Q², Q³, q, r, s, t, and n are as defined in formula (1a). 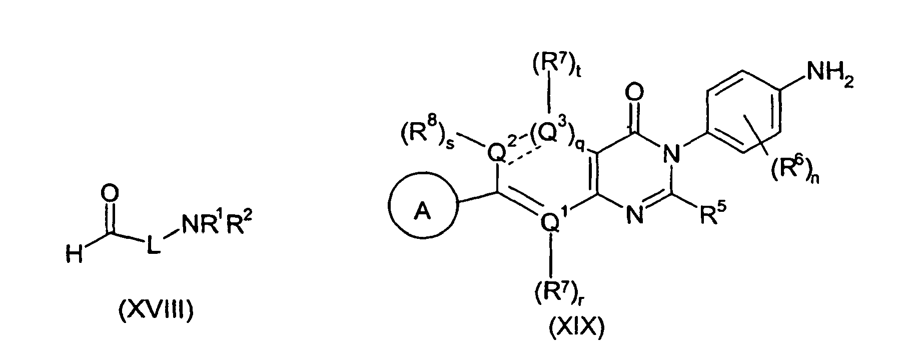 Compounds of formula (la) in which M is O can be prepared by alkylation of a phenol of formula (XX) by an alkylating agent of formula (XXI) in which T is a leaving group and wherein Ⓐ, R⁸, R⁷, R⁶, R⁵, R², R¹, M, L, Q¹, Q², Q³, q, r, s, t, and n are as defined in formula (Ia).

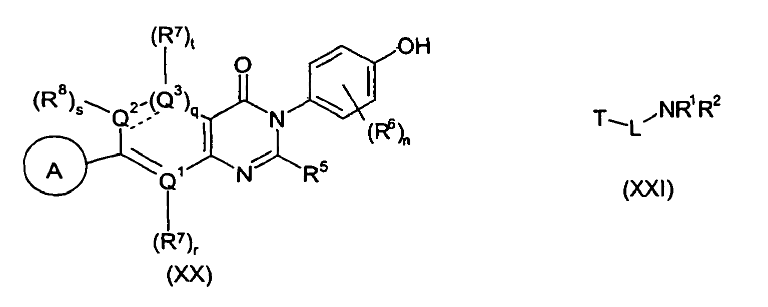

Alternatively, compounds of this type can be made by reductive amination of an aldehyde of formula (XXII) by an amine of formula (XV) in the presence of a reducing agent such as a borohydride or hydrogen and a catalyst and wherein L is CH₂ or CH₂CH₂ and Ⓐ, R⁸, R⁷, R⁶, R⁵, R², R¹, M ,Q¹, Q², Q³, q, r, s, t, and n are as defined in formula (1a).

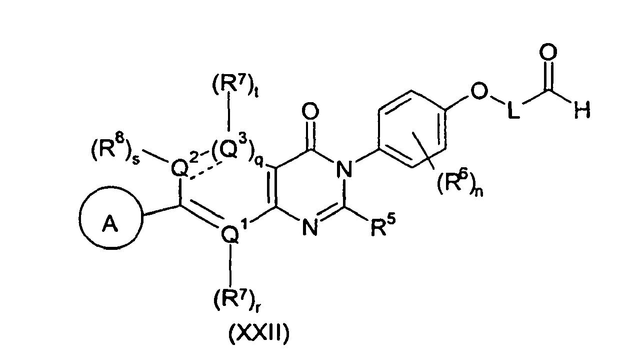

Compounds of formula (XXII) can be prepared by acid treatment of an acetal of formula (XXIII) in which R¹⁰ is alkyl and wherein Ⓐ, R⁸, R⁷, R⁶, R⁵, R², R¹, M, L, Q¹, Q², Q³, q, r, s, t, and n are as defined in formula (Ia).

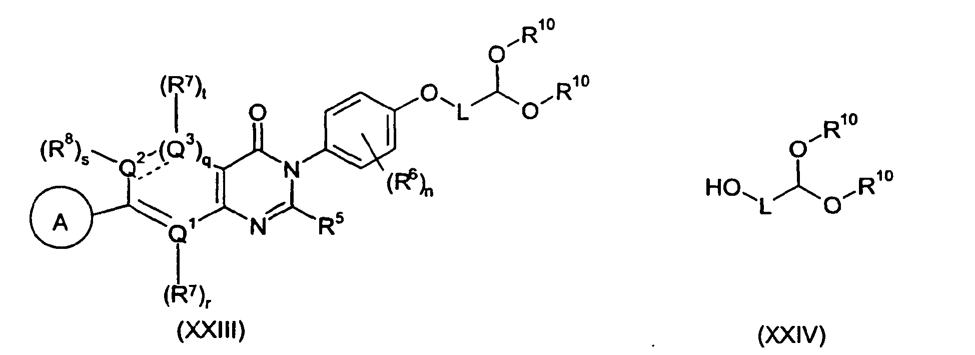

Compounds of formula (XXIII) can be made via alkylation of an alcohol of formula (XX) with a compound of formula (XXIII) followed by the protocol as hereinbefore described.

Compounds of formula (la) in which M is ○ and Ⓐ, R⁸, R⁷, R⁶, R⁵, R¹, M, Q¹, Q², Q³, q, r, s, t, and n are as defined in formula (la) can be made by reductive alkylation of amines of formula (XXV) with an aldehyde and wherein for formula (XXV) G is H and Ⓐ, R⁸, R⁷, R⁶, R⁵, R², R¹, M, L, Q¹, Q², Q³, q, r, s, t, and n are as defined in formula (la). Amines of formula (XXV) can be made by acid treatment of N-t-butoxycarbonyl protected derivatives of formula (XXVI) that in turn can be prepared by alkylation of alcohols of formula (XXVII) in which G is t-butoxycarbonyl with a compound of formula (VIII) followed by the protocol as hereinbefore described.

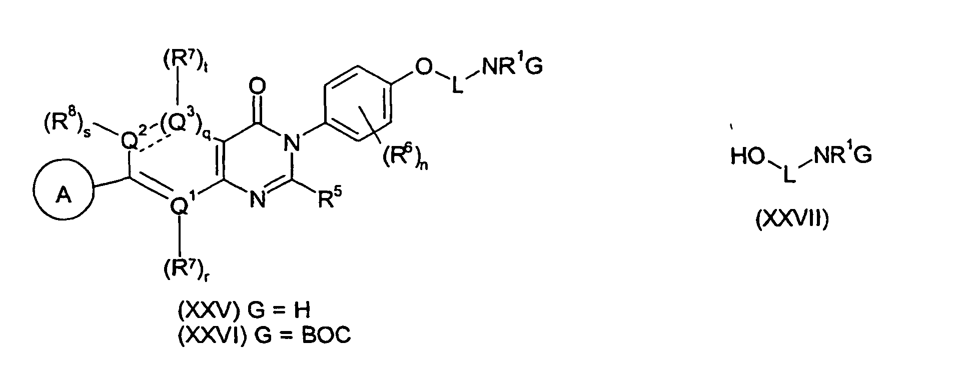

Compounds of formula (la) in which L is -C(O)CH₂- can be prepared by reaction of an amine of formula (XV) with an alkylating agent of formula (XXVIII) in which T is a leaving group such as chloro or bromo and wherein Ⓐ, R⁸, R⁷, R⁶, R⁵, R², R¹, M, L, Q¹, Q², Q³, q, r, s, t, and n are as defined in formula (la).

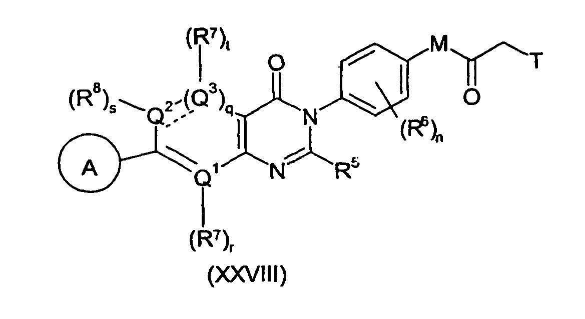

The compounds of formula (la) are believed to have a role in the treatment of obesity and/or diabetes. Compounds of the present invention are antagonists of a MCHR1 and can be used for the treatment of a disease caused by or attributable to a melanin-concentrating hormone. Compounds of the invention may reduce hunger, suppress appetite, control eating, and/or induce satiety.

The present invention provides methods for the treatment of several conditions or diseases such as obesity, diabetes, depression (eg., major depression and/or bipolar disorder), and/or anxiety. Such treatment comprises the step of administering a therapeutically effective amount of the compound of formula (Ia), including a salt, solvate, or physiologically functional derivative thereof. Such treatment can also comprise the step of administering a therapeutically effective amount of a pharmaceutical composition containing a compound of formula (la), including a salt, solvate, or physiologically functional derivative thereof. As used herein, the term "treatment" refers to alleviating the specified condition, eliminating or reducing the symptoms of the condition, slowing or eliminating the progression of the condition, and preventing or delaying the reoccurrence of the condition in a previously afflicted patient or subject.

As used herein, the term "therapeutically effective amount" means an amount of a compound of formula (1a) which is sufficient, in the subject to which it is administered, to elicit the biological or medical response of a cell culture, tissue, system, animal (including human) that is being sought, for instance by a researcher or clinician.

The precise therapeutically effective amount of the compounds of formula (Ia) will depend on a number of factors including, but not limited to, the age and weight of the subject being treated, the precise disorder requiring treatment and its severity, the nature of the formulation, and the route of administration, and will ultimately be at the discretion of the attendant physician or veterinarian. Typically, the compound of formula (1a) will be given for treatment in the range of 0.1 to 200 mg/kg body weight of recipient (animal) per day and more usually in the range of 1 to 100 mg/kg body weight per day. Acceptable daily dosages, may be from about 0.1 to about 200 mg/day, and preferably from about 0.1 to about 100 mg/day.

The administration of compounds of the invention to an animal, particularly a mammal such as a human, may be by way of oral (including sub-lingual), parenteral, nasal, rectal or transdermal administration. Preferably oral administration is employed.

While it is possible that, for use in therapy, a therapeutically effective amount of a compound of formula (la) may be administered as the raw chemical, it is typically presented as the active ingredient of a pharmaceutical composition or formulation. Accordingly, the invention further provides a pharmaceutical composition comprising a compound of formula (1a). The pharmaceutical composition may further comprise one or more pharmaceutically acceptable carriers, diluents, and/or excipients. The carrier(s), diluent(s), and/or excipient(s) must be acceptable in the sense of being compatible with the other ingredients of the formulation and not deleterious to the recipient thereof.

In accordance with another aspect of the invention there is also provided a process for the preparation of a pharmaceutical formulation including admixing a compound of formula (Ia) with one or more pharmaceutically acceptable carriers, diluents, and /or excipients.

Pharmaceutical formulations may be presented in unit dose form containing a predetermined amount of active ingredient per unit dose. Such a unit may contain a therapeutically effective dose of the compound of formula (la) or a fraction of a therapeutically effective dose such that multiple unit dosage forms might be administered at a given time to achieve the desired therapeutically effective dose. Preferred unit dosage formulations are those containing a daily dose of sub-dose, as herein above recited, or an appropriate fraction thereof, of an active ingredient. Furthermore, such pharmaceutical formulations may be prepared by any of the methods well known in the pharmacy art.

Pharmaceutical formulations may be adapted for administration by any appropriate route, for example, by the oral (including buccal or sublingual), rectal, nasal, topical (including buccal, sublingual, or transdermal), vaginal or parenteral (including subcutaneous, intramuscular, intravenous or intradermal) route. Such formulations may be prepared by any method know in the art of pharmacy, for example, by bringing into association the active ingredient with the carrier(s), diluent(s), and/or excipient(s).

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil emulsions. For instance, for oral administration in the form of a tablet or capsule, the active drug component can be combined with an oral, non-toxic pharmaceutically acceptable inert carrier such as ethanol, glycerol, water, and the like. Powders are prepared by comminuting the compound to a suitable fine size and mixing with a similarly comminuted pharmaceutical carrier such as an edible carbohydrate, as, for example, starch or mannitol. Flavoring, preservative, dispersing and coloring agent or dye can also be present.

Capsules are made by preparing a powder mixture as described above, and filling formed gelatin sheaths. Glidants and lubricants, such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or beta-lactose, com sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators (disintegrents) include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granuated by wetting with a binder such as a syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with a free flowing inert carrier and compressed into dablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of active ingredient. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax, or the like. The compound of formula (la) can also be incorporated into a candy, a wafer, and/or tongue tape formulation for administration as a "quick-dissolve" medicament.

Additionally, the present invention comprises a compound of formula (1a) in combination with at least one specie selected from the group consisting of an agent for treating diabetes, an agent for treating hypertension, and an agent for treating arteriosclerosis.

Reagents are commercially available or are prepared according to procedures in the literature.

### Example H1

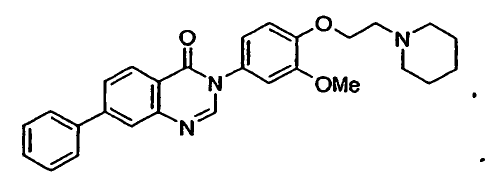

### 3-{3-methoxy-4-[2-(1-piperidinyl)ethoxy]phenyl}-7-phenyl-4(3H)-quinazolinone

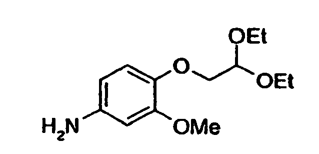

### 4-(2,2-diethoxyethoxy)-3-methoxyaniline

To a solution of 2,2-diethoxyethanol (50 mmol, 6.71 g) was added 60% sodium hydride (50 mmol, 2.0 g) and the solution was stirred for 10 minutes at which point a solution of 2-chloro-5-nitroanisole (50 mmol, 9.38 g) in DMF was added dropwise. The reaction mixture was stirred for 18 h. The DMF was removed by rotary evaporation. The residue was partitioned between ethyl acetate and water. The organic layer was washed with brine. The organic phase was dried over Na₂SO₄ and concentrated. The principal component was purified by silica gel column chromatography. The product was subjected to reduction over 18 h using 1 atm hydrogen and 10% Pd on carbon. After the reaction took up a theoretical amount of hydrogen the catalyst was removed by filtration.

The filtrate was concentrated giving the intermediate (5.21 g, 41%). ¹H NMR (DMSO-D6): δ 1.12 (6H, t), 3.62-3.81 (9H, m), 4.73 (3H, m), 6.05 (1H, d, J = 6.8 Hz), 6.25 (1 H, d, J = 1.8 Hz), 6.63 (1 H, m). LCMS m/z = 278 (m + Na+).

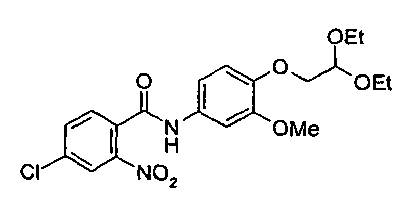

### 4-chloro-N-[4-(2,2-diethoxyethoxy)-3-methoxyphenyl]-2-nitrobenzamide

To a solution of 2-nitro-4-chlorobenzoic acid (5.09, 25.2 mmol) in DCM (100 mL) was added oxalyl chloride (16.8 mL, 34 mmol, 2M in DCM) and DMF (5 drops). The solution was stirred for 1 h and then concentrated by rotary evaporation. The resulting acid chloride was dissolved in DCM. To a solution of 4-(2,2-diethoxyethoxy)-3-methoxyaniline (5.36 g, 21 mmol), triethylamine (4.2 g) and DMAP (0.48 g) in DCM at 0°C was added the acid chloride solution in 4 portions over 15 min. The reaction was stirred for 18 h and the ice was allowed to melt. The solvents were removed by rotary evaporation and the residue partitioned between water and ethyl acetate. The organic layer was washed with brine, dried and concentrated. The product was purified by by silica gel column chromatography affording the intermediate as a yellow solid (7.91 g, 86%). ¹H NMR (DMSO-D6): δ 1.18 (6H, t, J = 6.9 Hz), 3.57 (2H, q, J = 12.1 Hz), 3.67 (2H, q, J = 12.2 Hz), 3.75 (s, 3H), 3.91 (2H, d, J = 5.1 Hz), 4.79 (1H, t, J = 5.1 Hz), 7.00 (1H, m), 7.18 (1H, d), 7.34 (1H, s), 7.82 (1H, d), 7.95 (1 H, d), 8.26 (1H, s), 10.57 (1H, d). LCMS m/z = 461 (m + Na+).

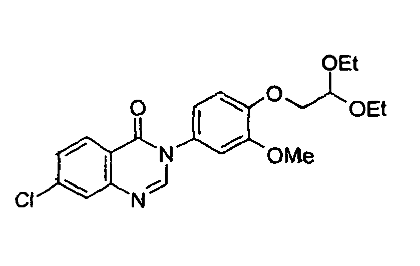

### 7-chloro-3-[4-(2,2-diethoxyethoxy)-3-methoxyphenyl]-4(3H)-quinazolinone

The intermediate from above (2.52 g, 5.74 mmol) was dissolved in dioxane (40 mL), water (40 mL) and ammonium hydroxide (8 mL). To this solution was added sodium hydrosulfite (Na₂S₂O₄,8 eq, 8.0 g). The reaction mixture was stirred for 20 min. The dioxane was removed by rotary evaporation. The aqueous solution was extracted with ethyl acetate. The organic layer was washed with brine and concentrated to give a white solid. The solid was added to triethylorthoformate (30 mL) and the mixture was heated at 100°C for 16 h. The reaction mixture was allowed to cool and then partitioned between dilute aqueous sodium bicarbonate solution and ethyl acetate. The organic layer was concentrated on a rotary evaporator to give a residue that was triturated with petroleum ether. The solids were filtered affording pure product (1.90 g, 79%). ¹H NMR (DMSO-D6): δ 1.15 (6H, t, J = 7.1 Hz), 3.60 (2H, q, J = 12.2 Hz), 3.69 (2H, q, J = 12.3 Hz), 3.79 (s, 3H), 4.00 (2H, d, J = 5.2 Hz), 4.85 (1 H, t, J = 5.2 Hz), 7.05 (1 H, m), 7.15 (1 H, d, J = 8.5 Hz), 7.20 (1 H, d, J = 2.0 Hz), 7.64 (1 H, dd), 7.82 (1 H, d, J = 1.7 Hz), 8.20 (1H, d, J = 8.6 Hz), 8.37 (1 H, s). LCMS m/z = 419 (m + H+).

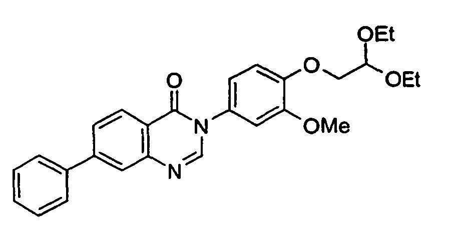

### 3-[4-(2,2-diethoxyethoxy)-3-methoxyphenyl]-7-phenyl-4(3H)-quinazolinone

The aryl chloride from the preceding step (0.80 g, 1.92 mmol), phenyl boronic acid (0.35 g, 1.5 eq), bis-t-butylbiphenylphosphine (114 mg, 20 mol%), palladium acetate (46 mg, 10 mol%) and potassium fluoride (0.334 g, 1 eq) were added to a dry round bottom flask. To this was added degassed dioxane (20 mL) and di-isopropylethylamine (0.32 mL). The reaction mixture was heated at 70°C for 3 h. The reaction mixture was diluted with ether, washed with 1 N NaOH and with water. The aqueous washes were back extracted twice with ether. The organic portion was dried and concentrated. The product was purified by silica gel column chromatography to give the intermediate (0.36 g, 41%). ¹H NMR (DMSO-D6): δ 1.20 (6H, t), 3.60 (2H, q, J = 12.2 Hz), 3.70 (2H, q, J = 12.3 Hz), 3.80 (s, 3H), 4.02 (2H, d, J = 5.1 Hz), 4.85 (1 H, t, J = 5.0 Hz), 7.05 (1 H, m), 7.04-7.22 (3H, m), 7.45-7.58 (3H, m), 7.83-7.94 (4H, m), 8.27 (d, J = 8.4 Hz), 8.36 (1 H, s). LCMS m/z = 461 (m + H+).

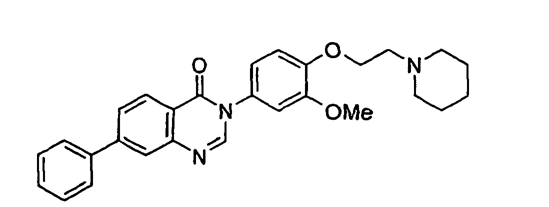

### 3-{3-methoxy-4-[2-(1-piperidinyl)ethoxy]phenyl}-7-phenyl-4(3H)-quinazolinone

To a solution of the diethyl acetal from the preceding step (30 mg, 0.065 mmol) was added DCM (5 mL) and TFA (1 mL). The solution was stirred for 15 min and the solvents were removed by rotary evaporation. The residue was dissolved in THF (3 mL). To this solution was added piperidine (16 mg, 3 eq), and sodium triacetoxyborohydride (41 mg, 3 eq). The reaction mixture was stirred for 18 h. The solvents were removed and the residue partitioned between ethylacetate and aqueous sodium carbonate solution. The organic layer was dried and concentrated. The product was purified by silica gel column chromatography affording the title compound (30 mg, 76%). ¹H NMR (DMSO-D₆) δ 1.20 (1 H, m), 1.63 (1 H, m), 1.85 (1H, m), 2.02 (4H, m), 3.20 (2H, m), 3.40 (2H, m),3.79 (1 H, m), 3.90 (3H, s), 4.64 (2H, m), 7.01 (2H, m), 7.10 (1 H, d, J = 7.2 Hz), 7.54 (3H, m), 7.73-7.78 (3H, m), 8.00 (s, 1H), 8.15 (s, 1H), 8.44 (1H, d, J = 8.2 Hz). LCMS m/z = 456 (m + H+).

### Examples H2-H15 were prepared according to the procedures described in Example H1.

### Example H2

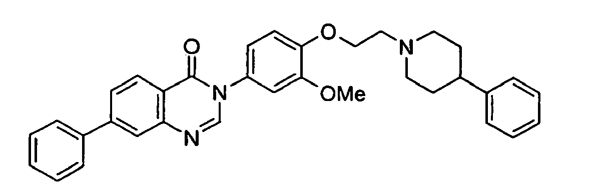

### 3-{3-methoxy-4-[2-(4-phenyl-1-piperidinyl)ethoxy]phenyl}-7-phenyl-4(3H)-quinazolinone

¹H NMR (DMSO-D₆) δ 2.01 (3H, m), 2.60 (2H, m), 3.02 (2H, m), 3.58 (2H, m), 3.90 (5H, m), 4.72 (2H, m), 6.98 (2H, m), 7.02 (1 H, m), 7.28 (5H, m), 7.50 (3H,m), 7.74 (1 H, d, J = 7.1 Hz), 7.79 (1 H, d, J = 2.0 Hz), 7.85 (1 H, d, J = 8.3 Hz), 8.07 (1 H,s), 8.30 (1 H, m), 8.45 (1 H, d, J = 8.4 Hz). LCMS m/z = 532 (m + H+).

### Example H3

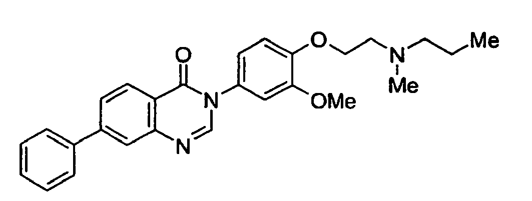

### 3-(3-methoxy-4-{2-[methyl(propyl)amino]ethoxy}phenyl)-7-phenyl-4(3H)-quinazolinone

¹H NMR (DMSO-D₆) δ 0.85 (3H, t, J = 7.2 Hz), 1.51 (2H, m), 2.38 (4H, m), 2.89 (3H, br s), 3.80 (3H, s), 4.17 (2H, br s), 7.15 (3H, m), 7.56 (3H, m), 7.86 (3H, m), 8.36 (3H, m). LCMS m/z = 444 (m + H+).

### Example H4

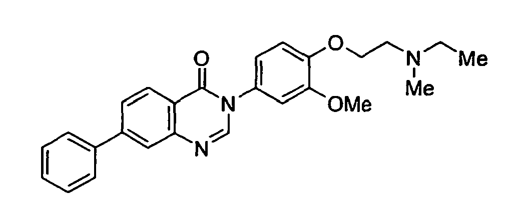

### 3-(4-{2-[ethyl(methyl)amino]ethoxy}-3-methoxyphenyl)-7-phenyl-4(3H)-quinazolinone

¹H NMR (DMSO-D₆) δ 1.08 (3H, t, J = 7.1 Hz), 2.20 (3H, br s), 3.32 (2H, m), 3.44 (2H, m), 3.80 (3H, s), 4.21 (2H, br s), 7.16 (3H, m), 7.51 (3H, m), 7.86 (3H, m), 8.01 (1 H, s), 8.27 (1 H, d, J = 8.4 Hz), 8.36 (1 H, s). LCMS m/z = 430 (m + H+).

### Example H5

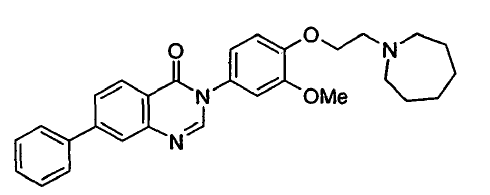

### 3-{4-[2-(1-azepanyl)ethoxy]-3-methoxyphenyl}-7-phenyl-4(3H)-quinazolinone

¹H NMR (DMSO-D₆) δ 1.22 (4H, m), 1.60 (6H, m), 2.82 (4H, m), 3.09 (2H, m), 3.80 (3H, s), 4.14 (2H, br s), 7.14 (3H, m), 7.56 (3H, m), 7.92 (3H, m), 8.20 (1H, d, J = 8.6 Hz), 8.27 (1H, d, J = 8.3 Hz), 8.36 (1 H, s). LCMS m/z = 470 (m + H+).

### Example H6

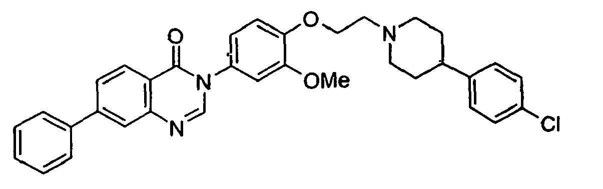

### 3-(4-{2-[4-(4-chlorophenyl)-1-piperidinyl]ethoxy}-3-methoxyphenyl)-7-phenyl-4(3H)-quinazolinone

¹H NMR (CDCl₃) δ 2.00 (2H, m), 2.32 (2H, m), 2.70 (1 H, m), 2.88 (2H, m), 3.44 (2H, m), 3.75 (2H, m), 3.90 (3H, s), 4.52 (2H, br s), 6.92-7.07 (4H, m), 7.29 (2H, m), 7.32 (2H, m), 7.53 (2H, m), 7.73 (2H, m), 8.00 (1 H, s), 8.17 (1H, d, J = 11.6 Hz), 8.30 (1 H, d, J = 8.5 Hz), 8.42 (1 H, d, J = 8.3 Hz). LCMS m/z = 566 (m + H+).

### Example H7

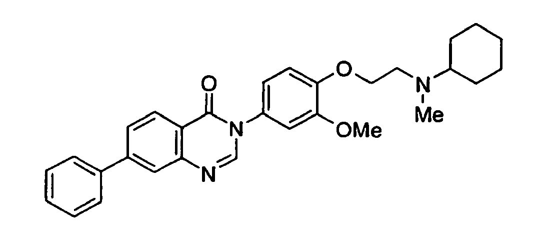

### 3-(4-{2-[cyclohexyl(methyl)amino]ethoxy}-3 methoxyphenyl)-7-phenyl-4(3H)-quinazolinone

¹H NMR (CDCl₃) δ 1.15-2.35 (C₆H₁₁), 2.90 (3H, s), 3.55 (2H, m), 3.89 (3H, s), 4.61 (2H, br s), 6.99 (2H, m), 7.10 (1 H, d, J = 8.2 Hz), 7.53 (3H, m), 7.73-7.83 (3H, m), 8.00 (1 H, d, J = 1.3 Hz), 8.20 (1H, s), 8.42 (1H, d, J = 8.4 Hz). LCMS m/z = 484 (m + H+).

### Example H8

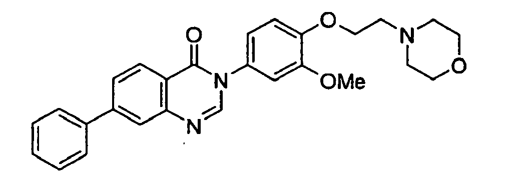

### 3-{3-methoxy-4-[2-(4-morpholinyl)ethoxy]phenyl}-7-phenyl-4(3H-quinazolinone

¹H NMR (CDCl₃) δ 3.21 (2H, m), 3.58 (2H, br s), 3.74 (2H, m), 3.90 (3H, s), 4.05 (2H, m), 4.32 (2H, t), 4.71 (2H, br s), 6.99 (2H, m), 7.10 (1H, d, J = 8.2 Hz), 7.44-7.56 (3H, m), 7.75 (2H, m), 7.81 (1 H, dd, J = 8.3 Hz, J' = 1.5 Hz), 8.02 (1 H, s), 8.19 (1 H, s), 8.41 (1 H, d, J = 8.5 Hz). LCMS m/z = 458 (m + H+).

### Example H9

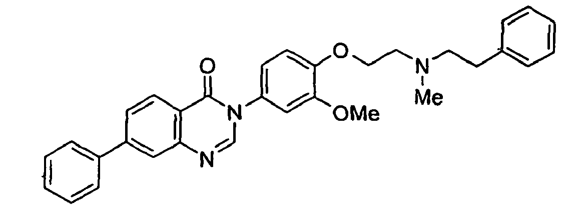

### 3-(3-methoxy-4-{2-[methyl(2-phenylethyl)amino]ethoxy}phenyl)-7-phenyl-4(3H)-quinazolinone

¹H NMR (CDCl₃) δ 2.99 (3H, s), 3.22 (2H, m), 3.38 (2H, m), 3.51 (2H, t, J = 4.7 Hz), 3.82 (3H, s), 4.54 (2H, t, J = 4.7 Hz), 6.99 (2H, m), 7.10 (1 H, d, J = 8.2 Hz), 7.26-7.36 (5H, m), 7.44-7.56 (3H, m), 7.75 (2H, m), 7.82 (1H, dd, J = 8.3 Hz, J' = 1.6 Hz), 8.01 (1H, d, J = 1.5 Hz), 8.17 (1H, s), 8.42 (1H. d, J = 8.2 Hz). LCMS m/z = 506 (m + H+).

### Example H10

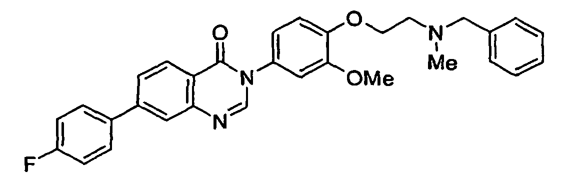

### 3-(4-{2-[benzyl(methyl)amino]ethoxy}-3-methoxyphenyl)-7-(4-fluorophenyl)-4(3H)-quinazolinone

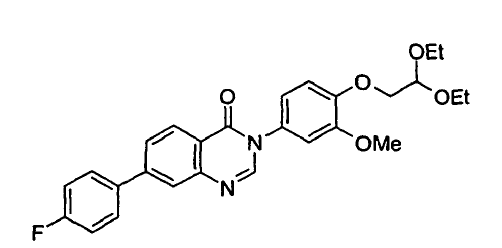

### 3-[4-(2,2-diethoxyethoxy)-3-methoxyphenyl]-7-(4-fluorophenyl)-4(3H)-quinazolinone

¹H NMR (DMSO-D6) δ 1.08 (6H, t), 3.59-3.65 (4H, m), 3.80 (3H, s), 4.02 (2H, d), 4.85 (1 H, t, J = 5.1 Hz), 7.03-7.16 (3H, m), 7.38 (2H, m), 7.92 (3H, m), 8.00 (1 H, s), 8.26 (1 H, d, J = 8.4 Hz), 8.36 (1 H, s). LCMS m/z = 501 (m + Na+).

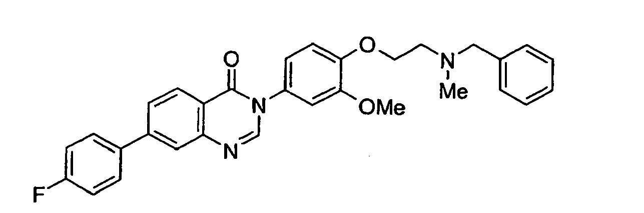

### 3-(4-{2-[benzyl(methyl)amino]ethoxy}-3-methoxyphenyl)-7-(4-fluorophenyl)-4(3H)-quinazolinone

¹H NMR (DMSO-D6) δ 2.75 (3H, s), 3.37 (2H, s), 3.91 (3H, s), 4.25 (2H, s), 4.55 (2H, s), 6.96-7.09 (3H, m), 7.20-7.28 (2H, m), 7.44 (3H, m), 7.63-7.77 (5H, m), 7.95 (1H, d, J = 1.5 Hz), 8.17 (1 H, s), 8.42 (1 H, d, J = 8.3 Hz). LCMS m/z=510(m+H+).

### Example H11

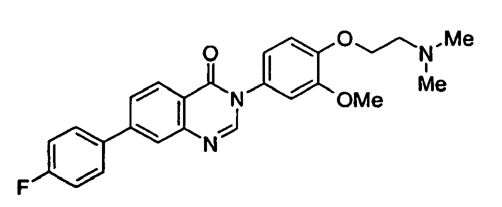

### 3-{4-[2-(dimethylamino)ethoxy]-3-methoxyphenyl}-7-(4-fluorophenyl)-4(3H)-quinazolinone

¹H NMR (DMSO-D6) δ 2.51 (6H, s), 3.44 (2H, s), 3.79 (3H, s), 4.35 (2H, br s), 7.04-7.20 (3H, m), 7.38 (2H, m), 7.91-8.01 (4H, m), 8.27 (1H, d, J = 8.2 Hz), 8.36 (1 H, s). LCMS m/z = 434 (m + H+).

### Example H12

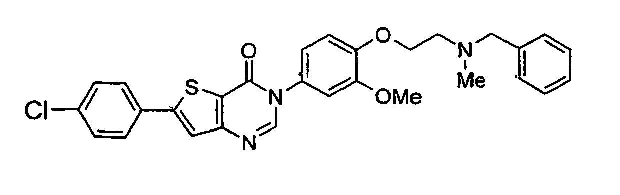

### 3-(4-{2-[benzyl(methyl)amino]ethoxy}-3-methoxyphenyl)-6-(4-chlorophenyl)thieno[3,2-d]pyrimidin-4(3H)-one.

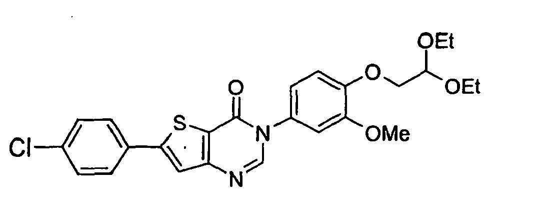

### 6-(4-chlorophenyl)-3-[4-(2,2-diethoxyethoxy)-3-methoxyphenyl]thieno[3,2-d]pyrimidin-4(3H)-one.

To a solution of methyl 3-amino-5-(4-chlorophenyl)-2-thiophenecarboxylate (2.0 g, 7.47 mmol, Maybridge, Inc) in ethanol (20 mL) was added dimethylformamide dimethyl acetal (2.5 mL, 2.5 eq, Aldrich). The reaction mixture was refluxed in a 90°C oil bath for 3 h at which point the solvents were removed by rotary evaporation. Coevaporation with toluene removed the excess dimethylformamide dimethyl acetal. To the resulting residue was added ethanol (20 mL) and 4-(2,2-diethoxyethoxy)-3-methoxyaniline (2.3 g, 1.2 eq, Example H1). The reaction mixture was heated to 100°C for 36 hours. The solvent was removed by rotary evaporation and the product purified by column chromatography followed by recrystallization from hot MeOH yielding the intermediate (0.82 g, 22%). ¹H NMR (CDCl₃) δ 1.22 (6H, t), 3.63-3.83 (4H, m), 3.90 (3H, s), 4.14 (2H, d, J = 5.3 Hz), 4.92 (1 H, t, J = 5.2 Hz), 6.97 (2H), 7.06 (1H, d, J = 8.5 Hz), 7.46 (2H, d, J = 8.5 Hz), 7.58 (1H, s), 7.68 (2H, d, J = 8.4 Hz), 8.24 (1 H, br, s). LCMS m/z = 501 (m + H+).

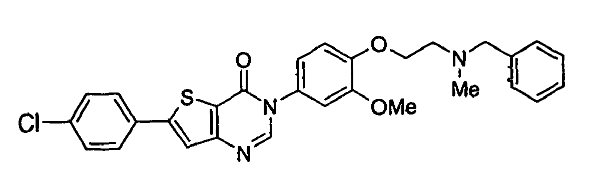

### 3-(4-{2-[benzyl(methyl)amino]ethoxy}-3-methoxyphenyl)-6-(4-chlorophenyl)thieno[3,2-d]pyrimidin-4(3H)-one.

Reductive amination of the product from the preceding step with N-methyl-N-benzylamine according to the procedure described in Example H1 provided the title compound.
¹H NMR (CDCl₃) δ 2.89 (2H, m), 3.90 (3H, s), 4.43 (2H, m), 4.68 (2H, m), 7.02 (2H), 7.10 (1 H, d, J = 8.5 Hz), 7.28 (2H, s), 7.48 (4H, m), 7.48 (1H, s), 7.67 (2H, d, J = 8.3 Hz), 7.73 (1 H, m), 8.19 (s, 1H). LCMS m/z = 532 (m + H+).

Examples H13-H15 were prepared according to the procedures described in Example H12.

### Example H13

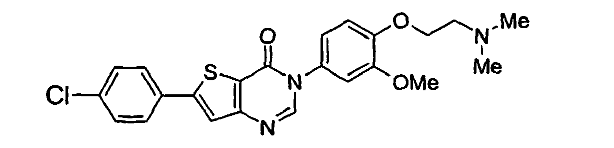

### 6-(4-chlorophenyl)-3-{4-[2-(dimethylamino)ethoxy]-3-methoxyphenyl}thieno[3,2-d]pyrimidin-4(3H)-one

¹H NMR (CDCl₃) δ 3.03 (6H, s), 3.90 (3H, s), 3.78 (2H, m), 3.91 (3H, s), 4.63 (2H, m), 7.01 (2H, m), 7.11 (1 H, d, J = 8.3 Hz), 7.48 (2H, d, J = 8.6 Hz), 7.61 (1H, s), 7.68 (2H, d, J = 8.6 Hz), 8.26 (1 H, s). LCMS m/z = 456 (m + H+).

### Example H14

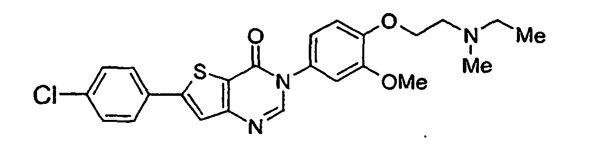

### 6-(4-chlorophenyl)-3-(4-{2-[ethyl(methyl)amino]ethoxy}-3-methoxyphenyl)thieno[3,2-d]pyrimidin-4(3H)-one

¹H NMR (CDCl₃) δ 1.54 (5H, m), 2.97 (3H, s), 3.51 (2H, m), 3.90 (3H, s), 4.66 (2H, m), 6.98 (2H, m), 7.11 (1H, d, J = 8.4 Hz), 7.48 (2H, d, J = 8.6 Hz), 7.56 (1H, s), 7.68 (2H, d, J = 8.4 Hz), 8.15 (1H, s). LCMS m/z = 470 (m + H+).

### Example H15

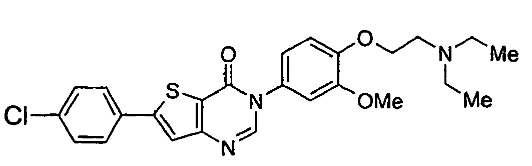

### 6-(4-chlorophenyl)-3-{4-[2-(diethylamino)ethoxy]-3-methoxyphenyl}thieno[3,2-d]pyrimidin-4(3H)-one

¹H NMR (CDCl₃) δ 1.54 (6H, m), 3.027 (4H, m), 3.61 (2H, m), 3.89 (3H, s), 4.55 (2H, m), 6.99 (2H, m), 7.11 (1 H, d, J = 8.4 Hz), 7.47 (2H, d, J = 8.4 Hz), 7.58 (1 H, s), 7.68 (2H, d, J = 8.5 Hz), 8.21 (1 H, s). LCMS m/z = 484 (m + H+).

### Example H16

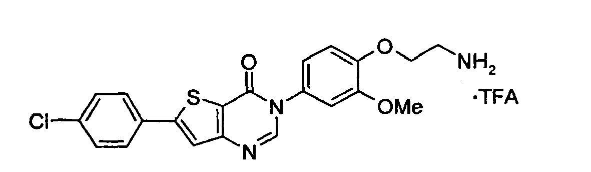

### 3-[4-(2-aminoethoxy)-3-methoxyphenyl]-6-(4-chlorophenyl)thieno[3,2-d]pyrimidin-4(3H)-one trifluoroacetate salt

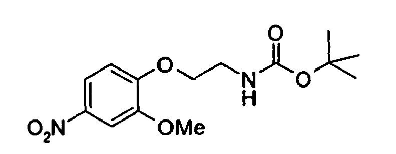

### tert-butyl 2-(2-methoxy-4-nitrophenoxy)ethylcarbamate

To a solution of 4-nitroguiacol (5.0 g, 30 mmol), *tert*-butyl-2-hydroxyethylcarbamate (5.3 g, 33 mmol) and triphenylphosphine (9.8 g, 37.5 mmol) in THF (50 mmol) was added dropwise diisopropylazodicarboxylate (7.4 mL, 37.5 mmol). The reaction mixture was stirred for 1 day. The solvent was removed by rotary evaporation and the residue was dissolved in chloroform and loaded onto a silica gel column. The product was eluted with 50% ethylacetate in petroleum ether giving the product as a white solid. ¹H NMR (CDCl₃) δ 1.46 (9H, s), 3.63 (2H, dd, J = 5.4 Hz, J' = 10.2 Hz), 3.96 (3H, s), 4.17 (2H, dd, J = 5.3 Hz, J' = 10.4 Hz), 6.95 (1 H, d, J = 8.9 Hz), 7.76 (1 H, d, J = 2.6 Hz), 7.90 (1 H, dd, J = 9.0 Hz, J' = 2.6 Hz). LCMS m/z = 335 (m + Na+).

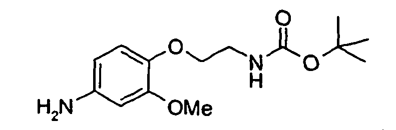

### tert-butyl 2-(4-amino-2-methoxyphenoxy)ethylcarbamate

The material from the preceding step was dissolved in ethanol and subjected to reduction with 1 atm hydrogen and catalytic 10% Pd on carbon dust. The mixture was stirred overnight. The catalyst was removed by filtration and the filtrate was concentrated and the product purified by silica gel column chromatography giving the product (5.7 g, 68% yield). ¹H NMR (CDCl₃) δ 1.46 (9H, s), 3.49 (2H, m), 3.83 (3H, s), 4.01 (2H, m), 5.30 (2H, br s), 6.60 (2H, m), 6.80 (1 H, d, J = 8.4 Hz), LCMS m/z = 305 (m + Na+).

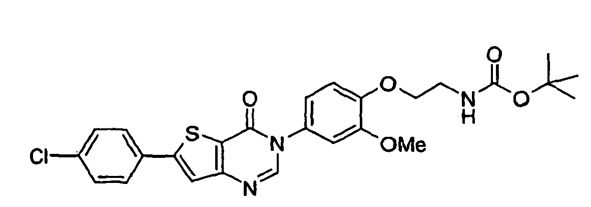

### tert-butyl 2-(4-(6-(4-chlorophenyl)-4-oxothieno(3,2-d]pyrimidin-3(4H)-yl)-2-methoxyphenoxy]ethylcarbamate

¹H NMR (CDCl₃) δ 1.48 (9H, s), 3.62 (2H, m), 3.92 (3H, s), 4.16 (2H, m), 5.15 (1H, br s), 6.93-7.08 (3H, m), 7.47 (1H, d, J = 8.4 Hz), 7.56 (1H, s), 7.68 (1H, d, J = 8.6 Hz), 8.16 (1 H, s). LCMS m/z = 550 (m + Na+). Calcd: C, 59.14, H, 4.96, N, 7.96. Found: C, 58.85, H, 5.03, N, 7.85.

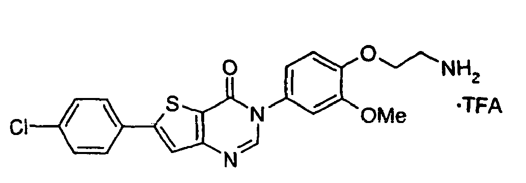

### 3-[4-(2-aminoethoxy)-3-methoxyphenyl]-6-(4-chlorophenyl)thieno[3,2-d]pyrimidin-4(3H)one trifluoroacetate salt

To the material from the preceding step (100 mg, 0.19 mmol) was added DCM (1 mL) and trifluoroacetic acid (1 mL). The reaction mixture was stirred for 20 min and the solvents removed by rotary evaporation and the residue pumped under a high vacuum. This yielded the title compound as a tan solid (105 mg, 100% yield). ¹H NMR (CDCl₃) δ 3.27 (2H, m), 3.82 (3H, s), 4.24 (2H, m), 7.09-7.29 (3H, m), 7.61 (1 H, d, J = 8.5 Hz), 7.09-8.03 (3H, m), 8.41 (1H, s). LCMS m/z = 428 (m + H+).

### Example H17

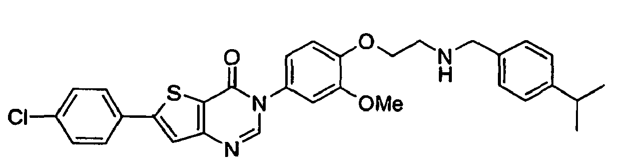

### 6-(4-chlorophenyl)-3-(4-{2-[(4-isopropylbenzyl)amino]ethoxy}-3-methoxyphenyl)thieno[3,2-d]pyrimidin-4(3H)-one

The title compund from Example H16 (30 mg, 0.055 mmol) was dissolved in DMF (1 mL). To this solution was added 4-isopropylbenzaldehyde (10 mg, 1.2 eq) and sodium triacetoxyborohydride (23 mg, 2 eq). The reaction mixture was stirred 18 h and then loaded onto a silica gel column. The product eluted with 20% ethanol /DCM giving the title compound (12 mg, 40%). ¹H NMR (CDCl₃) δ 1.25 (6H, d), 2.90 (1 H, m), 3.22 (2H, m), 3.85 (3H, s), 4.36 (2H, m), 6.91-7.09 (3H, m), 7.25 (3H, m), 7.46 (3H, m), 7.53 (1H, s). 7.67 (2H, m), 8.14 (1 H, s). LCMS m/z = 560 (m + H+).

### Example H18

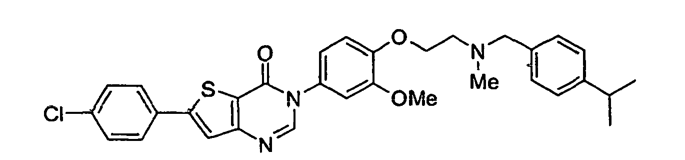

### 6-(4-chlorophenyl)-3-(4-{2-[(4-isopropylbenzyl)(methyl)amino]ethoxy}-3-methoxyphenyl)thieno[3,2-d]pyrimidin-4(3H)-one

The product from Example H17 (12 mg) was dissolved in 37% formaldehyde (1 mL) and 88% formic acid (1 mL). The solution was refluxed for 2 h. The solvents were removed by rotary evaporation and the product was purified by silica gel column chromatography giving the title compound (10 mg, 81%). ¹H NMR (CDCl₃) δ 1.25 (6H, d), 2.40 (3H, s), 2.90 (2H, m), 3.65 (2H, m), 3.90 (3H, s), 4.21 (2H, m), 6.91-7.00 (3H, m), 7.19-7.34 (4H, m), 7.46 (2H, d, J = 8.4 Hz). 7.55 (1 H, s), 7.68 (2H, d, J = 8.6 Hz), 8.15 (1H, s). LCMS m/z = 574 (m + H+).

### Examples H19 -H24 were prepared according to the procedures for H17 and H18.

### Example H19

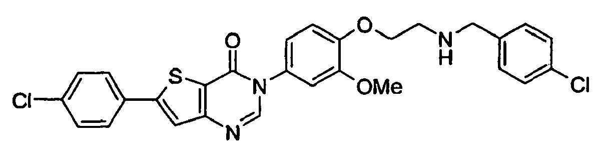

### 3-(4-{2-[(4-chlorobenzyl)amino]ethoxy}-3-methoxyphenyl)-6-(4-chlorophenyl)thieno[3,2-d]pyrimidin-4(3H)-one

¹H NMR (CDCl₃) δ 3.85 (2H, m), 3.95 (3H, s), 4.36 (2H, m), 4.43 (2H, m), 6.91-7.17 (3H, m), 7.28-7.53 (5H, m), 7.57-7.84 (4H, m), 8.26 (1 H, s). LCMS m/z = 552 (m + H+).

### Example H20

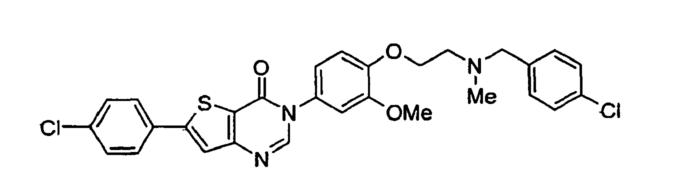

### 3-(4-{2-[(4-chlorobenzyl)(methyl)amino]ethoxy}-3-methoxyphenyl)-6-(4-chlorophenyl)thieno[3,2-d]pyrimidin-4(3H)-one

¹H NMR (CDCl₃) δ 2.38 (3H, s), 2.92 (2H, t, J = 6.1 Hz), 3.64 (2H, s), 3.90 (3H, s), 4.21 (2H, t, J = 6.1 Hz), 6.96 (3H, m), 7.32 (4H, m), 7.46 (2H, d, J = 8.6 Hz). 7.55 (1H, s), 7.68 (2H, d, J = 8.6 Hz), 8.16 (1 H, s). LCMS m/z = 566 (m + H+).

### Example H21

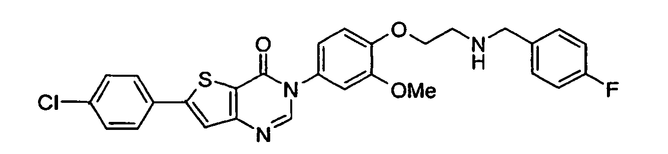

### 6-(4-chlorophenyl)-3-(4-{2-[(4-fluorobenzyl)amino]ethoxy}-3-methoxyphenyl)thieno[3,2-d]pyrimidin-4(3H)-one

¹H NMR (CDCl₃) δ 2.10 (2H, s), 3.28 (2H, m), 3.83 (3H, s), 4.13 (2H, s), 4.30 (2H, m), 6.91-7.12 (5H, m), 7.44-7.53 (5H, m), 7.66 (2H, d, J = 8.6 Hz), 8.12 (1H, s). LCMS m/z = 558 (m + Na+).

### Example H22

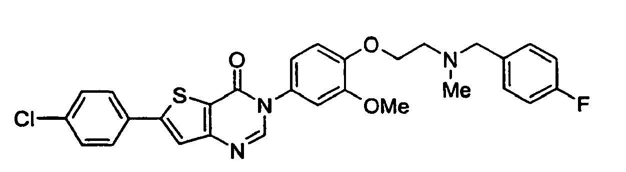

### 6-(4-chlorophenyl)-3-(4-{2-[(4-fluorobenzyl)(methyl)amino]ethoxy}-3-methoxyphenyl)thieno[3,2-d]pyrimidin-4(3H)-one

¹H NMR (CDCl₃) δ 2.39 (3H, s), 2.95 (2H, t, J = 6.1 Hz), 3.67 (2H, s), 3.90 (3H, s), 4.22 (2H, t, J = 6.1 Hz), 6.91-7.06 (5H, m), 7.32 (2H, m), 7.46 (2H, d, J = 8.6 Hz). 7.55 (1 H, s), 7.68 (2H, d, J = 8.6 Hz), 8.16 (1 H, s). LCMS m/z = 550 (m + H+).

### Example H23

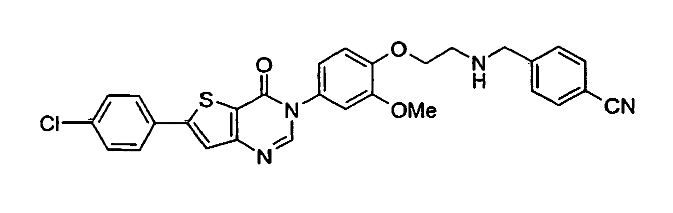

### 4-[({2-[4-(6-(4-chlorophenyl)-4-oxothieno[3,2-d]pyrimidin-3(4H)-yl)-2-methoxyphenoxy]ethyl}amino)methyl]benzonitrile

¹H NMR (CDCl₃) δ 3.10 (2H, d, J = 5.1 Hz), 3.90 (3H, s), 3.98 (2H, s), 4.22 (2H, d. J = 5.1 Hz), 6.93-7.05 (3H, m), 7.46-7.56 (5H, m), 7.67 (3H, m), 8.16 (1 H, s). LCMS m/z = 565 (m + Na+).

### Example H24

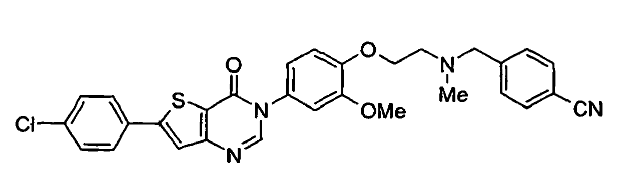

### 4-{[{2-[4-(6-(4-chlorophenyl)-4-oxothieno[3,2-d]pyrimidin-3(4H)-yl)-2-methoxyphenoxy]ethyl}(methyl)amino]methyl}benzonitrile

¹H NMR (CDCl₃) δ 2.38 (3H, s), 2.95 (2H, t, J = 5.9 Hz), 3.73 (2H, s), 3.90 (3H, s), 4.22 (2H, t, J = 6.0 Hz), 6.93-7.01 (3H, m), 7.45-7.56 (5H, m), 7.62-7.71 (4H, m), 8.16 (1H, s). LCMS m/z = 579 (m + Na+).

### Example H25

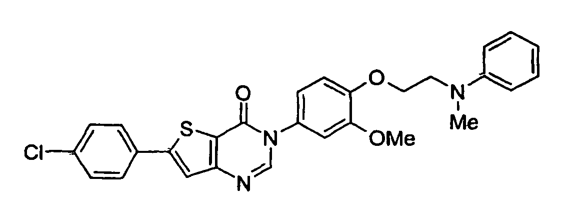

### 6-(4-chlorophenyl)-3-{3-methoxy-4-[2-(methylanilino)ethoxy]phenyl}thieno[3,2-d]pyrimidin-4(3H)-one

To a solution of 6-(4-chlorophenyl)3-(4-hydroxy-3-methoxyphenyl)thieno[3,2-*d*]pyrimidin-4(3*H*)-one (96 mg, 0.25 mmol, the preparation of which may be found in Example K1) in DMF was added 2-(methylanilino)ethyl toluenesulfonate (153 mg, 0.50 mmol) and cesium carbonate (0.24 g, 0.75 mmol) and the mixture was stirred with heating at 75°C for 12 h. The reaction was allowed to cool and a 10 mL solution of 20% water/ ethanol was added. The resulting precipitate was filtered and dried in a vacuum oven to give the title compound (104 mg, 80%). ¹H NMR (DMSO-D6) δ 3.01 (3H, s), 3.78 (5H, m), 4.18 (2H, t, J = 3.7 Hz), 6.63 (1H, t, J = 7.3Hz), 6.78 (2H, d, J = 7.2 Hz), 7.02-7.20 (5H, m), 7.60 (2H, d, J = 8.6 Hz), 7.96 (3H, m), 8.39 (1 H, s). LCMS m/z = 518 (m + H+). Calcd for C₂₈H₂₄CIN₃O₃Sx1H₂O : C, 62.74, H, 4.89, N, 7.84. Found: C, 62.76, H, 4.65, N, 7.82.

### Example H26

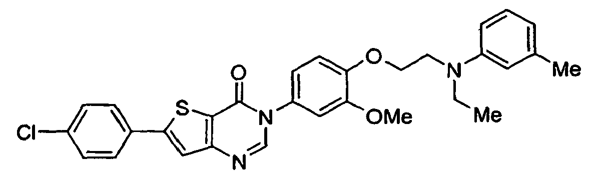

### 6-(4-chlorophenyl)-3-{4-[2-(ethyl-3-methylanilino)ethoxy]-3-methoxyphenyl}thieno[3,2-d]pyrimidin-4(3H)-one

To a solution of 6-(4-chlorophenyl)-3-(4-hydroxy-3-methoxyphenyl)thieno[3,2-*d*]pyrimidin-4(3*H*)-one (96 mg, 0.25 mmol, the preparation of which can be found in the section detailing the preparation of Example K1) in DMF was added *N*-(2-chloroethyl)-*N*-ethyl-3-methylaniline (99 mg, 0.50 mmol) and cesium carbonate (0.24 g, 0.75 mmol) and the mixture was stirred with heating at 75°C for 12 h. The reaction was allowed to cool and 10 mL solution of 20% water/ ethanol was added. The resulting precipitate was filtered and dried in a vacuum oven to give the title compound (58 mg, 42%). ¹H NMR (DMSO-D6) δ 1.13 (3H, t, J = 7.0 Hz), 2.24 (3H, s), 3.45 (2H, q, J = 7.0 Hz), 3.70 (2H, d, J = 5.8 Hz), 3.79 (3H, s), 4.16 (2H, d, J = 5.8 Hz), 6.43 (1 H, t, J = 7.3Hz), 6.57 (2H, m), 7.02-7.20 (4H, m), 7.60 (2H, d, J = 8.6 Hz), 7.96 (3H, m), 8.39 (1 H, s). LCMS m/z = 546 (m + H+).

### Example H27

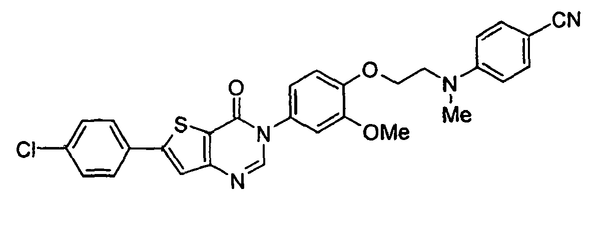

### 4-[{2-[4-(6-(4-chlorophenyl)-4-oxothieno[3,2-d]pyrimidin-3(4H)-yl)-2-methoxyphenoxy]ethyl}(methyl)amino]benzonitrile

To a solution of 6-(4-chlorophenyl)-3-(4-hydroxy-3-methoxyphenyl)thieno[3,2-*d*]pyrimidin-4(3*H*)-one (96 mg, 0.25 mmol, the preparation of which can be found in the section detailing the preparation of Example K1) in DMF was added 2-[4-cyano(methyl)anilino]ethyl p-toluenesulfonate (165 mg, 0.50 mmol) and cesium carbonate (0.24 g, 0.75 mmol) and the mixture was stirred with heating at 75°C for 12 h. The reaction was allowed to cool and 10 mL solution of 20% water/ ethanol was added. The resulting precipitate was filtered and dried in a vacuum oven to give the title compound (133 mg, 98%). ¹H NMR (DMSO-D6) δ 3.10 (3H, s), 3.76 (3H, s), 3.87 (2H, t, J = 3.7 Hz), 4.21 (2H, t, J = 3.7 Hz), 6.87 (1 H, d, J = 7.3Hz), 7.02-7.20 (3H, m), 7.58 (4H, m), 7.99 (3H, m), 8.38 (1H, s). LCMS m/z = 543 (m + H+). Calcd for C₂₉H₂₃ClN₄O₃Sx1HCl: C, 60.11, H, 4.17, N, 9.67. Found: C, 59.78, H, 4.18, N, 9.44.

### Example H28

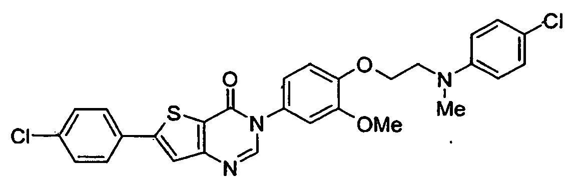

### 3-(4-{2-[4-chloro(methyl)anilino]ethoxy}-3-methoxyphenyl)-6-(4-chlorophenyl)thieno[3,2-d]pyrimidin-4(3H)-one

To a solution of 6-(4-chlorophenyl)-3-(4-hydroxy-3-methoxyphenyl)thieno[3,2-*d*]pyrimidin-4(3*H*)-one (96 mg, 0.25 mmol, the preparation of which can be found in the section detailing the preparation of Example K1) in DMF was added 2-[4-chloro(methyl)anilino]ethyl p-toluenesulfonate (170 mg, 0.50 mmol) and cesium carbonate (0.24 g, 0.75 mmol) and the mixture was stirred with heating at 75°C for 12 h. The reaction was allowed to cool and 10 mL solution of 20% water/ ethanol was added. The resulting precipitate was filtered and dried in a vacuum oven to give the title compound (136 mg, 98%). ¹H NMR (DMSO-D6) δ 3.00 (3H, s), 3.77 (5H, m), 4.21 (2H, m), 6.81 (2H, d, J = 9.0 Hz), 7.02-7.20 (5H, m), 7.58 (2H, d, J = 8.6 Hz), 7.95 (3H, m), 8.39 (1H, s). Calcd for C₂₈H₂₃Cl₂N₃O₃Sx3HCl: C, 50.81, H, 3.96, N, 6.35. Found: C, 50.91, H, 3.95, N, 6.23.

### Example H29

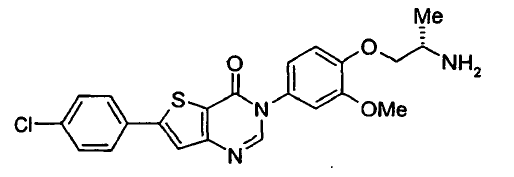

### 3-(4-{[(2S)-2-aminopropyl]oxy}-3-methoxyphenyl)-6-(4-chlorophenyl)thieno[3,2-d]pyrimidin-4(3H)-one

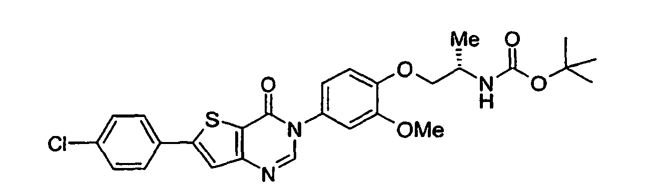

### tert-butyl (1S)-2-[4-(6-(4-chlorophenyl)-4-oxothieno[3,2-d]pyrimidin-3(4H)-yl)-2-methoxyphenoxy]-1-methylethylcarbamate

To a solution of 6-(4-chlorophenyl)-3-(4-hydroxy-3-methoxyphenyl)thieno[3,2-*d*]pyrimidin-4(3*H*)-one (0.29 g, 0.75 mmol, the preparation of which can be found in the section detailing the preparation of Example K1) in DMF was added (2*S*)-2-[(*tert*-butoxycarbonyl)amino]propyl toluenesulfonate (0.5 g, 1.50 mmol) and cesium carbonate (0.72 g, 2.25 mmol) and the mixture was stirred with heating at 75°C for 12 h. The reaction was allowed to cool and 25 mL of a solution of 20% water/ ethanol was added. The resulting precipitate was filtered and dried in a vacuum oven to give the product (200 mg, 49%). ¹H NMR (DMSO-D6) δ 1.15 (3H, d, J = 6.1 Hz), 1.40 (9H, s), 3.79 (6H, m), 7.22-7.20 (3H, m), 7.59 (2H, d, J = 8.4 Hz), 7.95-7.99 (3H, m), 8.40 (1 H, s). LCMS m/z = 542 (m + H+).

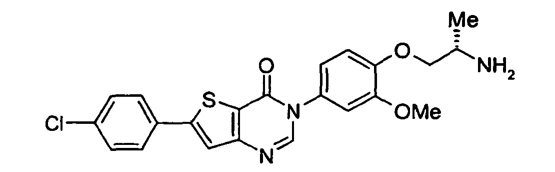

### 3-(4-{[(2S)-2-aminopropyl]oxy}-3-methoxyphenyl)-6-(4-chlorophenyl)thieno[3,2-d]pyrimidin-4(3H)-one

To the material from the preceding step (62 mg, 0.11 mmol) was added DCM (4 mL) and trifluoroacetic acid (4 mL) and the mixture was stirred at RT for 20 min. The solvents were removed by rotary evaporation. To the resulting gum was added 1 N NaOH and the solution was extracted with ether 3x. The organic extracts were dried and concentrated to yield the title compound (44 mg, 87%). ¹H NMR (methanol -D4) δ 1.25 (3H, d, J = 6.5 Hz), 3.91 (3H, s), 4.05 (1 H, m), 7.05 (1 H, m), 7.15 (2H, m), 7.53 (2H, d, J = 8.5 Hz), 7.73 (1 H, s), 7.84 (2H, d, J = 8.5 Hz), 8.370 (1 H, s). LCMS m/z = 442 (m + H+).

### Example H30

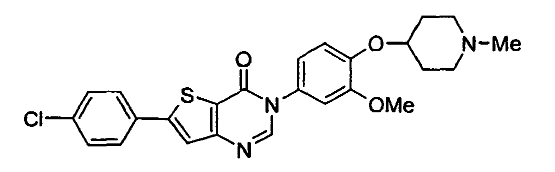

### 6-(4-chlorophenyl)-3-{3-methoxy-4-((1-methyl-4-piperidinyl)oxy]phenyl}thieno[3,2-d]pyrimidin-4(3H)-one

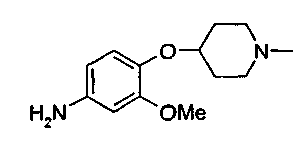

### 3-methoxy-4-[(1-methyl-4-piperidinyl)oxy]aniline

To a solution of 4-[4-nitro-2-methoxyphenoxy]-1-methylpiperidine (2.17 g, 8.15 mmol) in ethanol (150 mL) was added 10% palladium on carbon (400 mg). The mixture was subjected to 1 atm hydrogen with stirring overnight. Removal of the catalyst by filtration and evaporation of the solvent yielded the product (1.75 g, 91%). ¹H NMR (CDCl₃) δ 1.81-2.04 (4H, m), 2.34 (5H, m), 2.81 (2H, m), 3.56 (2H, br s), 3.80 (3H, s), 4.04 (1H, s), 6.20 (1 H, dd, J = 2.7 Hz, J' = 8.4 Hz), 6.29 (1H,d, J = 2.5 Hz), 6.77 (1 H, d, J = 8.3 Hz). LCMS m/z = 237 (m + H+).

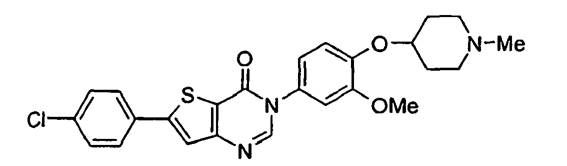

### 6-(4-chlorophenyl)-3-{3-methoxy-4-[(1-methyl-4-piperidinyl)oxy]phenyl}thieno[3,2-d]pyrimidin-4(3H)-one

To a solution of methyl 3-amino-5-(4-chlorophenyl)-2-thiophenecarboxylate (0.27 g, 1.0 mmol, Maybridge, Inc) in ethanol (5 mL) was added dimethylformamide dimethyl acetal (0.33 mL, 2.5 eq, Aldrich). The reaction mixture was refluxed in a 90°C oil bath for 3 h at which point the solvents were removed by rotary evaporation. Coevaporation with toluene removed the excess dimethylformamide dimethyl acetal. To the resulting residue was added ethanol (5 mL) and the product from the preceding step (3-methoxy-4-[(1-methyl-4-piperidinyl)oxy]aniline, 0.28 g, 1.2 eq). The reaction mixture was heated to 100°C for 24 hours. Upon cooling to RT a precipitate formed which was filtered and washed with ethanol. The gray powder was dried in a vacuum oven yielding the title compound (0.10 g, 21%). ¹H NMR (CDCl₃) δ 1.68 (2H, m), 1.93 (2H, m), 2.18, 5H, m), 2.64 (2H, m), 3.78 (3H, s), 4.36 (1 H, m), 7.04 (1 H, dd, J = 8.4 Hz, J' = 2.3 Hz), 7.20 (2H, m), 7.58 (2H, d, J = 8.6 Hz), 7.98 (3H, m), 8.41 (1H, s). LCMS m/z = 482 (m + H+). CalCd for C₂₅H₂₄Cl₂N₃O₃SCl x 0.65 H₂O: C, 60.82, H, 5.17, N, 8.51. Found: C, 60.82, H, 5.04, N, 8.41.

### Example I1

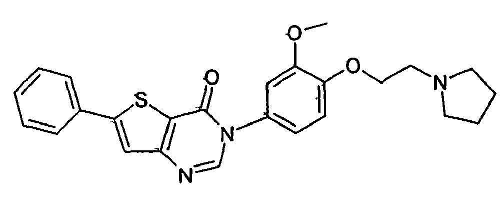

### 3-[3-Methoxy-4-(2-pyrrolidin-1-ylethoxy)phenyl]-6-phenylthieno[3,2-d]pyrimidin-4(3H)-one

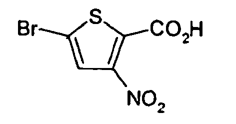

### 5-Bromo-3-nitrothiophene-2-carboxylic acid

5-Bromo-3-nitrothiophene-2-carbafdehyde (0.5953 g, 2.5012 mmol, prepared according to Sone, C; Matsuki, *Y Bull Chem* Soc Japan, **1963,** *36(5),* 618-20.) was dissolved in acetone (5 mL) and Jones Reagent (1.5 mL) was added. The reaction was stirred at RT for 1 h. The reaction was diluted with water (30 mL) and extracted with EtOAc (3 x 30 mL). The combined organics were washed with water (4 x 100 mL), dried over MgSO₄ filtered and concentrated to give 0.4993 g (1.9892 mmol, 80%) of the product as a light yellow solid.
1 H NMR (CDCl₃) δ 7.58 (s, 1H). LRMS M-H 250.

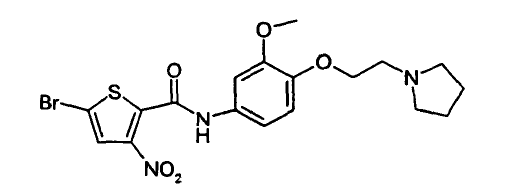

### 5- Bromo-N-[3-methoxy-4-(pyrrolidin-1ylethoxy)phenyl]-3-nitrothiophene-carboxamide

5-Bromo-3-nitrothiophene-2-carboxylic acid (0.4993 g, 1.9892 mmol) and 3-methoxy-4-(2-pyrrolidin-1-eylethoxy)aniline (0.4695 g, 1.9892 mmol) were dissolved in CH₂Cl₂ (10 mL). HOBT (0.2960 g, 2.1881 mmol) and Hunig's base (0.866 mL, 4.9730 mmol) were added. The reaction was stirred at RT for 10 min and then EDC (0.4576 g, 2.3870 mmol) was added. The reaction was stirred at RT for 72 h. The reaction was washed with water (2 X 30 mL), dried over MgSO₄, filtered and concentrated. The crude material was purified on a chromatatron (95:5 CH₂Cl₂:MeOH, with 0.1 % NEt₃) to give 0.2926 g (0.6239 mmol, 31%) of the product as a dark red gum. 1 H NMR (CDCl₃) δ 10.58(s, 1 H), 7.70 (s, 1H). 7.42 (s, 1H), 7.06 (dd, 1 H J = 2.4 Hz, 8.6 Hz), 6.88 (d, 1H, J = 8.6 Hz), 4.16 (t, 2H, J = 6.2 Hz), 3.90 (s, 3H), 3.00 (t, 2H, J = 6.2 Hz), 2.8 2.6 (bs, 4H), 1.9 -1.7 (bs, 4H).

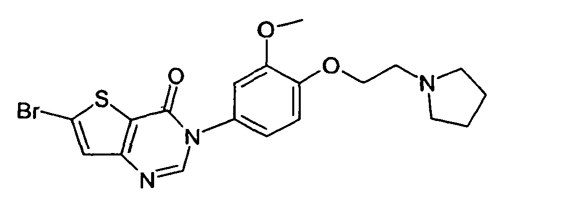

### 6-Bromo-3-[3-methoxy-4-(2pyrrolidin-1-ylethoxy)phenyl]thieno[3,2-d]pyrimidin-4(3H)-one

5- Bromo-N-[3- methoxy- 4- (pyrrolidin-1 ylethoxy)phenyl]- 3- nitrothiophene-carboxamide (0.2926 g, 0.6239 mmol) was dissolved in ethanol (10 mL). Tin chloride monohydrate (0.7038 g, 3.1195 mmol) was added and the reaction was heated to reflux. Stirred for 30 min, cooled to RT and concentrated. Ethyl acetate (20 mL) and a saturated solution of Rochelle's salts (20 mL) were added and the mixture was stirred for 2 h. Organics were removed and dried over MgSO₄, filtered and concentrated. The residue was taken up in formic acid (5 mL), stirred at reflux for 1 h, and then concentrated to give 0.1182 g (0.2633 mmol, 42%) of the product. 1 H NMR (CDCl₃) δ 8.03 (s, 1H), 7.38 (s, 1H), 7.01 d, 1 H J = 8.4 Hz), 6.92 - 6.88 (m, 2H)), 4.41 (t, 2H, J = 5.0Hz), 3.85 (s, 3H), 3.44 (t, 2H, J = 5.0 Hz), 3.28- 3.2 (bs, 4H), 2.01 -1.9(bs, 4H).

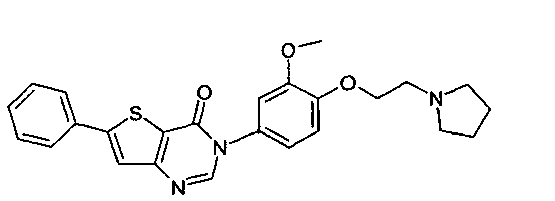

### 3-[3-Methoxy-4-(2-pyrrolidin-1-ylethoxy)phenyl]-6-phenylthieno[3,2-d]pyrimidin-4(3H)-one

6-Bromo-3-[3-methoxy-4-(2-pyrrolidin-1-ylethoxy)phenyl]thieno[3,2-d]pyrimidin-4(3H)-one (0.0386 g, 0.086 mmol) and phenyl boronic acid (0.011 g, 0.091 mmol) were dissolved in a THF (3 mL) and EtOH (1 mL) mixture. Nitrogen was bubbled through the reaction for 5 min. Pd(dppf)₂Cl₂ and 1 M Na₂CO₃ (1 mL) were added. The reaction was heated to 75°C and stirred for 30 min. The reaction was partioned between water (20 mL) and EtOAc (20 mL). The organics were removed, dried over MgSO₄, filtered and concentrated. The crude product was purified by chromatatron (98:2 CH₂Cl₂:MeOH) to give 0.005 g (0.011 mmol, 13%) of the product as a light brown solid. ¹H NMR (CDCl₃) δ 8.13 (s, 1 H), 7.73 (d, 2H, J = 7.1 Hz), 7.56 (s, 1 H), 7.5 - 7.4 (m, 3H), 7.02 (d, 1 H J = 8.2 Hz), 6.96 - 6.92 (m, 2H)), 4.26 (t, 2H, J = 6.0Hz), 3.88 (s, 3H), 3.06 (t, 2H, J = 6.1 Hz), 2.8-2.6 (bs, 4H), 1.9 -1.7 (bs, 4H).

### Examples I2-I4 were prepared according to the procedures described in Example I1.

### Example I2

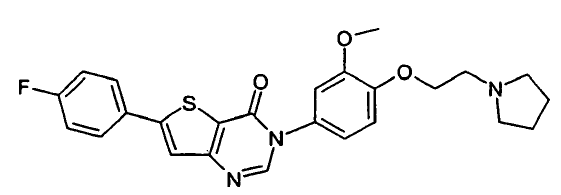

### 6-(4-Fluorophenyl)-3-[3-methoxy-4-(2-pyrrolidin-1-ylethoxy)phenyl]thieno[3,2-d]pyrimidin-4(3H)-one

¹H NMR (D6- DMSO) δ 8.37 (s, 1 H), 7.93 (t, 2H, J = 4.5 Hz), 7.90 (s, 1H), 7.34 (t, 2H, J = 8.6 Hz), 7.19 (s, 1 H), 7.12 (d, 1 H J = 8.6 Hz), 7.03 (d, 1H, J = 8.4 Hz), 4.26 (bs, 2H), 3.88 (s, 3H), 3.06 (bs, 2H), 2.8-2.7 (bs, 4H), 1.9 -1.7 (bs, 4H). LCMS M=H 467

### Example I3

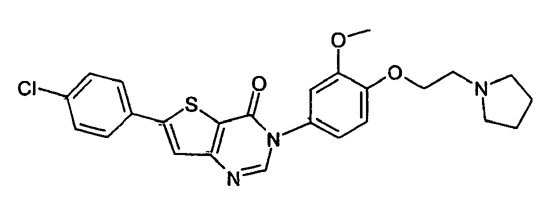

### 6-(4-Chlorophenyl)-3-[3-methoxy-4-(2-pyrrolidin-1-ylethoxy)phenyl]thieno[3,2-d]pyrimidin-4(3H)-one

¹H NMR (CDCl₃) δ 8.13 (s, 1H), 7.65 (d, 2H, J = 8.6 Hz), 7.53 (s, 1H), 7.44 (d, 2H, J = 8.6 Hz), 7.01 (d, 1 H, J = 8.8 Hz), 6.94 - 6.90 (m, 2H), 4.22 (t, 2H, J = 6.4 Hz), 3.88 (s, 3H), 3.01 (t, 6.2 Hz), 2.8-2.7 (bs, 4H), 1.9 -1.7 (bs, 4H). LCMS M+H 482

The title compound of Example 13 was also prepared as follows:

Methyl 5-(4-chlorophenyl)-3-{[(*E*)-(dimethylamino)methylidene]amino}-2-thiophenecarboxylate (12.06 g, 0.03735 mol, the preparation of which is found in Example J13) was stirred in absolute ethanol (60 mL) and 3-methoxy-4-(2-pyrrolidin-1-ylethoxy)aniline (8.82 g, 0.03735 mol) was added. The mixture was heated at reflux temperature for 72 h and then cooled to 4°C and kept at this temperature for 16 h. The resultant precipitatate was collected on a fritted glass funnel and the precipitate was then dried in a vacuum oven overnight at 50°C to provide 9.57 g of a gray solid. This solid was dissolved in dichloromethane (80 mL) and to the resultant mixture was added maleic acid (2.88 g, 24.8 mmol) in methanol (9 mL). The mixture was stirred for 5 min at room temperature and then diethyl ether (7 mL) was added. The mixture was stirred for 10 min at room temperature. The resultant precipitate was collected on fritted glass, washed with ether, and dried at 50°C overnight in a vacuum oven to produce 8.18 g of the title compound as its maleate salt, mp 214-215°C. ¹H NMR (400 MHz, CDCl₃) δ 8.14 (s, 1H), 7.65 (d, J = 8.6 Hz, 2H), 7.53 (s, 1 H), 7.44 (d, J = 8.6 Hz, 2H), 6.92-7.04 (m, 3H), 6.27 (s, 2H), 4.45 (t, J = 4.6 Hz, 2H), 3.89-4.00 (m, 2H), 3.87 (s, 3H), 3.59 (t, J = 4.6 Hz, 2H), 3.00-3.14 (m, 2H), 2.10-2.23 (m, 4H).

### Example I4

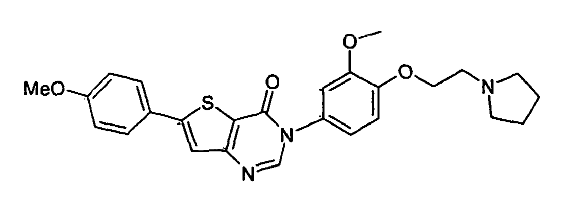

### 6-(4-Methoxyphenyl)-3-[3-methoxy-4-(2-pyrrolidin-1-ylethoxy)phenyl]thieno[3,2-d]pyrimidin-4(3H)-one

¹H NMR (CDCl₃) δ 8.11 (s, 1 H), 7.66 (d, 2H, J = 8.8 Hz), 7.44 (s, 1 H), 7.01-6.90 (m, 5H), 4.21 (t, 2H, J = 6.2 Hz), 3.88 (s, 3H), 3.86 (s, 3H), 2.98 (t, 6.2 Hz), 2.8-2.7 (bs, 4H), 1.9 -1.7 (bs, 4H). LCMS M+H 478

### Example I5

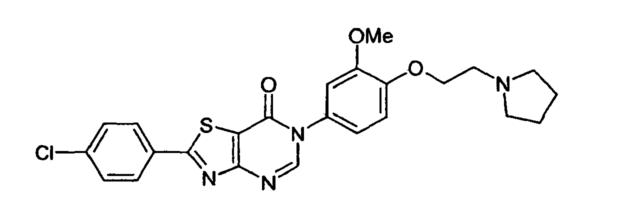

### 2-(4-Chlorophenyl)-6-[3-methoxy-4-(2-pyrrolidin-1ylethoxy)phenyl][1,3]thiazolo[4,5-d]pyrimidin-7(6H)-one

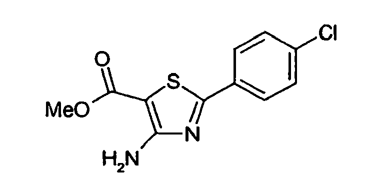

### Methyl 4-amino-2-(4-chlorophenyl)-1,3-thiazole-5-carboxylate

Methyl 4-chlorobenzenecarbodithioate (0.690, 3.4158 mmol) and cyanamide (0.1436 g, 3.1458 mmol) were taken up in dry methanol (10 mL). Potassium methoxide (0.2396 g, 3.4158 mmol) was added. The reaction was stirred at RT for 3 h. The reaction mixture was concentrated to dryness to give a red solid. The residue was taken up in DMF (10 mL) and methyl iodide (0.727 g, 5.123 mmol) were added. The reaction was stirred for 2 hours, then diluted with EtOAc (50 mL). The organics were washed with water (3 x 100 mL), dried over MgSO₄, filtered and concentrated. The residue was taken up in dry methanol (10 mL) and thioglycolate methyl ester (0.739 g, 9.96 mmol) was added, followed by triethylamine (1.3 mL). The reaction was stirred overnight at RT. The precipitate was collected to give 0.16 g (0.5970 mmol, 17%) of product. ¹H NMR (CDCl₃) δ 7.85 (d, 2H, J = 8.4 Hz), 7.40 (d, 2H, J = 8.6 Hz), 5.89 (bs, 2H), 3.85 (s, 3H).

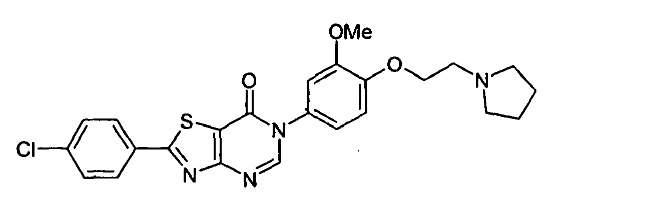

### 2-(4-Chlorophenyl)-6-[3-methoxy-4-(2-pyrrolidin-1 ylethoxy)phenyl][1,3]thiazolo[4,5-d]pyrimidin-7(6H)-one

Methyl 4-amino-2-(4-chlorophenyl)-1,3-thiazole-5-carboxylate(0.4589 g, 1.7123 mmol) was dissolved in dimethylformaide dimethyl acetal (5 mL) and heated to 100°C. The reaction was stirred for 3 h and then concentrated to dryness. 3-Methoxy-4-(2-pyrrolidin-1-ylethoxy)aniline (0.404 g, 1.7123 mmol) in anhydrous ethanol (2 mL) was added to the residue. The reaction was heated to 100°C and stirred for 18 h. The precipitate was collected and purified by chromatatron (3:1 Hex:EtOAc to 9:1 CH₂Cl₂:MeOH) to give 0.0978 g (0.2029 mmol, 12 %) of the product as a cream colored solid. The title compound was dissolved in CH₂Cl₂ (4 mL) and methanol (0.5 mL). Maleic acid (0.025 g, 0.2130 mmol) was added and the reaction stirred at RT for 4 h. The reaction was concentrated and fresh CH₂Cl₂ (3 mL) was added. The solids were then collected to give 0.066 g of the maleate salt.
¹H NMR (CDCl₃) (free base) δ 8.28 (s, 1 H), 8.08 (d, 2H, J = 8.5 Hz), 7.49 (d, 2H, J = 8.6 Hz), 7.03, (d, 1H, J = 9.2 Hz), 6.93 (bs, 2H), 4.27 (bs, 2H), 3.88 (s, 3H), 3.05 (bs, 2H), 2.74 (bs, 4H), 1.86 (bs, 4H). ¹H NMR (D₆-DMSO) (maelate salt) δ 9.67 (bs, 1H), 8.55 (s, 1 H), 8.16 (d, 2H, J = 8.5 Hz), 7.66 (d, 2H, J = 8.4 Hz), 7.28, (s, 1 H), 7.20 (d, 1H, J = 8.6 Hz) 7.10 (d, 2H, J = 8.4 Hz), 6.00 (s, 2H), 4.33 (bs, 2H), 3.78 (s, 3H), 3.60 (bs, 2H), 3.20 (bs, 4H), 2.0 -1.90 (bs, 4H). LRMS M + H 469

### Example I6

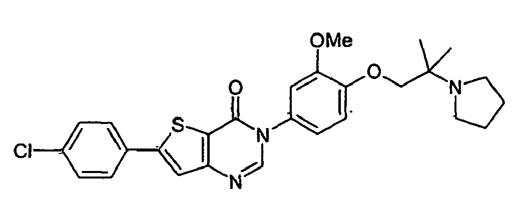

### 6-(4-Chlorophenyl)-3-[3-mothoxy-4-(2-methyl-2-pyrrolidin-1-ylpropoxy)phenyl]thieno[3,2-d]pyrimidin-4(3H)-one

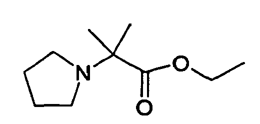

### Ethyl 2-methyl-2-pyrrolidin-1-ylpropanoate

Ethyl 2-bromo-2-methylpropanoate (0.6800 g, 3.4861 mmol) was taken up in pyrrolidine (4 mL) and heated to 70°C. The reaction was stirred for 18 h and then concentrated. The residue was taken up in EtOAc (100 mL) and washed with water (1 x 100 mL), dried over MgSO₄, filtered and concentrated to afford 0.4754 g ( 2.5697 mmol, 74%) of the product. 1 H NMR (CDCl₃) δ 4.16 (q, 2H, J = 7.1 Hz), 2.76 (bs, 4H), 1,76 (bs, 4H), 1.36 (s, 6H), 1.27 (t, 2H, J = 7.1 Hz).

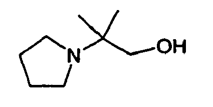

### 2-Methyl-2-pyrrolidin-1-ylpropan-1-ol

Ethyl 2-methyl-2-pyrrolidin-1-ylpropanoate (0.6162 g, 3.3308 mmol) was taken up in dry THF (5 mL). A 1 M solution of LAH in THF (3.7 mL, 3.6639 mmol) was added drop wise. The reaction was warmed to 50°C and stirred for 18 h. Cooleed to 0°C in an ice bath and methanol (5 mL) was added slowly. The reaction was diluted with water (50 mL) and extracted with Et₂O (2 x 50 mL). The organics were concentrated by blowing a slow stream of N₂ over the flask to give 0.3578 g (2.0682 mmol, 62%) of the product as a light brown oil. ¹H NMR (CDCl₃) δ 3.22 (s, 2H), 2.61 (bs, 4H), 1.79 (bs, 4H), 1.01 (s, 6H).

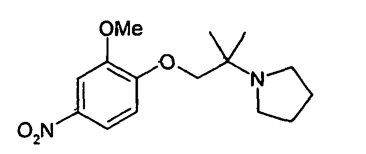

### 1-[2-(2-Methoxy-4-nitrophenoxy)-1,1-dimethylethyl]pyrrolidine

2-Methyl-2-pyrrolidin-1-ylpropan-1-ol (0.3776 g, 2.0682 mmol) was taken up in anhydrous DMF (5 mL). Sodium hydride (0.083 g of a 60 %dispersion, 2.0682 mmol) was added. The reaction was stirred at RT until gas evolution was complete. 1-Chloro-2-methoxy-4- nitrobenzene (0.3578 g, 2.0682 mmol) was added. The reaction was heated to 80°C and stirred for 72 h. Cooled to RT and diluted with EtOAc, (50 mL) and washed with water (3 x 50 mL). Organics were dried over MgSO₄, filtered and concentrated. Crude product was purified by chromatatron (100% CH₂Cl₂ to 95:5 CH₂Cl₂:MeOH) to give 0.075 g (0.2551 mmol, 12%) of the product. ¹H NMR (CDCl₃) δ 7.81 (d, 1H, J = 8.8 Hz), 7.73 (s, 1H), 7.15 (d, 1H, J = 8.8 Hz), 3.88 (s, 3H), 2.76 (s, 2H), 2.68 (bs, 4H), 1.75 (bs, 4H), 1.38 (s, 6H).

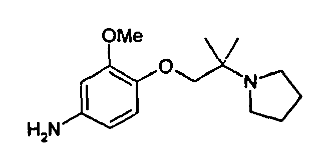

### 3-Methoxy-4-(2-methyl-2-pyrrolidin-1 -ylpropoxy)aniline

1-[2-(2-Methoxy-4-nitrophenoxy)-1,1-dimethylethyl]pyrrolidine (0.075 g, 0.2551 mmol) was dissolved in EtOAc (10 mL). 10% Pd/C (0.007 g) was added and the reaction hydrogenated under 1 atm of H₂. The reaction was stirred for 18 h, filtered through celite and the celite washed with EtOAc (3 x 30 mL). The combined organics were concentrated to afford 0.063 g (0.2386 mmol, 93%) of the product. ¹H NMR (CDCl₃) δ 6.80 (d, 1H, J = 8.4 Hz), 6.22 (s, 1H), 6.18 (d, 1H, J = 8.3 Hz), 3.78 (s, 3H), 3.50 (bs, 2H), 2.68 (bs, 6H), 1.78 (bs, 4H), 1.22 (s, 6H).

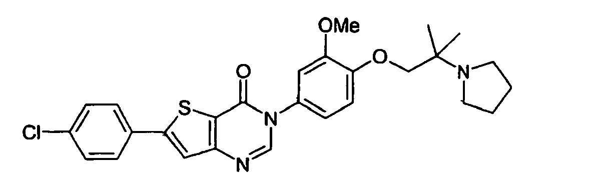

### 6-(4-Chlorophenyl)-3-[3-methoxy-4-(2-methyl-2-pyrrolidin-1 - ylpropoxy)phenyl]thieno[3,2-d]pyrimidin-4(3H)-one

Methyl 3-amino-5-(4-chlorophenyl)thiophene-2-carboxylate (0.639 g, 0.2390 mmol) was taken up in DMF-DMA (3 mL) and heated to 110°C. The reaction was stirred for 3 h and then concentrated. 3-Methoxy-4-(2-methyl-2-pyrrolidin-1-ylpropoxy)aniline (0.0631 g, 0.2390 mmol) in absolute ethanol (3 mL) was added to the residue and the mixture was then concentrated. Fresh absolute ethanol (1 mL) was added and the reaction heated to reflux. The reaction was stirred for 18 h and then cooled to RT and the precipitate was collected to give 0.010 g (0.020 mmol, 8%) of the product as a white solid. ¹H NMR (CDCl₃) δ 8.15 (s, 1 H), 7.65 (d, 2H, J = 8.3 Hz), 7.53 (s, 1H), 7.44 (d, 2H, J = 8.5 Hz), 7.18 (d, 1H, J = 8.3 Hz), 6.96 (s, 1H), 6.88 (d, 1H, J = 8.3 Hz), 3.83 (s, 3H, 2.77 (s, 2H), 2.72 (bs, 4H), 1.78 (bs, 4H), 1.36 (s, 6H). LRMS M + H 511

### Example I7

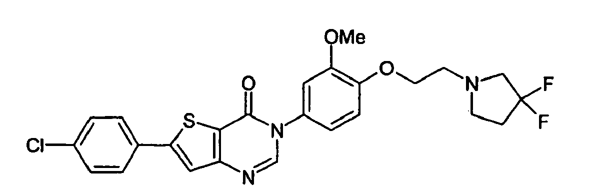

### 6-(4-Chlorophenyl)-3-{4-[2-(3,3-difluoropyrrolidin-1-yl)ethoxy]-3-methoxyphenyl}thieno[3,2-d]pyrimidin-4(3H)-one

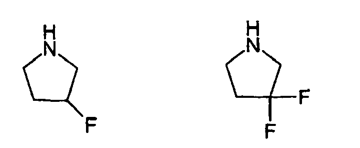

### 3-fluoropyrolidine and 3,3-difluoropyrrolidine

3-fluoropyrolidine and 3,3-difluoropyrrolidine were prepared following procedures described in the literature.
Giardina,G.: Dondio, G.; Grugni, M., *Synlett,* 1995, *(1),* 55-57

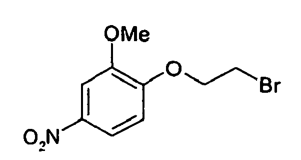

### 1-(2-Bromoethoxy)-2-methoxy-4-nitrobenzene

4- Nitroguaiacol (1.0171 g, 6.0134 mmol) was dissolved in anhydrous DMF (20 mL). Cesium carbonate (3.9207 g, 12.0268 mmol) and 1,2-dibromoethane (2.07 mL, 24.0534 mmol) were added. The reaction was heated to 80°C and stirred for 18 h. The mix was then cooled to RT and diluted with EtOAc (100 mL), washed with water (3 x 100 mL), and the organics dried over MgSO₄, filtered and concentrated. The residue was filtered through a 3" plug of basic alumina (100% CH₂Cl₂) to give 0.7075 g (2.5634 mmol, 42%) of the product as a white solid. ¹H NMR (CDCl₃) δ 7.90 (d, 1 H, J = 8.8 Hz), 7.78 (s, 1 H), 6.92 (d, 1 H, J = 8.8 Hz), 4.42 (t, 2H, J = 6.4 Hz), 3.96 (s, 3H), 3.70 (t, 2H, J = 6.5 Hz).

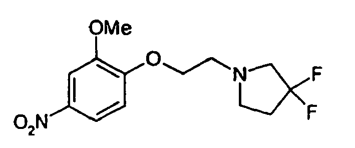

### 3,3-Difluoro-1-[2-(2-methoxy-4-nitrophenoxy)ethyl]pyrrolidine:

3,3-Difluoropyrrolidine (0.4589 g, 2.0859 mmol) and 1-(2-bromoethoxy)-2-methoxy-4-nitrobenzene (0.5757 g, 2.0859 mmol) were combined in DMF (5 mL). Triethylamine (0.6332 g, 6.2577 mmol) was added. The reaction was heated to 80°C and stirred for 18 h. The reaction was cooled to RT and diluted with EtOAc (50 mL). The organics were washed with water (3 x 100 mL), dried over MgSO₄, filtered and concentrated. The residue was purified by chromatatron (1:1 Hex:EtOAc) to give 0.173 g (0.5728 mmol, 27%) of the product as a light yellow oil. ¹H NMR (CDCl₃) δ 7.90 (d, 1H, 9 Hz), 7.75 (s, 1H), 6.89 (d, 1H, J = 9 Hz), 4.21 (t, 2H, J = 5.6 Hz), 3.94 (s, 3H), 3.07 (t, 2H, J = 13.3 Hz), 2.99 (t, 2H, J = 5.7 Hz), 2.89 (t, 2H, J = 7.1 Hz), 2.28 (m, 2H).

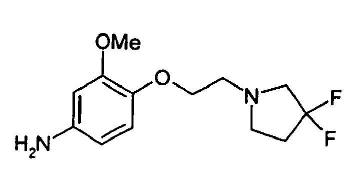

### 4-[2-(3,3-Difluoropyrrolidin-1-yl)ethoxy]-3-methoxyaniline

3,3-Difluoro-1-[2-(2-methoxy-4-nitrophenoxy)ethyl]pyrrolidine (0.173 g, 0.5728 mmol) was dissolved in EtOAc (5 mL). 10% Pd/C (0.017 g) was added. The reaction was stirred under 1 atm of H₂. for 18 h. The mix was then filtered through celite and the celite washed with EtOAc (3 x 10 mL). The organics were concentrated to give 0.1389 g (0.5107 mmol, 89%) of the product as a red oil. ¹H NMR (CDCl₃) δ 6.73 (d, 1H. J = 8.4 Hz), 6.29 (s, 1H), 6.19 (d, 1H, J = 8.4 Hz), 4.02 (t, 2H, J = 5.8 Hz), 3.80 (s, 3HO, 3.04 (t, 2H, J = 13.4 Hz), 2.87 (m, 4H), 2.27 (m, 2H).

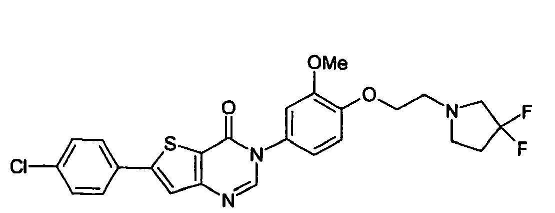

### 6-(4-Chlorophenyl)-3-{4-[2-(3,3-difluoropyrrolidin-1-yl)ethoxy]-3-methoxyphenyl}thieno[3,2-d]pyrimidin-4(3H)-one

Methyl 3-amino-5-(4-chlorophenyl)thiophene-2-carboxylate (0.1367 g, 0.5107 mmol) was taken up in dimethylformamdide dimethyl acetal (3 mL) and heated to 110°C. The reaction was stirred for 3 h and then concentrated. 4-[2-(3,3-Difluoropyrrolidin-1-yl)ethoxy]-3-methoxyaniline (0.1389 g, 0.5107 mmol) in absolute ethanol (3 mL) was added to the residue and concentrated. Fresh absolute ethanol (1 mL) was added and the reaction heated to reflux. The reaction was stirred for 18 h and then cooled to RT and the precipitate was collected to give 0.058 g (0.1120 mmol, 22%) of the product as a white solid. ¹H NMR (D₆-DMSO) δ 8.38 (s, 1H), 7.96 (s, 1H, 7.91 (d, 2H, J = 8.4 Hz), 7.56 (d, 2H, J = 8.4 Hz), 7.18 (s, 1 H), 7.11 (d, 1 H, J = 8.5 Hz), 7.04 (d, 1H, J = 8.6 Hz), 4.12 (t, 2H, J = 5.5 Hz), 3.77 (s, 3H), 3.00 (t, 2H, J = 13.6 Hz), 2.85 (t, 2H, J = 5.5 Hz), 2.81 (t, 2H, J = 7.0 Hz), 2.22 (m, 2H). LRMS M + H

### Example I8

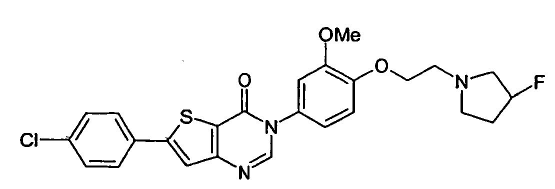

### 6-(4-chlorophenyl)-3-{4-[2-(3-fluoropyrrolidin-1-yl)ethoxy]-3-methoxyphenyl)thieno[3,2-d]pyrimidin-4(3H)-one

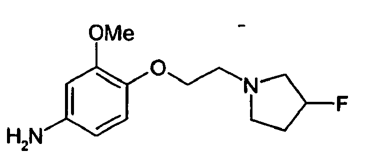

### 3-Fluoro-1-[2-(2-methoxy-4-nitrophenoxy)ethyl]pyrrolidine 1-(2-Bromoethoxy)-2-methoxy-4-nitrobenzene (0.2346 g, 0.8499 mmol) and

3-fluoropyrrolidine (0.3187 g, 2.5496 mmol) were combined in DMF (5 mL). Triethylamine (0.43 g, 4.2495 mmol) was added. The reaction was heated to 80°C and stirred for 18 h. The reaction was cooled to RT and diluted with EtOAc (50 mL). The organics were washed with water (3 x 100 mL), dried over MgSO₄, filtered and concentrated. The residue was purified by chromatatron (98:2 CH₂Cl₂:MeOH). The product was subjected to hydrogenation using 10% Pd/C under 1 atm of H₂. The reaction was stirred for 18 h and was then filtered through celite and the celite washed with EtOAc (2 x 10mL). The organics were concentrated to give 0.0442 g (0.1740 mmol, 20%) of the product. ¹H NMR (CDCl₃) δ 6.75 (d, 1H, J = 8.4 Hz), 6.28 (s, 1H), 6.19 (d, 1H, J = 8.3 Hz), 5.26-5.13 (m, 1H), 4.10 (t, 2H, J = 5.7 Hz), 3.80 (s, 3H), 3.10-2.78 (m, 6H), 2.27-2.05 (m, 2H).

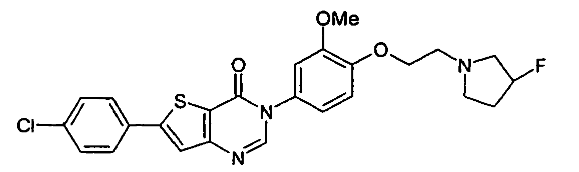

### 6-(4-ch)orophenyl)-3-{4-[2-(3-fluoropyrrolidin-1-yl)ethoxy]-3-methoxyphenyl}thieno[3,2-d]pyrimidin-4(3H)-one

3-Fluoro-1-[2-(2-methoxy-4-nitrophenoxy)ethyl]pyrrolidine (0.0442 g, 0.1740 mmol) and methyl 5-(4-chlorophenyl)-3-{[(1*E*)-(dimethylamino)methylidene]amino)thiophene-2-carboxylate (0.056 g, 0.1740 mmol) were charged to a flask. Phenol (1 g) was added and the reaction heated to 200°C. The reaction was stirred for 45 min until all starting material was gone. The mix was then cooled to RT, diluted with CH₂Cl₂ (30 mL) and washed with 1 N NaOH (2 x 30 mL), water (1 x 30 mL), brine (1 x 30 mL), dried over MgSO₄, filtered, and concentrated. The residue was purified on a chromatatron (98:2 CH₂Cl₂:MeOH) to give 0.010 g (0.020 mmol, 11%) of the product as a tan solid. ¹H NMR (CDCl₃) δ 8.13 (s, 1 H), 7.65 (d, 2H, J = 8.3 Hz), 7.52 (s, 1H), 7.43 (d, 2H, J = 8.4 Hz), 7.00 (d, 1H, J = 8.4 Hz), 6.93 (m, 2H), 5.29-5.13 (m, 1H), 4.23 (t, 2H, J = 5.7 Hz), 3.88 (s, 3H); 3.12-2.93 (m, 5H), 2.68 (bs, 1 H), 2.25-2.06 (m, 2H).
LRMS M + H 500

### Example J1

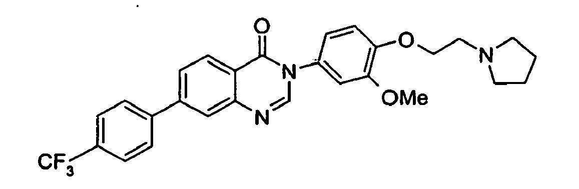

### 3-{3-methoxy-4-[2-(1-pyrrolidinyl)ethoxy]phenyl}-7-[4-(trifluoromethyl)phenyl]-4(3H)-quinazolinone

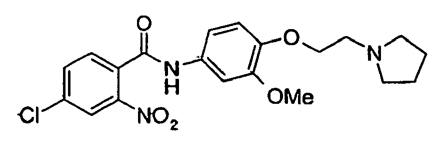

### 4-chloro-N-{3-methoxy-4-[2-(1-pyrrolidinyl)ethoxy]phenyl}-2-nitrobenzamide

To a solution of 3-methoxy-4-[2-(1-pyrrolidinyl)ethoxy]aniline (43 mmol, 10.0g) in DCM was added triethylamine (52 mmol, 5.2 g) and 4-chloro-2-nitrobenzoyl chloride (9.5g, 43 mmol). The solution was stirred overnight. The reaction mixture was extracted with 2N HCl. The resulting acidic aqueous solution was made basic by adding 2N NaOH. The combined basic aqueous solution was extracted with DCM. The organic phase was washed with brine, dried and concentrated giving the title compound (11.5g, 69%). ¹H NMR (DMSO-D6): δ 1.66 (4H, m), 2.49 (4H, m), 2.75 (2H, t, J = 6.0 Hz), 3.72 (3H, s), 4.00 (2H, t, J = 6.0 Hz), 6.94 (1 H, d, J = 8.6 Hz), 7.14 (1 H, d, J = 8.6 Hz), 7.79 (1H, d, J = 8.1 Hz), 7.94 (1 H, d, J = 8.1 Hz), 8.23 (1 H, s), 10.55 (1 H, s). LCMS m/z = 420 (m + H⁺).

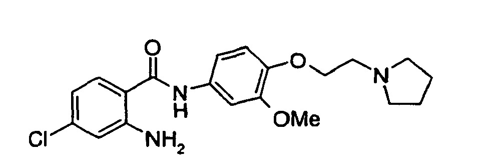

### 2-amino-4-chloro-N-{3-methoxy-4-[2-(1-pyrrolidinyl)ethoxy]phenyl)benzamide

To a solution of 4-chloro-*N*-{3-methoxy-4-[2-(1-pyrrolidinyl)ethoxy]phenyl}-2-nitrobenzamide (11.9 mmol, 5.0g) in ethanol was added tin(II) chloride dihydrate (35.8 mmol, 8.1 g). The resulting mixture was heated to reflux for 4 h and then concentrated by rotary evaporation. The residue was dissolved in ethyl acetate and was washed with potassium sodium tartrate tetrahydrate solution repeatedly. The crude product was first extracted with 2N HCl, then made basic by adding NaOH with ice cooling. The combined basic aqueous solution was extracted with DCM. The organic extraction was washed with brine, dried and concentrated giving the product (4.5g, 97%). ¹H NMR (DMSO-D6): δ 1.67 (4H, m), 2.48 (4H, m), 2.81 (2H, t, J = 5.8 Hz), 3.75 (3H, s), 4.10 (2H, t, J = 5.8 Hz), 7.02 (1 H, d, J = 6.2 Hz), 7.10 (1 H, d. J = 8.6 Hz), 7.16 (1H. s), 7.62 (1 H, d, J = 8.6 Hz), 7.80 (1 H, s), 8.17 (1 H, d, 8.6 Hz), 8.35 (1 H, s). LCMS m/z = 390 (m + H⁺).

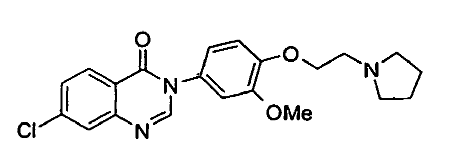

### 7-chloro-3-{3-methoxy-4-[2-(1-pyrrolidinyl)ethoxy]phenyl}-4(3H)-quinazolinone

2-Amino-4-chloro-*N*-{3-methoxy-4-[2-(1-pyrrolidinyl)ethoxy]phenyl}benzamide (11.6 mmol, 4.5g) was dissolved in 90 mL 88% formic acid and was heated to reflux for 3 h. The solvents were removed by rotary evaporation and the residue was purified by silica gel column chromatography affording the product (4.2g, 91%). ¹H NMR (DMSO-D6): δ 1.82 (4H, m), 2.65 (4H, m), 2.88 (2H, t, J = 6.2 Hz), 3.88 (3H, s), 4.20 (2H, t, J = 6.4 Hz), 6.89-6.91 (2H, overlapping), 7.01 (1H, d, J = 10.1 Hz), 7.49 (1H, d, J = 8.6 Hz), 7.76 (1H, s), 8.12 (1 H, s), 8.29 (1 H, d, 8.7 Hz). LCMS m/z = 400 (m + H⁺).

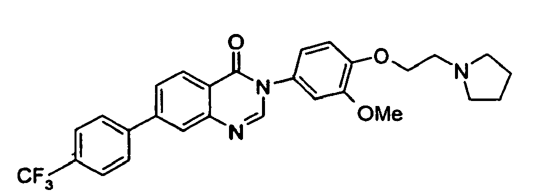

### 3-{3-methoxy-4-[2-(1-pyrrolidinyl)ethoxy]phenyl}-7-[4-(trifluoromethyl)phenyl]-4(3H)-quinazolinone

The aryl chloride from the preceding step (150 mg, 0.38 mmol), trifluoromethylphenyl boronic acid (130 mg, 1.5 eq), bis-t-butylbiphenylphosphine (24 mg, 20 mol%), palladium acetate (9 mg, 10 mol%) and potassium fluoride (65 mg, 3 eq) were added to a dry round bottom flask. To this was added anhydrous THF (2.0 mL). The reaction mixture was heated at 70°C for 3 h. The reaction mixture was filtered. The filtrate was diluted with ethyl acetate and was washed with 1 N NaOH and with water. The aqueous washes were back extracted twice with ethyl acetate. The organic portion was dried and concentrated. The residue was purified by silica gel column chromatography eluting with 10% acetone in DCM with 1% triethylamine to give the title compound (0.36 g, 61 %). ¹H NMR (CDCl₃): δ 1.98 (4H, m), 3.02 (4H, m), 3.24 (2H, m), 3.89 (3H, s), 4.38 (2H, t, J= 5.5 Hz), 6.93-6.97 (2H, overlapping), 7.05 (1 H, d, J = 9.5 Hz), 7.76-7.83 (5H, overlapping), 7.99 (1H, s), 8.16 (1 H, s), 8.44 (1 H, d, 8.3 Hz). LCMS m/z = 510 (m + H⁺).

### Examples J2-J12 were prepared according to the procedures described in Example J1.

### Example J2

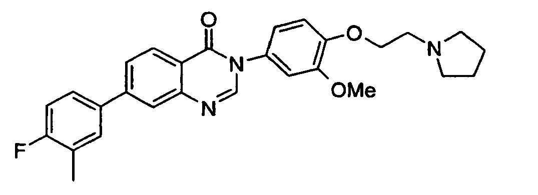

### 7-(4-fluoro-3-methylphenyl)-3-(3-methoxy-4-[2-(1-pyrrolidinyl)ethoxy]phenyl}-4(3H)-quinazolinone

¹H NMR (CDCl₃): δ 2.05 (4H, m), 2.37 (3H, s), 3.30 (4H, m), 3.46 (2H, m), 3.88 (3H, s), 4.42 (2H, m), 6.93-6.97 (2H, overlapping), 7.04 (1 H, d, J = 8.4 Hz), 7.13 (1 H, d, J = 8.9 Hz), 7.48-7.54 (2H, overlapping), 7.73 (1H, d, J = 8.3 Hz), 7.91 (1 H, s), 8.14 (1 H, s), 8.38 (1 H, d, 8.5), 8.45 (1 H, bs). LCMS m/z = 474 (m + H⁺).

### Example J3

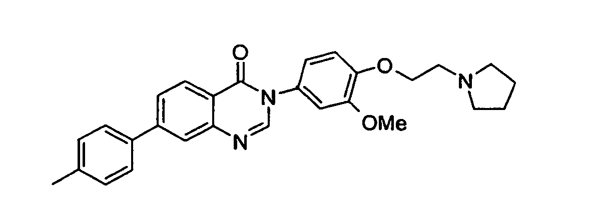

### 3-{3-methoxy-4-[2-(1-pyrrolidinyl)ethoxy]phenyl}-7-(4-methylphenyl)-4(3H)-quinazolinone

¹H NMR (CDCl₃): δ 1.99 (4H, m), 2.17 (3H, s), 3.14 (4H, m), 3.34 (2H, t, J = 5.3 Hz), 3.88 (3H, s), 4.38 (2H, t, J = 5.3 Hz), 6.93-6.97 (2H, overlapping), 7.03 (1H, d, J = 8.4 Hz), 7.32 (1H, d, J = 8.1 Hz), 7.63 (1 H, d, J = 8.1 Hz), 7.78 (1H, d, J = 8.2 Hz), 7.96 (1 H, s), 8.13 (1H, s), 8.39 (1 H, d, 8.3 Hz), 8.48 (1H, s). LCMS m/z = 455 (m + H⁺).

### Example J4

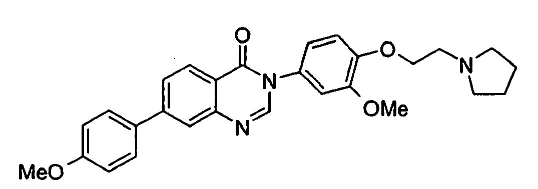

### 7-(4-methoxyphenyl)-3-{3-methoxy-4-2-(1-pyrrolidinyl)ethoxy]phenyl)-4(3H)-quinazolinone

¹H NMR (CDCl₃): δ 1.85 (4H, m), 2.72 (4H, m), 3.04 (2H, t, J = 6.1 Hz), 3.88 (3H, s), 3.89 (3H, s), 4.25 (2H, t, J=6.2 Hz), 6.94-7.05 (5H, overlapping), 7.67 (2H, d, J = 8.4 Hz), 7.75 (1 H, d, J = 8.3 Hz), 7.93 (1H, s), 8.13 (1H, s), 8.37 (1 H, d, 8.2 Hz). LCMS m/z = 472 (m + H⁺).

### Example J5

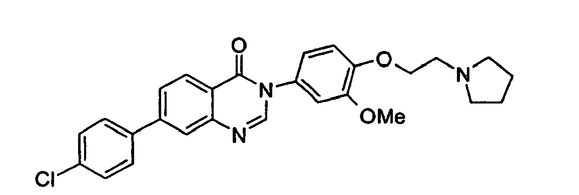

### 7-(4-chlorophenyl)-3-{3-methoxy-4-[2-(1-pyrrolidinyl)ethoxy]phenyl}-4(3H)-quinazolinone

¹H NMR (CDCl₃): δ 1.97 (4H, m), 3.03 (4H, m), 3.27 (2H, t, J = 5.6 Hz), 3.88 (3H, s), 4.34 (2H, t, J =5.6 Hz), 6.95-6.96 (2H, m, overlapping), 7.04 (1 H, d, J = 8.4 Hz), 7.48 (2H, d, J =8.4 Hz), 7.65 (2H, d, J = 8.2 Hz), 7.74 (1H, d, J = 8.2 Hz), 7.94 (1 H, s), 8.14 (1 H, s), 8.41 (1H, d, J = 8.3 Hz), 8.50 (1H, s). LCMS m/z = 476 (m + H⁺).

### Example J6

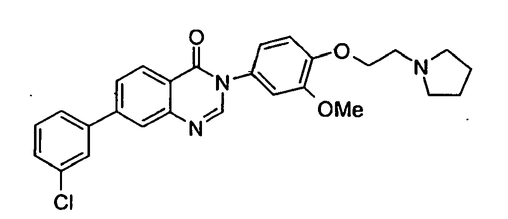

### 7-(3-chlorophenyl)-3-(3-methoxy-4-[2-(1-pyrrolidinyl)ethoxy]phenyl)-4(3H)-quinazolinone

¹H NMR (CD₃OD) : (maleic acid salt) δ 2.14 (4H, bs), 3.47-3.54 (4H, bs), 3.70 (2H, t, J = 5.3 Hz), 3.92 (3H, s), 4.40 (2H, t, J = 4.8 Hz), 6.24 (2H, s, maleic acid), 7.08 (1 H, d, J = 8.4 Hz), 7.22-7.25 (2H, m, overlapping), 7.45-7.54 (2H, m, overlapping), 7.71 (1H, d, J = 7.6 Hz), 7.78 (1 H, s), 7.88 (1H, d, J = 8.3 Hz), 8.28 (1 H, s), 8.33-8.37 (2H, m, overlapping). LCMS m/z = 476 (m + H⁺).

### Example J7

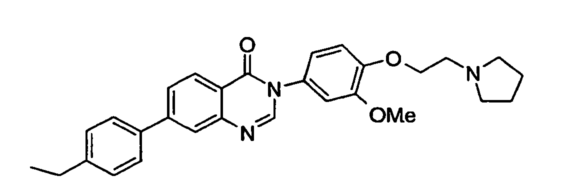

### 7-(4-ethylphenyl)-3-{3-methoxy-4-[2-(1-pyrrolidinyl)ethoxy]phenyl}-4(3H)-quinazolinone

¹H NMR (CDCl₃) δ 1.29 (3H, t, J = 7.6 Hz), 1.82 (4H, m), 2.62 (4H, m), 2.73 (2H, q, J = 7.6 Hz), 2.99 (2H, t, J = 3.6 Hz), 3.87 (3H, s), 4.22 (2H, t, J = 3.6 Hz), 6.91-7.02 (3H, m, overlapping), 7.34 (2H, d, J = 8.1 Hz), 7.65 (2H, d, J = 8.1 Hz), 7.78 (1 H, d, J = 8.3), 7.96 (1H, s), 8.12 (1 H, d, J = 8.8 Hz), 8.38 (1H, d, J = 8.3 Hz). LCMS m/z = 470 (m + H⁺).

### Example J8

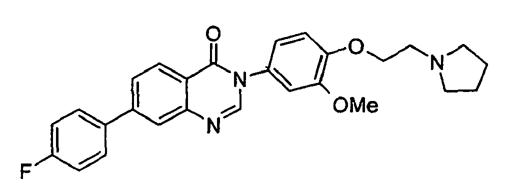

### 7-(4-fluorophenyl)-3-{3-methoxy-4-[2-(1-pyrrolidinyl)ethoxy]phenyl}-4(3H)-quinazolinone

¹H NMR (CDCl₃) δ 1.83 (4H, bs), 2.66 (4H, bs), 2.99 (2H, d, J = 6.4 Hz), 3.89 (3H, s), 4.22 (2H, t, J = 6.4 Hz), 6.92-6.95 (2H, m, overlapping), 7.20 (1 H, t, J = 8.1 Hz), 7.67-7.71 (3H, m, overlapping), 7.92 (1 H, s), 8.15 (1 H, s), 8.40 (1 H, d, J = 8.3 Hz). LCMS m/z = 460 (m + H⁺).

### Example J9

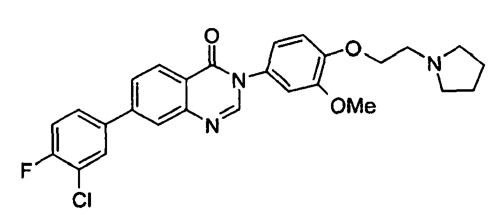

### 7-(3-chloro-4-fluorophenyl)-3-{3-methoxy-4-[2-(1-pyrrolidinyl)ethoxy]phenyl}-4(3H)-quinazolinone

¹H NMR (CDCl₃): (maleic acid salt) δ 2.05 (4H, bs), 3.18 (4H, bs), 3.34 (2H, bs), 3.89 (3H, s), 4.46 (2H, bs), 5.58 (2H, s, maleic acid), 6.93-6.97 (2H, m, overlapping), 7.07 (1 H, d, J = 8.5 Hz), 7.28 (1H, overlapping with CDCl₃), 7.58 (1 H, m), 7.69 (1 H, d, J= 8.4 Hz), 7.75 (1 H, d, J = 6.8 Hz), 7.9 (1H, s), 8.14 (1 H, s), 8.41 (1H, d, J = 8.3 Hz). LCMS m/z = 494 (m + H⁺).

### Example J10

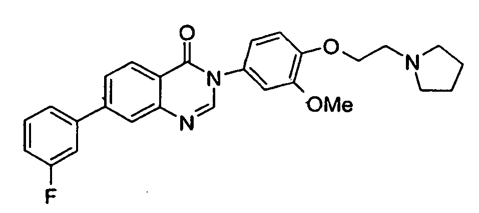

### 7-(3-fluorophenyl)-3-{3-methoxy-4-[2-(1-pyrrolidinyl)ethoxy]phenyl}-4(3H)-quinazolinone

¹H NMR (CDCl₃) δ 1.94 (4H, bs), 2.94 (4H, bs), 3.19 (2H, bs), 3.88 (3H, s), 4.35 (2H, t, J = 5.7 Hz), 6.92-7.05 (2H, m, overlapping), 7.11 (1 H, m), 7.39-7.50 (4H, m, overlapping), 7.74,(1 H, d, J = 8.3 Hz), 7.95 (1 H, s), 8.14 (1 H, s), 8.41 (1 H, d, J = 8.5 Hz). LCMS m/z = 460 (m + H⁺).

### Example J11

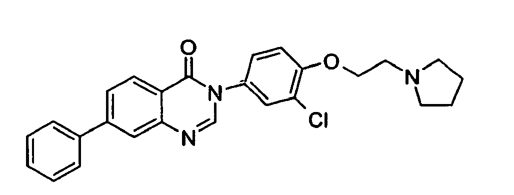

### 3-{3-chloro-4-[2-(1-pyrrolidinyl)ethoxy]phenyl}-7-phenyl-4(3H)-quinazolinone

¹H NMR (CDCl₃) δ 1.84 (4H, bs), 2.73 (4H, bs), 3.04 (2H, t, J = 5.8 Hz), 4.27 (2H, t, J = 5.8 Hz), 7.08 (1 H, d, J = 8.8 Hz), 7.29 (1 H, d, J = 8.6), 7.44-7.53 (3H, m, overlapping), 7.72 (2H, d, J = 7.2 Hz), 7.80 (1H, d, J = 8.3 Hz), 7.98 (1 H, s), 8.10 (1 H, s), 8.40 (1 H, d, J = 8.2 Hz). LCMS m/z = 446 (m + H⁺).

### Example J12

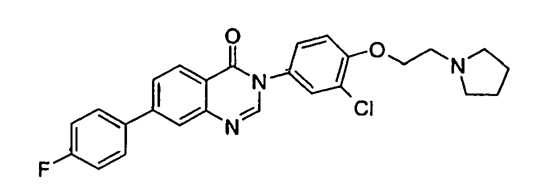

### 3-{3-chloro-4-[2-(1-pyrrolidinyl)ethoxy]phenyl}-7-(4-fluorophenyl)-4(3H)-quinazolinone

¹H NMR (CDCl₃) δ 1.84 (4H, bs), 2.75 (4H, bs), 3.05 (3H, t, J = 5.5 Hz), 4.28 (3H, t, J = 5.5 Hz), 7.07 (1 H, d, J = 8.8 Hz), 7.17-7.29 (3H, m, overlapping), 7.47 (1 H, s), 7.66-7.73 (3H, m, overlapping), 7.91 (1 H, s), 8.09 (1H, s), 8.38 (1H, d, J = 8.3 Hz). LCMS m/z = 464 (m + H⁺).

### Example J13

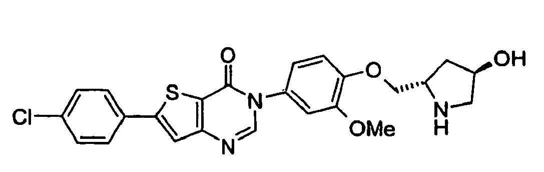

### 6-(4-chlorophenyl)-3-(4-{[(2S,4R)-4-hydroxypyrrolidinyl]methoxy}-3-methoxyphenyl)thieno[3,2-d]pyrimidin-4(3H)-one

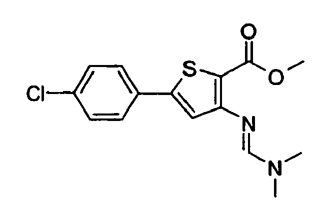

### Methyl 5-(4-chlorophenyl)-3-{[(E)-(dimethylamino)methylidene]amino}-2-thiophenecarboxylate

A mixture of methyl 3-amino-5-(4-chlorophenyl)-2-thiophenecarboxylate (37.3 mmol, 10.0 g) and N,N-dimethylformamide dimethyl acetal (74.7 mmol, 8.9 g) in ethanol (350 mL) was heated at reflux for 3 hours. The solvent was removed by rotary evaporation. To the residue 15 mL of toluene was added and the solvent was removed by rotary evaporation. This was repeated three times. To the resulting sticky residue, 20 mL hexanes was added followed by the gradual addition of ethyl acetate at 0°C until it solidified. The resulting solid was collected by filtration giving the product (11.9 g, 98.9%). ¹H NMR (CDCL₃): δ 3.08 (6H, d, J = 6.5 Hz), 3.81 (3H, s), 6.98 (1 H, s), 7.35 (2H, d, J = 8.6 Hz), 7.53 (2H, d, J = 8.5 Hz), 7.69 (1 H, s). LCMS m/z = 323 (m + H⁺).

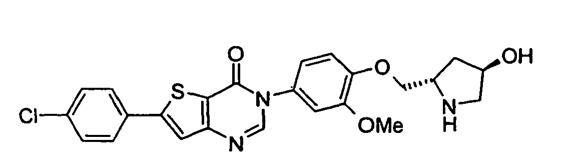

### 6-(4-chlorophenyl)-3-(4-{[(2S,4R)-4-hydroxypyrrolidinyl]methoxy}-3-methoxyphenyl)thieno[3,2-d]pyrimidin-4(3H)-one

Methyl 5-(4-chlorophenyl)-3-{[(E)-(dimethylamino)methylidene]amino)-2-thiophenecarboxylate (857 mg, 2.66 mmol) was mixed with *tert-*butyl (2*S*,4*R*)-2-[(4-amino-2-methoxyphenoxy)methyl]-4-hydroxypyrrolidine-1-carboxylate (900 mg) in phenol (1.0 g) at 120°C for 15 min. Workup and purification of the reaction mixture provided 650 mg of *tert*-butyl (2S,4*R*)-2-({4-[6-(4-chlorophenyl)-4-oxothieno[3,2-*d*]pyrimidin-3(4*H*)yl]-2-methoxyphenoxy}methyl)-4-hydroxypyrrolidine-1-carboxylate. A portion of this material (105 mg, 0.179 mmol) was dissoved in a 1:1 mixure of dichloromethane and trifluoroacetic acid. The mixture was stirred at room temperature for 10 min and was then concentrated under reduced pressure. The residue was dissolved in dichloromethane and washed with a saturated solution of NaHCO₃, brine, dried (Na₂SO₄) and concentrated to provide the title compound.
¹H NMR (CDCl₃): δ 1.80 (1H, m), 2.01 (1 H, m), 2.98 (1 H, d, J = 11.9 Hz), 3.15 (1H, dd, J = 11.9, 4.3 Hz), 3.87 (3H, s), 3.88-4.00 (3H, m, overlapping), 4.50 (1 H, m), 6.89-6.94 (2H, m, overlapping), 6.99 (1 H, d, J = 8.4 Hz), 7.44 (2H, d, J = 8.4 Hz), 7.51 (1 H, s), 7.63 (2H, d, J = 8.6 Hz), 8.12 (1 H, s). LCMS m/z = 484 (m + H⁺).

### Example J14

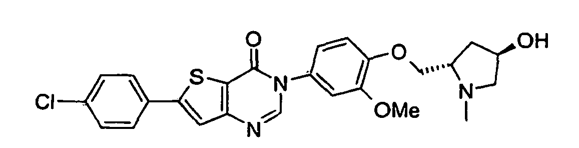

### 6-(4-chlorophenyl)-3-(4-{[(2S,4R)-4-hydroxy-1-methylpyrrolidinyl]methoxy}-3-methoxyphenyl)thieno[3,2-d]pyrimidin-4(3H)-one

The title compound was obtained through treatment of the title compound from Example J13 with the procedures described in Example H18.
¹H NMR (CDCl₃): δ 2.03-2.10 (1H, m), 2.40-2.43 (1H, m), 2.53 (3H, s), 3.02-3.18 (2H, m), 3.41-3.48 (1H, m), 3.98-4.05 (2H, m, overlapping), 4.49 (1 H, m), 6.90-6.98 (3H, m, overlapping), 7.44 (2H, d, J = 8.6 Hz), 7.53 (1H, s), 7.65 (2H, d, J-8.6 Hz), 9.73 (1H, s). LCMS m/z = 498 (m + H⁺).

### Example J15

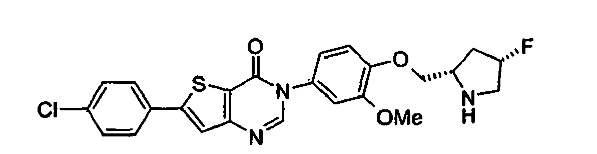

### 6-(4-chlorophenyl)-3-(4-{[(2S,4S)-4-fluoropyrrolidinyl]methoxy}-3-methoxyphenyl)thieno[3,2-d]pyrimidin-4(3H)-one

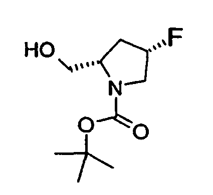

### tert-butyl (2S,4S)-4-fluoro-2-(hydroxymethyl)-1-pyrrolidinecarboxylate

To a THF (20 mL) solution of 1-*tert*-butyl 2-methyl (2*S*,4*S*)-4-fluoro-1,2-pyrrolidinedicarboxylate (950 mg, 3.9 mmol) at 0°C, 5.8 mL 2M LiBH₄ (11.6 mmol, 3 eq) was added slowly. The resulting mixture was left stirring overnight while allowing the temperature to warm up to room temperature. The reaction was quenched with 50% acetic acid with ice bath. The mixture was diluted with ethyl acetate. To this was added 50 mL silica gel and the mixture was stirred for 10 minutes. This was filtered and the filtrate was concentrated to give the intermediate (780 mg, 93%). ¹H NMR (CDCl₃) δ 1.48 (9H, s), 1.98-2.29 (2H, m, overlapping), 4.48-8.17 (5H, m, overlapping), 5.15 (1H, d, J = 43 Hz).

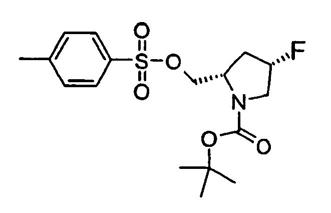

### tert-butyl (2S,4S)-4-fluoro-2-({[(4-methylphenyl)sulfonyl]oxy}methyl)-1-pyrrolidinecarboxylate

Reaction of the product from the previous step with p-toluene sulfonyl chloride under standard conditions provided the product.
¹H NMR (CDCl₃): δ 1.42 (9H, s), 2.04 (1 H, m), 2.34 (1 H, m), 2.45 (3H, s), 3.58 (2H, m, overlapping), 3.85 (1H, m), 4.11-4.31 (2H, m, overlapping), 5.19 (1H, d, J = 52.8 Hz), 7.35 (2H, m), 7.79 (2H, d, J = 8.4 Hz).

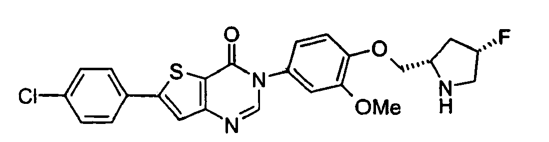

### 6-(4-chlorophenyl)-3-(4-{[(2S,4S)-4-fluoropyrrolidinyl]methoxy}-3-methoxyphenyl)thieno[3,2-d]pyrimidin-4(3H)-one

Alkylation of 6-(4-chlorophenyl)-3-(4-hydroxy-3-methoxyphenyl)thieno[3,2-*d*]pyrimidin-4(3*H*)-one (the preparation of which may be found in Example K1) (the preparation of which may be found in Example K1) with the product from the previous step was followed by removal of the BOC group with a 1:1 mixture of dichloromethane and trifluoroacetic acid to provide the title compound.
¹H NMR (CDCl₃): δ 1.98 (1 H, m), 2.25 (1 H, m), 3.00-3.12 (1 H, ddd, J = 34.9, 13.0, 4.0), 3.34-3.43 (1H, m), 3.87 (3H, s), 4.09 (2H, m), 5.18-5.34 (1H, d, J = 54.4 Hz), 6.88-6.95 (2H, m, overlapping), 7.03 (1 H, d, J = 8.5 Hz), 7.44 (2H, d, J = 8.5 Hz), 7.51 (1 H, s), 7.64 (2H, d, J = 8.4 Hz), 8.13 (1 H, s). LCMS m/z = 486 (m + H⁺).

### Example J16

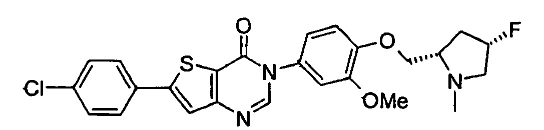

### 6-(4-chlorophenyl)-3-(4-{[(2S,4S)-4-fluoro-1-methylpyrrolidinyl]methoxy}-3-methoxyphenyl)thieno[3,2-d]pyrimidin-4(3H)-one

The title compound was obtained through treatment of the title compound from Example J15 with the procedures described in Example H18.
¹H NMR (CD₃OD) : (maleic acid salt) δ 2.26 (1H, m), 2.88 (1H, m), 3.25 (3H, s), 3.40-3.52 (1 H, dd, J = 39.6, 13.0 Hz), 3.90 (3H, s), 3.98 (1 H, m), 4.08 (1 H, m), 4.27 (1 H, t, J = 9.9 Hz), 4.54 (1 H, dd, J = 11.0, 3.5 Hz), 5.45 (1 H, d, J = 14.7 Hz), 6.24 (2H, s, maleic acid), 7.07 (1 H, d, J = 8.6 Hz), 7.18-7.22 (2H, m, overlapping), 7.51 (2H, d, J = 8.4 Hz), 7.72 (1 H, s), 7.82 (2H, d, J = 8.6 Hz), 8.34 (1 H, s). LCMS m/z = 500 (m + H⁺).

### Example J17

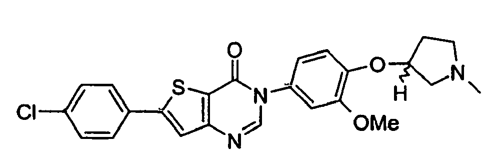

### 6-(4-chlorophenyl)-3-{3-methoxy-4-[(1-methyl-3-pyrrolidinyl)oxy]phenyl}thieno[3,2-d]pyrimidin-4(3H)-one

The title compound was obtained using procedures analogous to those described in Example H30.
¹H NMR (CDCl₃) δ 2.06 (1H, m), 2.34 (1H, m), 2.40 (3H, s), 2.53 (1H, m), 2.78 (2H, m) 2.93 (1 H, m), 3.86 (3H, s), 4.88 (1 H, m), 6.89-6.93 (2H, m, overlapping), 7.43 (2H, d, J = 8.6 Hz), 7.51 (1 H, s), 7.64 (2H, d, J = 8.6 Hz), 8.13 (1 H, s). LCMS m/z = 468 (m + H⁺).

### Example J18

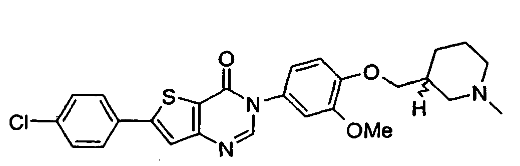

### 6-(4-chlorophenyl)-3-{3-methoxy-4-[(1-methyl-3-piperidinyl)methoxy]phenyl}thieno[3,2-d]pyrimidin-4(3H)-one

A solution containing 6-(4-chlorophenyl)-3-(4-hydroxy-3-methoxyphenyl)thieno[3,2-*d*]pyrimidin-4(3*H*)-one (77 mg, 0.20 mmol, the preparation of which may be found in Example K1) and 3-(chloromethyl)-1-methylpiperidine (74 mg) was mixed with Cs₂CO₃ (261 mg, 0.80 mmol) and the resultant mixture was heated at 85°C for 3 hours. The mixture was then cooled to room temperature, diluted with dichlormethane, washed with a saturated solution of NaHCO₃ and brine, dried (MgSO₄), and concentrated under reduced pressure. The resultant residue was purified by column chromatography to provide the title compound.
¹H NMR (CDCl₃) δ 1.15 (1H, m), 1.69-2.03 (5H, m), 2.23-2.27 (1H, m), 2.31 (3H, s), 2.78 (1 H, m), 2.99 (1H, m), 3.87 (3H, s), 3.94 (2H, m), 6.86-6.98 (3H, m, overlapping), 7.43 (2H, d, J = 8.3 Hz), 7.52 (1H, s), 7.65 (2H, d, J = 8.6 Hz), 8.12 (1 H, s). LCMS m/z = 496 (m + H⁺).

### Example J19

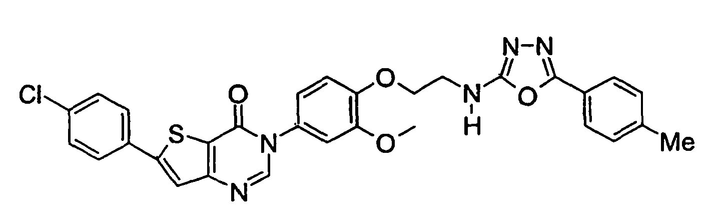

### 6-(4-chlorophenyl)-3-[3-methoxy-4-(2-{[5-(4-methylphenyl)-1,3,4-oxadiazol-2-yl]amino}ethoxy)phenyl]thieno[3,2-d]pyrimidin-4(3H)one

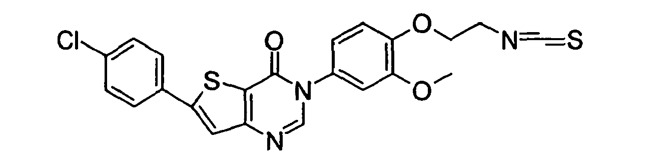

### 6-(4-chlorophenyl)-3-[4-(2-isothiocyanatoethoxy)-3-methoxyphenyl]thieno[3,2-d]pyrimidin-4(3H)-one

The primary amine product from Example H16 (0.82 mmol, 350 mg) was reacted with dipyridyllthionocarbonate (0.82 mmol, 190 mg) in DCM (10 mL) overnight. The solvent was removed by rotary evaporation. The residue was purified by flash chromatography eluting from straight DCM to 10% acetone in DCM giving the title compound (385 mg, 100%). ¹H NMR (CDCl₃) δ 3.91 (3H, s), 3.95 (2H, t, J = 5.5 Hz), 4.28 (2H, t, J = 5.5 Hz), 6.95 (1 H, dd, J = 8.4 Hz, 2,4 Hz), 7.00 (1 H, d, J = 2.4 Hz), 7.06 (1 H, d, J = 8.4 Hz), 7.45 (2H, dd, J = 6.6 Hz, 1.9 Hz), 7.54 (1 H, s), 7.66 (2H, dd, J = 6.6 Hz, 1.8 Hz), 8.14 (1 H, s). LCMS m/z = 470 (m + H⁺).

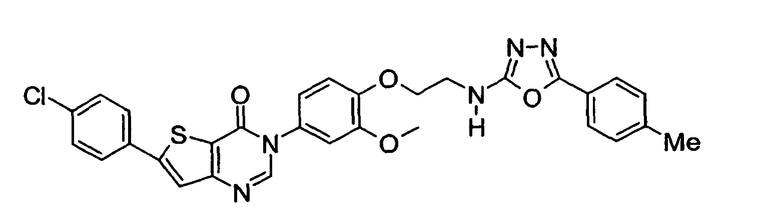

### 6-(4-chlorophenyl)-3-[3-methoxy-4-(2-{[5-(4-methylphenyl)-1,3,4-oxadiazol-2-yl]amino}ethoxy)phenyl]thieno[3,2-d]pyrimidin-4(3H)-one

The product from the preceding step (0.18 mmol, 85 mg) was reacted with 4-methylbenzohydrazide (0.20 mmol, 30 mg) in 2mL DMSO with 4 eq. EDC overnight. The reaction mixture was diluted with DCM and washed with water, saturated sodium bicarbonate solution. After drying over sodium sulfate, the solvent was removed by rotary evaporation. The final product was purified by recrystalization giving the title compound (86 mg, 82%). ¹H NMR (CDCl₃) δ 2.39 (3H, s), 3.88(5H, m, overlapping), 4.31 (2H, t, J= 4.9 Hz), 6.93 (1 H, dd, J = 8.4 Hz, 2.4 Hz), 6.98 (1 H, d, J = 2.4 Hz), 7.05 (1 H, d, J = 8.4 Hz), 7.25 (2H, m, overlapping with CDCL3), 7.44(2H, d, J = 8.6 Hz), 7.53 (1 H, s), 7.65 (2H, d, J = 8.7 Hz), 7.79 (2H, d, J = 8.3 Hz), 8.12 (1 H, s). LCMS m/z = 586 (m + H⁺).

### Example K1

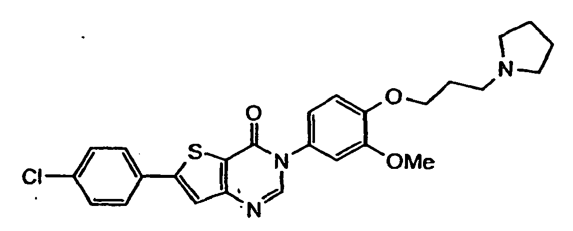

### 6-(4-Chlorophenyl)-3-[3-methoxy-4-(3-pyrrolidin-1-ylpropoxy)phenyl] thieno[3,2-d]pyrimidin-4(3H)-one

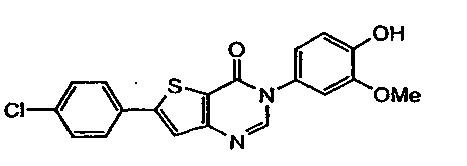

### 6-(4-Chlorophenyl)-3-(4-hydroxy-3-methoxyphenyl)thieno[3,2-d]pyrimidin-4(3H)-one

A dioxane (20 mL) solution of 2-methoxy-4-nitrophenol (6.76g, 0.04mol) with Pd(OH)₂/C (0.1g) was agitated on a Parr shaker apparatus under 310275 Nm⁻² (45 PSI) hydrogen pressure for 2 hours. The reaction mixture was removed to a nitrogen atmosphere, filtered through celite and added as a dioxane solution (30 mL) to methyl 5-(4-chlorophenyl)-3-{[(1Z)-(dimethylamino) methylidene]amino}thiophene-2-carboxylate (5.4g, 0.02mol, the preparation of which is found in Example J13). This solution was concentrated and refluxed overnight as a 40 mL absolute ethanol solution. When the reaction mixture was at room temperature the precipitated solid was filtered, and triturated with ethanol and then diethyl ether to give a white solid (2.7g, 0.007mol, 35%). LCMS m/z 385 (MH+). ¹H NMR (300 MHz, DMSO-d₆) δ 9.40 (s, 1H). 8.40 (s. 1H), 7.97 (s, 1H), 7.95 (d, 2H), 7.60 (d, 2H), 7.18 (s, 1H), 6.90 (m, 2H), 3.80 (s, 3H) ppm.

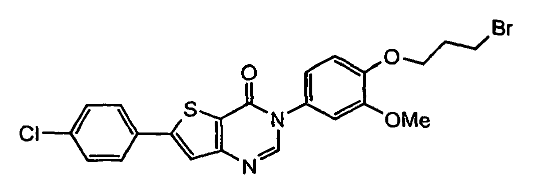

### 3-[4-(3-Bromopropoxy)-3-methoxyphenyl]-6-(4-chlorophenyl)thien[3,2-d]pyrimidin-4(3H)-one

A mixture of cesium carbonate (1.7g, 0.0052mol), 6-(4-chlorophenyl)-3-(4-hydroxy-3-methoxyphenyl)thieno[3,2-*d*]pyrimidin-4(3*H*)-one (0.5g, 0.0013mol), 1,3-dibromopropane (2.01g, 0.010mol) and DMF (30 mL) was warmed with intermittent mixing to 75°C overnight. The mix was then cooled to RT and diluted with a mixture of ether and water. The precipitated solid was filtered and then triturated with ether to give a white powder (0.60g, 92%). LCMS m/z 506 (MH+). ¹H NMR (300 MHz, DMSO-d₆) δ 8.40 (s, 1H), 8.00 (s, 1H), 7.95 (d, 2H), 7.60 (d, 2H), 7.22 (s, 1H), 7.18 (d, 1H), 7.05 (d, 1H), 4.18 (t, 2H), 3.80 (s, 3H), 3.70 (t, 2H), 2.30 (m, 2H) ppm.

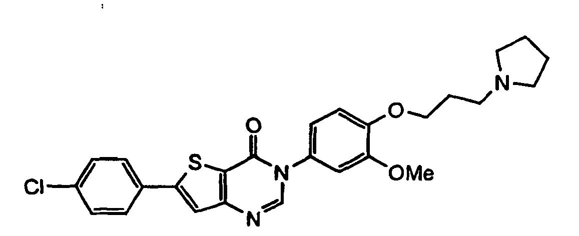

### 6-(4-Chlorophenyl)-3-[3-methoxy-4-(3-pyrrolidin-1-ylpropoxy)phenyl] thieno[3,2-d]pyrimidin-4(3H)-one.

A DMF (0.25 mL) solution of the intermediate from the preceding step (.050g, .0001 mol) and pyrrolidine was agitated at RT overnight, diluted with ether/water, filtered to give a yellow solid (0.026g, 53%). LCMS m/z 496 (MH+).

### Examples K2 - K12 were prepared according to the procedures detailed for Example K1.

### Example K2

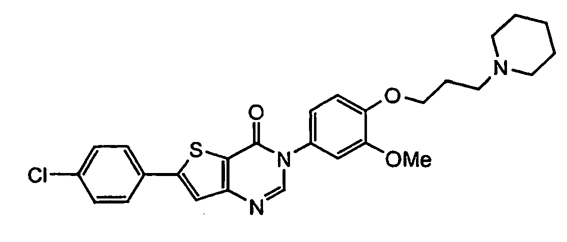

### 6-(4-Chlorophenyl)-3-[3-methoxy-4-(3-piperidin-1-ylpropoxy)phenyl] thieno[3,2-d]pyrimidin-4(3H)-one.

LCMS m/z 510 (MH+).

### Example K3

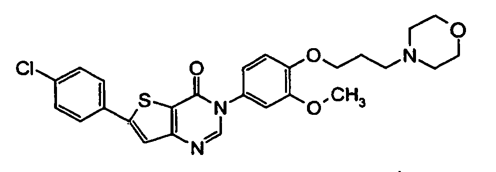

### 6-(4-Chlorophenyl)-3-[3-methoxy-4-(3-morpholin-4 ylpropoxy)phenyl] thieno[3,2-d]pyrimidin-4(3H)-one

LCMS m/z 512 (MH+). ¹H NMR (300 MHz, DMSO-d₆) δ 8.40 (s, 1H), 8.00 (s, 1H), 7.95 (d, 2H), 7.60 (d, 2H), 7.22 (s, 1 H), 7.10 (d, 1 H), 7.02 (d, 1 H), 4.05 (t, 2H), 3.80 (s, 3H), 3.60 (m, 4H), 2.45 (t, 2H), 2.40 (m, 4H), 1.90 (m, 2H) ppm.

### Example K4

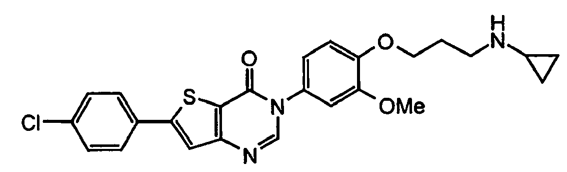

### 6-(4-Chlorophenyl)-3-{4-[3-(cyclopropylamino)propoxy]-3-methoxyphenyl}thieno[3,2-d]pyrimidin-4(3H)-one

LCMS m/z 482 (MH+). ¹H NMR (300 MHz, DMSO-d₆) δ 8.40 (s, 1H), 8.00 (s, 1H), 7.95 (d, 2H), 7.60 (d, 2H), 7.20(s, 1H), 7.10 (d, 1H), 7.02 (d, 1H), 4.10 (t, 2H), 3.80 (s, 3H), 2.78 (t, 2H), 2.25 (br, 1H), 2.09 (t, 1 H), 1.90 (m, 2H), 0.40 (m, 2H), 0.20 (m, 2H) ppm.

### Example K5

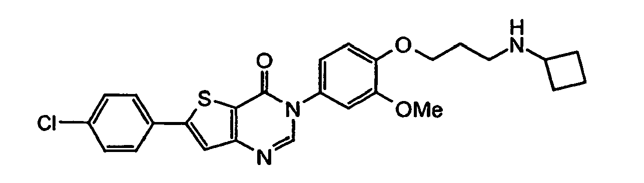

### 6-(4-Chlorophenyl)-3-{4-[3-(cyclobutylamino)propoxy]-3-methoxyphenyl}thieno[3,2-d]pyrimidin-4(3H)-one

LCMS m/z 496 (MH+). ¹H NMR (300 MHz, DMSO-d₆) δ 8.40 (s, 1H), 8.00 (s, 1H), 7.95 (d, 2H), 7.60 (d, 2H), 7.20 (s, 1H). 7.10 (d, 1H), 7.02 (d, 1 H), 4.10 (t, 2H), 3.80 (s, 3H), 3.30 (m, 1H), 3.20 (t, 1H), 2.60 (t, 2H), 2.10 (m, 2H), 1.83 (m, 2H), 1.61 (m, 4H) ppm.

### Example K6

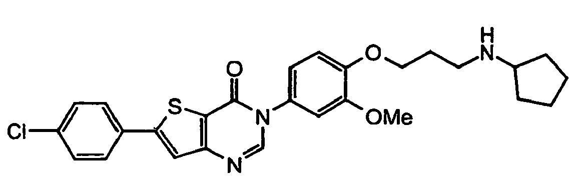

### 6-(4-Chlorophenyl)-3-{4-[3-(cyclopentylamino)propoxy]-3-methoxyphenyl}thieno[3,2-d]pyrimidin-4(3H)-one

LCMS m/z 510 (MH+). ¹H NMR (300 MHz, DMSO-d₆) δ 8.40 (s, 1H), 8.00 (s, 1H), 7.95 (d, 2H), 7.60 (d, 2H), 7.20 (s, 1H), 7.10 (d, 1 H), 7.02 (d, 1H), 4.10 (t, 2H), 3.80 (s, 3H), 3.30 (m, 1H), 3.00 (t, 1 H), 2.61 (t, 2H), 1.90 (t, 2H), 1.70 (m, 2H), 1.61 (m, 2H), 1.45 (m, 2H), 1.30 (m, 2H) ppm.

### Example K7

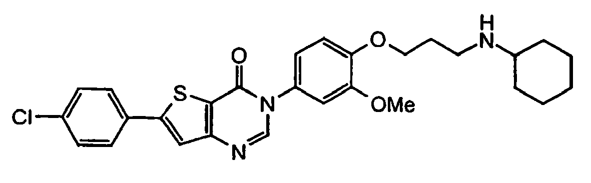

### 6-(4-Chlorophenyl)-3-{4-[3-(cyclohexylamino)propoxy]-3-methoxyphenyl}thieno[3,2-d]pyrimidin-4(3H)-one

LCMS m/z 524 (MH+). ¹H NMR (300 MHz, DMSO-d₆) δ 8.40 (s, 1H), 8.00 (s, 1 H), 7.95 (d, 2H), 7.60 (d, 2H), 7.20 (s, 1 H), 7.10 (d, 1 H), 7.02 (d, 1H), 4.10 (t, 2H), 3.80 (s, 3H), 3.30 (m, 1H), 3.00 (t, 1 H), 2.61 (t, 2H), 1.90 (t, 2H), 1.70 (m, 2H), 1.61 (m, 2H), 1.45 (m, 2H), 1.30 (m, 2H) ppm.

### Example K8

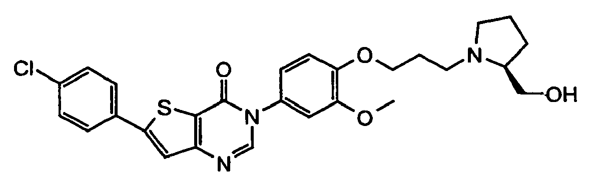

### 6-(4-Chlorophenyl)-3-(4-{3-[(2S)-2-(hydroxymethyl)pyrrolidin-1 - yl]propoxy}-3-methoxyphenyl) thieno[3,2-d]pyrimidin-4(3H)-one

LCMS m/z 526 (MH+).

### Example K9

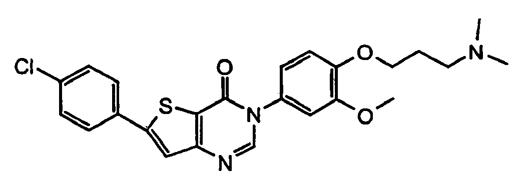

### 6-(4-Chlorophenyl)-3-{4-[3-(dimethylamino)propoxy]-3-methoxyphenyl}thieno[3,2-d]pyrimidin-4(3H)-one

LCMS m/z 470 (MH+).

### Example K10

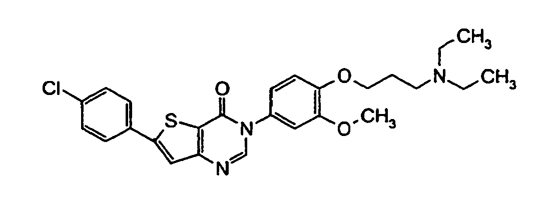

### 6-(4-Chlorophenyl)-3-{4-[4-(diethylamino)propoxy]-3-methoxyphenyl}thieno[3,2-dd]pyrimidin-4(3H-one

LCMS m/z 498 (MH+).

### Example K11

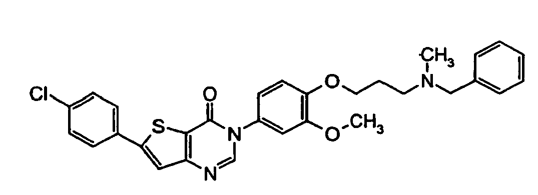

### 3-(4-{3-[benzyl(methyl)amino]propoxy}-3-methoxyphenyl)-6-(4-chlorophenyl)thieno[3,2-d]pyrimidin-4(3H)-one

LCMS m/z 546 (MH+).

### Example K12

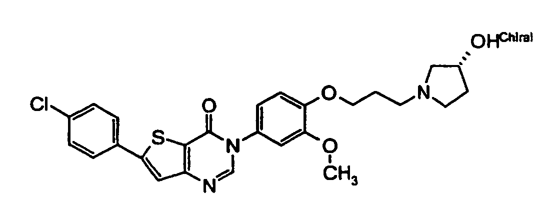

### 6-(4-Chlorophenyl)-3-(4-{3-[(3R)-3-hydroxypyrrolidin-1-yl]propoxy}-3-methoxyphenyl)thieno[3,2-d]pyrimidin-4(3H)-one

LCMS m/z 512 (MH+). ¹H NMR (300 MHz, DMSO-d₆) δ 8.40 (s, 1 H), 8.00 (s, 1H). 7.95 (d, 2H), 7.60 (d, 2H), 7.20 (s, 1H), 7.10 (d, 1H), 7.02 (d, 1H), 4.63 (d, 1 H), 4.18 (m, 1 H), 4.09 (t, 2H), 3.80 (s, 3H), 3.75 (m, 2H), 3.30-3.50 (m, 2H), 2.78 (t, 2H), 2.22-2.70 (m, 2H), 1.90 (m, 2H) ppm.

### Example K13

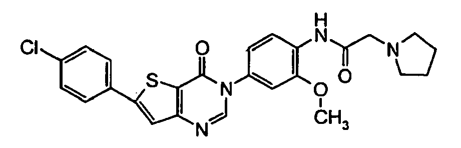

### N-{4-[6-(4-chlorophenyl)-4-oxothieno[3,2-d]pyrimidin-3(4H)-yl]-2-methoxyphenyl}-2-pyrrolidin-1-ylacetamide

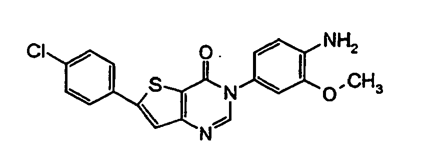

### 3-(4-Amino-3-methoxyphenyl)-6-(4-chlorophenyl)thieno[3,2-d]pyrimidin-4(3H)-one

The product was prepared according to the manner of 6-(4-chlorophenyl)-3-(4-hydroxy-3-methoxyphenyl)thieno[3,2-*d*]pyrimidin-4(3*H*)-one (the preparation of which may be found in Example K1), using 2-methoxybenzene-1,4-diamine to give the product as a yellow powder (0.19g, 50%).
LCMS m/z 384 (MH+). ¹H NMR (300 MHz, DMSO-d₆) δ 8.40 (s, 1H), 8.00 (s, 1 H), 7.95 (d, 2H), 7.60 (d, 2H), 7.00 (s, 1 H), 6.80 (d, 1H), 6.75 (d, 1 H), 5.05 (s, 2H), 3.80 (s, 3H), ppm.

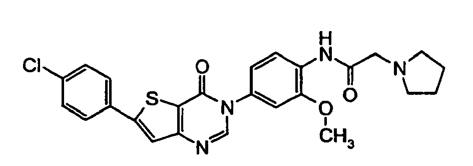

### N-{4-(6-(4-chlorophenyl)-4-oxothieno[3,2-d]pyrimidin-3(4H)-yl]-2-methoxyphenyt}-2-pyrrolidin-1-ylacetamide

The material from the preceding step was reacted with chloroacetyl chloride followed by reaction with pyrrolidine to provide the title compound as a golden powder (0.028g, 44%). LCMS m/z 495 (MH+). ¹H NMR (300 MHz, DMSO-d₆) δ 9.65 (s, 1 H), 8.42 (s, 1H), 8.35 (d, 1 H), 8.00 (s, 1 H), 7.95 (d, 2H), 7.60 (d, 2H), 7.35 (s, 1H), 7.10 (d, 1H), 3.90 (s, 3H), 3.42 (m, 2H), 2.60 (m, 4H), 1.80 (m, 4H) ppm.

### Example K14

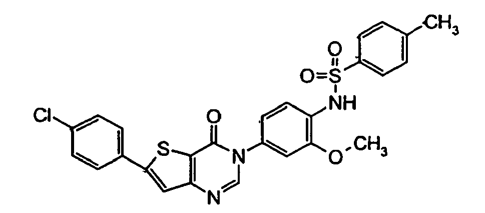

### N-{4-[6-(4-chlorophenyl)-4-oxothieno[3,2-d]pyrimidin-3(4H)-yl]-2-methoxyphenyl}-4-methylbenzenesulfonamide

A pyridine (1 mL) solution of 3-(4-Amino-3-methoxyphenyl)-6-(4-chlorophenyl)thieno[3,2-*d*]pyrimidin-4(3*H*)-one (0.050g, .0013mol, the preparation of which is found in Example K13) and p-toluenesulfonyl chloride and was agitated overnight at RT, then diluted with water and filtered to give a white powder (.056 g 81%). LCMS m/z 538 (MH+). ¹H NMR (300 MHz, DMSO-d₆) δ 9.72 (s, 1 H), 8.40 (s, 1 H), 8.00 (s, 1 H), 7.95 (d, 2H), 7.70 (d, 2H), 7.60 (d, 2H), 7.38 (d, 2H), 7.35 (d, 1H), 7.20 (s, 1 H), 7.05 (d, 1H), 3.62 (s, 3H), 2.41 (s, 3H) ppm.

### Example K15

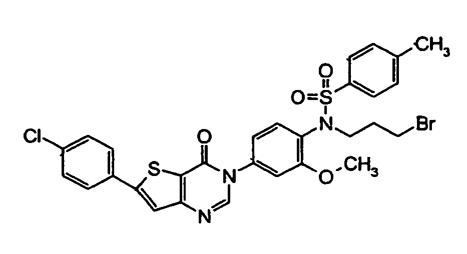

### N-(3-bromopropyl)-N-{4-[6-(4-chlorophenyl)-4-oxothieno[3,2-d]pyrimidin-3(4H)-yl]-2-methoxyphenyl}-4-methylbenzenesulfonamide

A mixture of cesium carbonate (2.9g, 0.0088mol), N-{4-[6-(4-chloro-phenyl)-4-oxothieno[3,2-d]pyrimidin-3(4H)-yl]-2-methoxyphenyl}-4-methylbenzenesulfonamide (1.2g, 0.0022mol, the title compound from Example K14), 1,3-dibromopropane (3.6 g, 0.018mol) and DMF (30 mL) was warmed with intermittent mixing to 75°C overnight and then when at RT, diluted with a mixture of ether and water. The precipitated solid was filtered and then triturated with ether to give a white powder (1.2g, 83%). LCMS m/z 659 (MH+). ¹H NMR (300 MHz, DMSO-d₆) δ 8.50 (s, 1H), 8.00 (s, 1H), 7.95 (d, 2H), 7.60 (d, 4H), 7.40 (d, 2H), 7.32 (s, 1H), 7.30 (d, 1H), 7.18 (d, 1H), 3.62 (t, 2H), 3.58 (t, 2H), 3.50 (s, 3H), 2.41 (s, 3H), 1.95 (t, 2H) ppm.

### Example K16

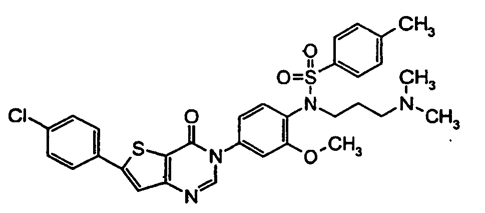

### N-{4-[6-(4-Chlorophenyl)-4-oxothieno[3,2-d]pyrimidin-3(4H)-yl]-2-methoxyphenyl}-N-[3-(dimethylamino)propyl]-4-methylbenzene-sulfonamide

The title compund was prepared by reaction of N-(3-bromopropyl)-N-{4-[6-(4-chlorophenyl)-4-oxothieno[3,2-d]pyrimidin-3(4H)-yl]-2-methoxyphenyl}-4-methylbenzenesulfonamide (the title compound from Example K15) and dimethylamine. LCMS m/z 623 (MH+).

### Examples K17-K19 were prepared through procedures analogous to those found in the preparation of Example K16.

### Example K17

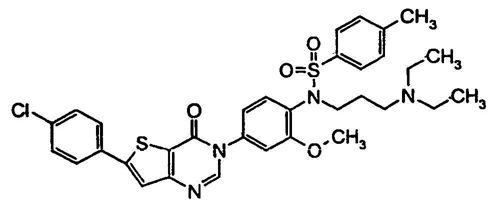

### N-{4-[6-(4-chlorophenyl)-4-oxothieno[3,2-d]pyrimidin-3(4H)-yl]-2-methoxyphenyl}-N-[3-(diethylamino)propyl]-4-methylbenzene-sulfonamide

LCMS m/z 651 (MH+).

### Example K18

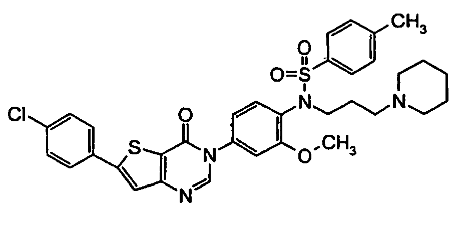

### N-{4-[6-(4-chlorophenyl)-4-oxothieno[3,2-d]pyrimidin-3(4H)-yl]-2-methoxyphenyl}-4-methyl-N-(3-piperidin-1-ylpropyl)benzenesulfonamide

LCMS m/z 663 (MH+).

### Example K19

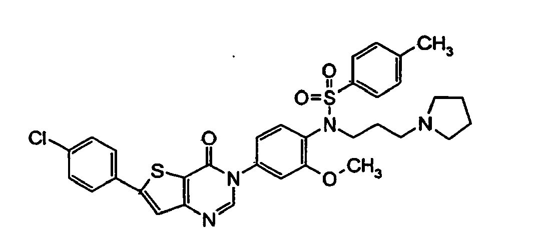

### N-{4-[6-(4-chlorophenyl)-4-oxothieno[3,2-d]pyrimidin-3(4H)-yl]-2-methoxyphenyl}-4-methyl-N-(3-pyrrolidin-1-ylpropyl)benzenesulfonamide

LCMS m/z 649 (MH⁺).

### Example K20

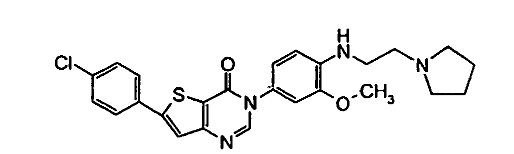

### 6-(4-chlorophenyl)-3-{3-methoxy-4-[(2-pyrrolidin-1-ylethyl)amino] phenyl}thieno[3,2-d]pyrimidin-4(3H)-one

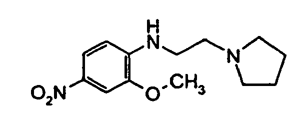

### 2-methoxy-4-nitro-N-(2-pyrrolidin-1-ylethyl)aniline

A neat mixture of 1-chloro-2-methoxy-4-nitrobenzene (2.0g, 10.7mmol) and 2-pyrrolidin-1-ylethanamine (2.5g, 22mmol) was heated to 75°C overnight, then chromatographed on silica gel with ethanol (100%) to give a yellow solid (0.67g, 24%).
LCMS m/z 266 (MH+). ¹H NMR (300 MHz, DMSO-d₆) δ 7.63 (d, 1H). 7.56 (s, 1H), 6.67 (d, 1H), 6.45 (br, 1 H), 3.90 (s, 3H), 3.38 (m, 2H), 2.77 (m, 2H), 2.63 (m, 2H), 2.50 (m, 2H), 1.73 (m, 4H) ppm.

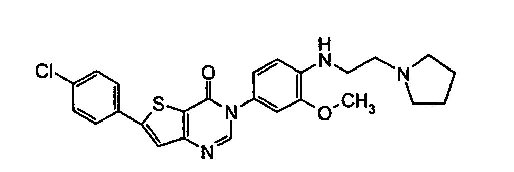

### 6-(4-chlorophenyl)-3-{3-methoxy-4-[(2-pyrrolidin-1-ylethyl)amino] phenyl}thieno[3,2-d]pyrimidin-4(3H)-one

The title compound was prepared from the product of the previous step by procedures analogous to those found in Example H30.
LCMS m/z 481 (MH+).
¹H NMR (300 MHz, DMSO-d₆) δ 8.40 (s, 1H), 8.00 (s, 1H), 7.95 (d, 2H), 7.60 (d, 2H), 7.00 (s, 1 H), 6.95 (d, 1 H), 6.60 (d, 1 H), 5.10 (t, 1 H), 3.80 (s, 3H), 3.20 (m, 2H), 2.69 (m, 2H), 2.50 (m, 4H), 1.70-(m, 4H) ppm.

### Examples K21-K23 were prepared through reactions of the title compound of Example K20 with an appropriate acylating reagent.

### Example K21

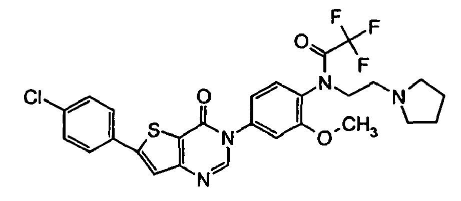

### N-{4-[6-(4-chlorophenyl)-4-oxothieno[3,2-d]pyrimidin-3(4H)-yl]-2-methoxyphenyl}-2,2,2-thrifluoro-N-(2-pyrrolidin-1-ylethyl)acetamide

LCMS m/z 577 (MH+). ¹H NMR (300 MHz, DMSO-d₆) δ 8.40 (s, 1H), 8.00 (s, 1 H), 7.95 (d, 2H), 7.60 (d, 2H), 7.00 (s, 1 H), 6.95 (d, 1H), 6.60 (d, 1 H), 5.10 (t, 1 H), 3.80 (s, 3H), 3.20 (m, 2H), 2.69 (m, 2H), 2.50 (m, 4H), 1.70 (m, 4H) ppm.

### Example K22

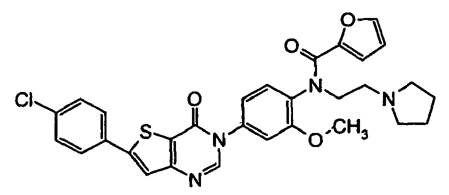

### N-{4-[6-(4-chlorophenyl)-4-oxothieno[3,2-d]pyrimidin-3(4H)-yl]-2-methoxyphenyl}-N-(2-pyrrolidin-1-ylethyl)-2-furamide

LCMS m/z 575 (MH+). ¹H NMR (300 MHz, DMSO-d₆) δ 8.50 (s, 1 H), 8.05 (s, 1H), 7.95 (d, 2H), 7.90 (s, 1 H), 7.60 (d, 2H), 7.42 (s, 1 H), 7.22 (d, 1 H), 7.20 (s, 1 H), 6.60 (m, 2H), 4.10 (m, 2H), 3.63 (m, 2H), 3.70 (s, 3H), 2.80 (m, 4H), 1.80 (m, 4H) ppm.

### Example K23

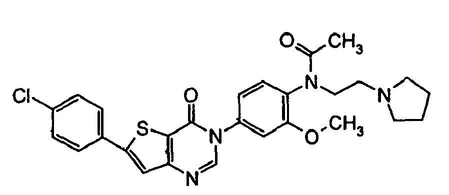

### N-{4-[6-(4-chlorophenyl)-4-oxothieno[3,2-d]pyrimidin-3(4H)-yl]-2-methoxyphenyl}-N-(2-pyrrolidin-1-ylethyl)acetamide

LCMS m/z 523 (MH+). ¹H NMR (300 MHz, DMSO-d₆) δ 8.50 (s, 1H), 8.05 (s, 1H), 7.95 (d, 2H), 7.60 (d, 2H), 7.50 (s, 1H), 7.42 (d, 1 H), 7.22 (d, 1 H), 3.90 (t, 2H), 3.80 (s, 3H), 3.38-3.50 (m, 2H), 2.40 (m, 4H), 1.75 (s, 3H), 1.65 (m, 4H) ppm.

### Example K24

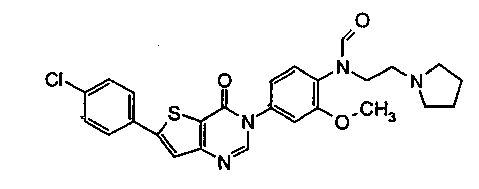

### 4-[6-(4-Chlorophenyl)-4-oxothieno[3,2-d]pyrimidin-3(4H)-yl]-2-methoxyphenyl(2-pyrrolidin-1-ylethyl)formamide

A solution of 6-(4-Chlorophenyl)-3-{3-methoxy-4-[(2-pyrrolidin-1-ylethyl)amino] phenyl}thieno[3,2-*d*]pyrimidin-4(3*H*)-one (.050g, 0.1 mmol) in formic acid (88%, 2 mL) was heated to reflux, concentrated, and then diluted with aqueous sodium hydroxide (0.1 N, 2 mL) to precipitate the product as a white solid (.051g, 100%). LCMS m/z 509 (MH⁺). ¹H NMR (300 MHz, DMSO-d₆) δ 8.50 (s, 1 H), 8.10 (s, 1 H), 8.00 (s, 1H), 7.95 (d, 2H), 7.60 (d, 2H), 7.43 (d, 1 H), 7.42 (s, 1H), 7.20 (d, 1 H), 3.82 (s, 3H), 3.78 (t, 2H), 3.30-3.50 (m, 2H), 2.40 (m, 4H), 1.70 (m, 4H) ppm.

### Example K25

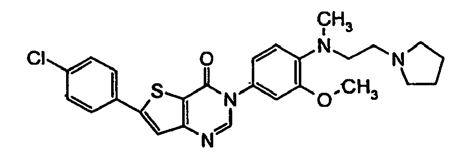

### 6-(4-Chlorophenyl)-3-(3-methoxy-4-[methyl(2-pyrrolidin-1-ylethyl)-amino]phenyl}thieno[3,2-d]pyrimidin-4(3H)-one

The title compound was obtained through treatment of the title compound from Example K20 with the procedures described in Example H18. LCMS m/z 495 (MH+). ¹H NMR (300 MHz, DMSO-d₆) δ 8.40 (s, 1H), 8.00 (s, 1H), 7.95 (d, 2H), 7.60 (d, 2H), 7.20 (s, 1H), 7.05 (m, 2H), 3.80 (s, 3H), 3.40-3.50 (m, 4H), 3.30 (m, 4H), 2.80 (s, 3H), 1.70 (m, 4H) ppm.

### Example K26

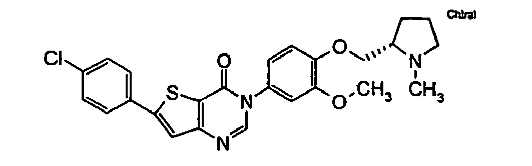

### 6-(4-Chlorophenyl)-3-(3-methoxy-4-{[(2S)-1-methylpyrrolidin-2-yl] methoxy}phenyl)thieno[3,2-d]pyrimidin-4(3H)-one

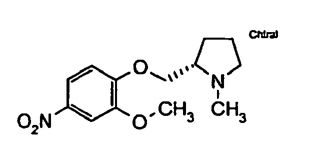

### (2S)-2-[(2-methoxy-4-nitrophenoxy)methyl]-1-methylpyrrolidine

A mixture of [(2S)-1-methylpyrrolidin-2-yl]methanol (11.5g, 0.10mo1), DMF (150 mL) and NaH (4.0 g, 60% in mineral oil, 0.1 mol) was agitated for 30 minutes under an atmosphere of nitrogen. A DMF (100mL) solution of 1-chloro-2-methoxy-4-nitrobenzene (18.7g, 0.10mol) was added and the mixture agitated overnight at RT. The mixture was concentrated, diluted with ethyl acetate, extracted three times with water, dried, filtered, then concentrated to give the product as a tan oil (16.78g, 63%). LCMS m/z 267 (MH+). ¹H NMR (300 MHz, DMSO-d₆) δ 7.88 (d, 1H), 7.73 (s, 1H), 7.19 (d, 1H), 4.06 (m, 2H), 3.88 (s, 3H), 2.80 (m, 1 H), 2.60 (m, 1 H), 2.36 (s, 3H), 2.20 (m, 1H). 1.90 (m, 1 H), 1.67 (m, 3H) ppm.

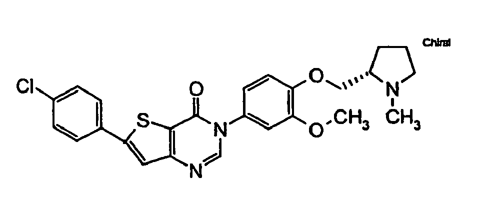

### 6-(4-Chlorophenyl)-3-(3-methoxy-4-{[(2S)-1-methylpyrrolidin-2-yl] methoxy}phenyl)thieno[3,2-d]pyrimidin-4(3H)-one

The title compound was prepared from the product of the previous step by employing procedures analogous to those found in Example H30.
LCMS m/z 482 (MH+). ¹H NMR (300 MHz, DMSO-d₆) δ 8.40 (s, 1H), 8.00 (s, 1 H), 7.95 (d, 2H), 7.60 (d, 2H), 7.20 (s, 1 H), 7.10 (d, 1H), 7.02 (d, 1H), 3.8-4.10 (m, 2H), 3.80 (s, 3H), 2.95 (t, 1 H), 2.60 (m, 1H), 2.40 (s, 3H), 2.20 (q, 1 H), 1.95 (m, 1 H), 1.50-1.80 (m, 3H) ppm.

### Example K27

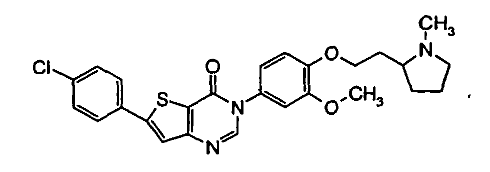

### 6-(4-Chlorophenyl)-3-{3-methoxy-4-[2-(1-methylpyrrolidin-2-yl)ethoxy] phenyl}thieno[3,2-d]pyrimidin-4(3H)-one

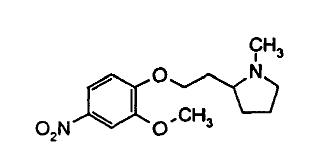

### 2-[2-(2-methoxy-4-nitrophenoxy)ethyl]-1-methylpyrrolidine

This intermediate was prepared using procedures analogous to those found in Example K26.
LCMS m/z 281 (MH+) ¹H NMR (300 MHz, DMSO-d₆) δ 7.88 (d, 1H), 7.75 (s, 1 H), 7.19 (d, 1H), 4.18 (t, 2H), 3.88 (s, 3H), 2.90 (m, 1 H), 2.20 (s, 3H), 2.10 (m, 2H), 1.60 (m, 2H), 1.90 (m, 1 H), 1.67 (m, 3H) ppm.

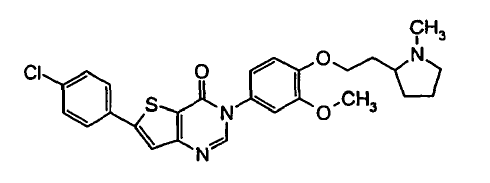

### 6-(4-Chlorophenyl)-3-(3-methoxy-4-[2-(1-methylpyrrolidin-2-yl)ethoxy] phenyl}thieno[3,2-d]pyrimidin-4(3H)-one

The title compound was obtained using procedures analogous to those described in Example K26.
LCMS m/z 496 (MH+). ¹H NMR (300 MHz, DMSO-d₆) δ 8.40 (s, 1 H), 8.00 (s, 1H), 7.95 (d, 2H), 7.60 (d, 2H), 7.20 (s, 1 H), 7.00-7.20 (m, 2H), 4.10 d(t, 2H), 3.80 (s, 3H), 3.00 (m, 3H), 2.20 (s, 3H), 2.10 (m, 1 H), 1.97 (m, 1 H), 1.70 (m, 4H) ppm.

### Example K28

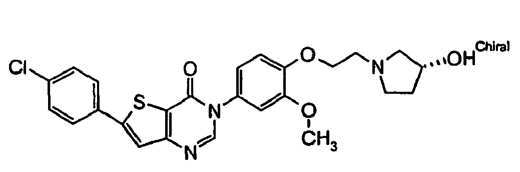

### 6-(4-Chlorophenyl)-3-(4-{2-[(3R)-3-hydroxypyrrolidin-1-yl]ethoxy}-3-methoxyphenyl)thieno[3,2-d]pyrimidin-4(3H)-one

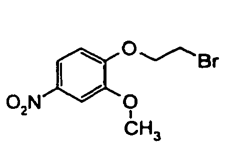

### 1-(2-bromoethoxy)-2-methoxy-4-nitrobenzene

A mixture of potassium carbonate (27.6g, 0.2mol), 2-methoxy-4-nitrophenol (16.9g, 0.1 mol), 1,2-dibromoethane (94g, 0.50mol) and acetonitrile was refluxed for 2 days, concentrated, extracted in ethyl acetate with water three times, dried, filtered, concentrated to a white powder, triturated with ether, filtered, then concentrated to give a pale yellow powder (15.8g, 58%). ¹H NMR (300 MHz, DMSO-d₆) δ 7.90 (d, 1H), 7.80 (s, 1H), 7.20 (d, 1H), 4.55 (t, 2H), 3.80 (s, 3H), 3.75 (t, 2H) ppm.

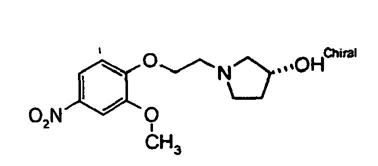

### (3R)-1 -[2-(2-methoxy-4-nitrophenoxy)ethyl]pyrrolidin-3-ol.

A mixture of the product from the preceding step (5.52g, .02mol), (3R)-pyrrolidin-3-ol (3.48g, .04mol) and dioxane was mixed at RT overnight, diluted with aqueous NaOH (20 mL, 1 N), extracted in ethyl acetate three times with water, dried, filtered and concentrated to a golden viscous oil (3.08g, .011 mol). LCMS m/z 283 (MH+). ¹H NMR (300 MHz, DMSO-d₆) δ 7.90 (d, 1H), 7.80 (s, 1H), 7.20 (d, 1 H), 4.70 (d, 1 H), 4.50 (t, 1H), 4.20 (t, 2H), 3.89 (m, 2H), 3.81 (s, 3H), 2.80 (m, 2H), 2.6 (q, 1H), 2.40 (m, 1H), 1.97 (m, 1 H), 1.50 (m, 1 H) ppm.

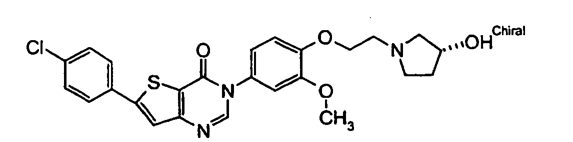

### 6-(4-Chlorophenyl)-3-(4-{2-[(3R)-3-hydroxypyrrolidin-1-yl]ethoxy)-3-methoxyphenyl)thieno[3,2-d]pyrimidin-4(3H)-one

The title compound was obtained from the product of the previous step by employing procedures analogous to those found in Example H30. LCMS m/z 498 (MH+). ¹H NMR (300 MHz, DMSO-d₆) δ 8.40 (s, 1H), 8.00 (s, 1H), 7.95 (d, 2H), 7.60 (d, 2H), 7.20 (s, 1H), 7.11 (d, 1H), 7.04 (d, 1H), 4.70 (d, 1H), 4.20 (br, 1 H), 4.10 (t, 2H), 3.80 (s, 3H), 2.82 (m, 2H), 2.70 (m, 1H), 2.40 (m, 1 H), 2.00 (m, 1 H), 1.58 (m, 3H) ppm.

### Example K29

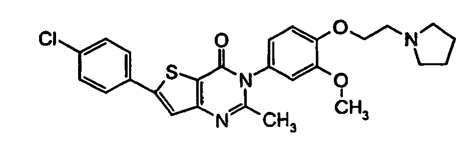

### 6-(4-Chlorophenyl)-3-[3-methoxy-4-(2-pyrrolidin-1-ylethoxy)phenyl]-2-methylthieno[3,2-d]pyrimidin-4(3H)-one

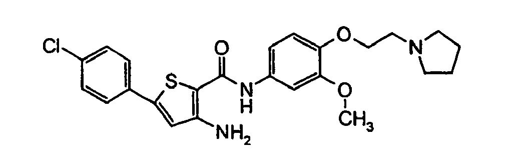

### 3-Amino-5-(4-chlorophenyl)-N-[3-methoxy-4-(2-pyrrolidin-1-ylethoxy) phenyl]thiophene-2-carboxamide.

Aqueous NaOH (4 mL, 1 N) was added to a DMSO solution of 6-(4-chlorophenyl)-3-[3-methoxy-4-(2-pyrrolidin-1-ylethoxy)phenyl] thieno[3,2-*d*]pyrimidin-4(3*H*)-one (0.50g, 0.001 mol) at 170°C. After 5 minutes a gummy precipitate was triturated with water and solidified to a tan solid (0.30g, 60%). LCMS m/z 472 (MH+). ¹H NMR (300 MHz, DMSO-d₆) δ 9.20 (s, 1 H), 7.65 (d, 2H), 7.55 (d, 2H), 7.35 (s, 1 H), 7.20 (d, 1 H), 7.04 (s, 1H), 6.90 (d, 1H), 6.63 (br s, 2H), 4.00 (t, 2H), 3.75 (s, 3H), 2.75 (t, 2H), 2.55 (m, 4H), 1.65 (m, 4H) ppm.

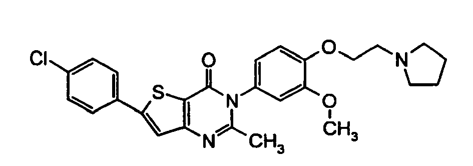

### 6-(4-Chlorophenyl)-3-[3-methoxy-4-(2-pyrrolidin-1-ylethoxy)phenyl]-2-methylthieno[3,2-d]pyrimidin-4(3H)-one

A mixture of 3-amino-5-(4-chlorophenyl)-*N*-[3-methoxy-4-(2-pyrrolidin-1-ylethoxy) phenyl]thiophene-2-carboxamide and glacial acetic acid was refluxed overnight, concentrated to an amber viscous oil, triturated with water then filtered to give the title compound as its acetic acid salt as a tan solid (.032g, 64%). LCMS m/z 496 (MH+). ¹H NMR (300 MHz, DMSO-d₆) δ 8.35 (br, 1H), 8.00 (s, 1H), 7.95 (d, 2H), 7.55 (d, 2H), 7.20 (s, 1H), 7.10 (d, 1H), 6.95 (d, 1H), 4.20 (m, 2H), 3.75 (s, 3H), 2.75 (m, 2H), 2.55 (m, 4H), 2.20 (s, 3H), 2.00 (s, 3H), 1.65 (m, 4H) ppm.

### Example K30

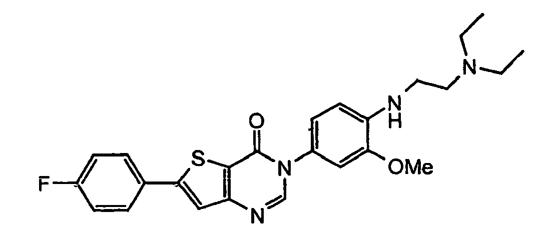

### 3-(4-{[2-(diethylamino)ethyl]amino}-3-methoxyphenyl)-6-(4-fluorophenyl) thieno[3,2-d]pyrimidin-4(3H)-one

The title compund was obtained by employing procedures analogous to those described in Example K20.
LCMS m/z = 467 (m + H+).
¹H NMR (DMSO-D6): δ 8.38 (s, 1 H); 7.98 (d, 2H); 7.90 (s, 1H)); 7.38 (d, 2H), 7.00 (s, 1 H), 6.95 (d, 1 H); 6.62 (d, 1 H); 5.20 (t, 1 H); 3.80 (s, 3H); 3.25-3.55 (m, 4H; 3.28 (m, 2H); 3.62 (t, 2H); 1.00 (t, 6H).

### Example K31

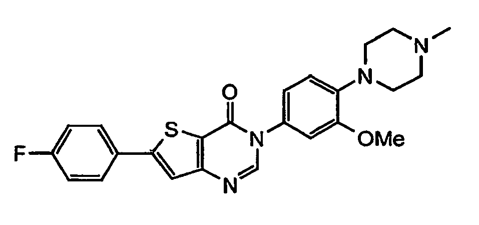

### 6-(4-Fluorophenyl)-3-[3-methoxy-4-(4-methylpiperazin-1 - yl)phenyl]thieno[3,2-d]pyrimidin-4(3H)-one

The title compund was obtained by employing procedures analogous to those described in Example K20.
LCMS m/z = 451 (m + H+).
¹H NMR (DMSO-D6): δ 8.40 (s, 1 H); 7.98 (d, 2H); 7.90 (s, 1H)); 7.38 (d, 2H), 7.18 (s, 1 H), 7.05 (m, 2H); 3.80 (s, 3H); 3.25-3.55 (m, 4H; 3.01 (m, 4H); 2.20 (s, 3H).

### Example K32

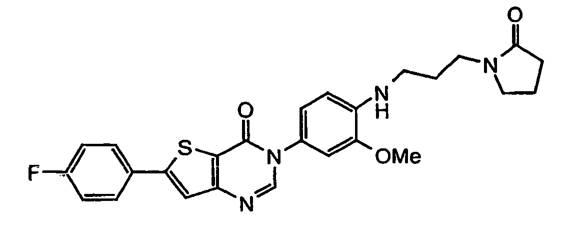

### 6-(4-Fluorophenyl)-3-(3-methoxy-4-{[3-(2-oxopyrrolidin-1-yl)propyl]amino}phenyl) thieno[3,2-d]pyrimidin-4(3H)-one

The title compund was obtained by employing procedures analogous to those described in Example K20.
LCMS m/z = 493 (m + H+).
¹H NMR (DMSO-D6): δ 8.40 (s, 1H); 7.98 (d, 2H); 7.90 (s, 1H)); 7.38 (d, 2H), 7.00 (s, 1 H), 6.90 (d, 1 H); 6.60 (d, 1H); 5.28 (t, 1 H); 3.80 (s, 3H); 3.25-3.55 (m, 4H); 3.12 (q, 2H); 2.20 (t, 2H); 1.95 (qnt, 2H); 1.77 (m, 2H).

### Example K33

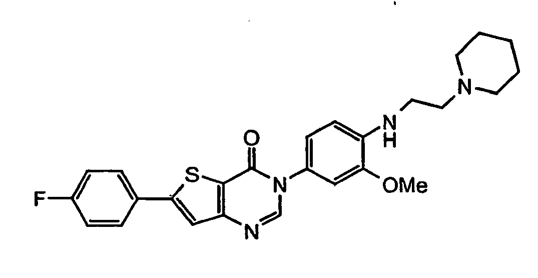

### 6-(4-Fluorophenyl)-3-(3-methoxy-4-[(2-piperidin-1-ylethyl)amino]phenyl) thieno[3,2-d]pyrimidin-4(3H)-one

The title compund was obtained by employing procedures analogous to those described in Example K20.
LCMS m/z = 479 (m + H+).
¹H NMR (DMSO-D6): δ 8.40 (s, 1 H); 7.98 (d, 2H); 7.90 (s, 1H)) ; 7.38 (d, 2H), 7.00 (s, 1 H), 6.95 (d, 1 H); 6.62 (d, 1 H); 5.20 (t, 1 H); 3.80 (s, 3H); 3.25-3.50 (m, 2H); 3.20 (m, 2H); 2.40 (t, 4H); 1.37-1.60 (m, 6H).

### Example K34

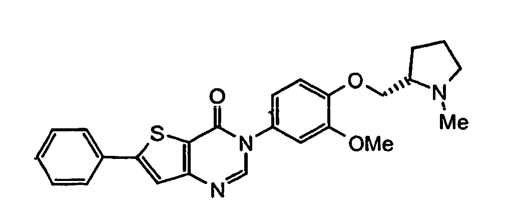

### 3-(3-Methoxy-4-{[(2R)-1-methylpyrrolidin-2-yl]methoxy}phenyl)-6-phenylthieno [3,2-d]pyrimidin-4(3H)-one

The title compound was prepared by employing procedures analogous to those described in Example K26.
LCMS m/z = 448 (m + H+).
¹H NMR (DMSO-D6): δ 8.40 (s, 1 H); 7.98 (s, 1 H); 7.92 (d, 1 H)); 7.50 (d, 1 H); 7.40-7.60 (m, 3H); 7.20 (s, 1 H); 7.10 (d, 1 H); 7.05 (d, 1 H); 4.02 (m, 1 H); 3.90 (m, 1 H); 3.80 (s, 3H); 3.00 (m, 1 H); 2.60 (m, 1 H); 2.40 (s, 3H); 2.20 (q, 1 H); 2.00 (m, 1 H); 1.65 (m, 2H); 1.60 (m, 1 H).

### Example K35

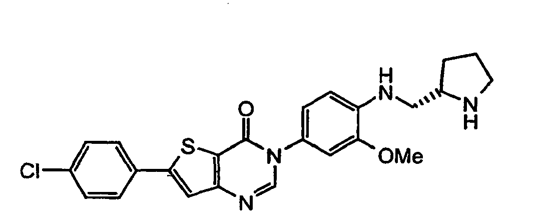

### 6-(4-Chlorophenyl)-3-(3-methoxy-4-{[(2R)-pyrrolidin-2-ylmethyl]amino}phenyl) thieno[3,2-d]pyrimidin-4(3H)-one

LCMS m/z = 467 (m + H+).
¹H NMR (DMSO-D6): δ 8.40 (s, 1H); 7.98 (s, 1H); 7.95 (d, 2H)); 7.60 (d, 2H), 7.08 (s, 1H), 6.98 (d, 1H); 6.78 (d, 1 H); 5.60 (m, 1H); 3.83 (s, 3H); 3.10-3.50 (m, 6H); 1.98 (m, 4H).

### Example K36

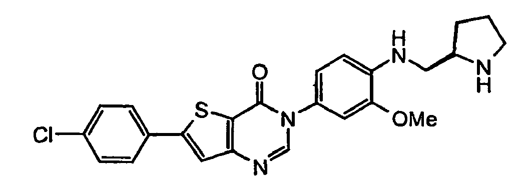

### 6-(4-Chlorophenyl)-3-(3-methoxy-4-{[(2S)-pyrrolidin-2-ylmethyl]amino}phenyl) thieno[3,2-d]pyrimidin-4(3H)-one

LCMS m/z = 467 (m + H+).
¹H NMR (DMSO-D6): δ 8.40 (s, 1 H); 7.98 (s, 1 H); 7.95 (d, 2H)); 7.60 (d, 2H), 7.08 (s, 1 H), 6.98 (d, 1H); 6.78 (d, 1 H); 5.60 (m, 1 H); 3.83 (s, 3H); 3.10-3.50 (m, 6H); 1.98 (m, 4H).

### Example K37

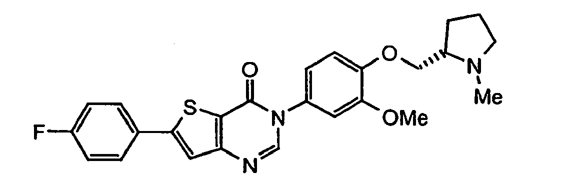

### 6-(4-Fluorophenyl)-3-(3-methoxy-4-{[(2R)-1-methylpyrrolidin-2-yl]methoxy}phenyl) thieno[3,2-d]pyrimidin-4(3H)-one

The title compound was prepared by employing procedures analogous to those described in Example K26.
LCMS m/z = 466 (m + H+).
¹H NMR (DMSO-D6) δ 8.40 (s, 1H), 8.00 (d of d, 1H), 7.95 (s, 1H), 7.40 (d of d, 2H), 7.20 (s, 1 H), 7.10 (d, 1 H), 7.02 (d, 1 H), 3.8-4.10 (m, 2H), 3.80 (s, 3H), 2.98 (t, 1 H), 2.62 (m, 1 H), 2.40 (s, 3H), 2.20 (q, 1H), 2.00 (m, 1 H), 1.50-1.80 (m, 3H) ppm.

### Example L1

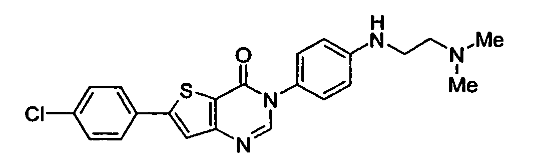

### 6-(4-chlorophenyl)-3-(4-{[2-(dimethylamino)ethyl]amino}phenyl)thieno[3,2-d]pyrimidin-4(3H)-one

The title compound was prepared by employing procedures analogous to those described in Example K20.
¹H NMR (DMSO-D₆) δ 8.35 (s, 1 H), 7.93 (m, 3H), 7.58 (d, 2H, J = 8.6 Hz), 7.20 (d, 2H, J = 8.7 Hz), 6.70 (d, 2H, J = 8.7 Hz), 5.81 (t, 1H, J = 5.4 Hz), 3.16 (q, 2H), 2.46 (q, 2H), 2.20 (s, 6H). LCMS m/z = 425 (m + H+). Calcd C, 62.18; H, 4.98; N, 13.18. Found C, 62.02; H, 4.97; N, 13.06.

### Example L2

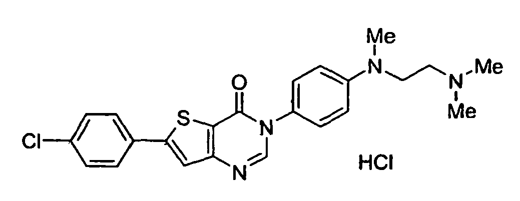

### 6-(4-chlorophenyl)-3-{4-[[2-(dimethylamino)ethyl](methyl)amino]phenyl}thieno[3,2-d]pyrimidin-4(3H)-one hydrochloride.

The title compound from Example L1 (0.12 g) was dissolved in 88% formic acid (1 mL) and 37% formaldehyde (2 mL). The reaction mixture was refluxed 2 h. The mixture was concentrated on the rotovap to give a white paste. The paste was dissolved in methanol and 1 eq of HCl in dioxane was added. The hydrochloride salt was triturated with ether and filtered to give the product as a solid (0.11 g). ¹H NMR (DMSO-D₆) δ 8.37 (s, 1H), 7.99 (m, 3H), 7.58 (d, 2H, J = 8.6 Hz), 7.45 (d, 2H, J = 8.5 Hz), 6.80 (d, 2H, J = 9.0 Hz), 4.93 (s, 2H), 3.98 (m, 2H), 3.81 (m, 2H), 3.28 (s, 6H). LCMS m/z = 437 (m + H+).

### Examples L3-L5 were prepared by employing procedures analogous to those described in Example K20.

### Example L3

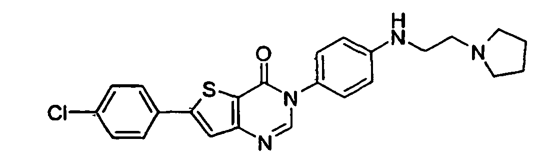

### 6-(4-chlorophenyl)-3-(4-{[2-(1-pyrrolidinyl)ethyl]amino}phenyl)thieno[3,2-d]pyrimidin-4(3H)-one

¹H NMR (DMSO-D₆) δ 8.34 (s, 1H), 7.95 (m, 3H), 7.59 (d, 2H, J = 8.6 Hz), 7.20 (d, 2H, J = 8.7 Hz), 6.68 (d, 2H, J = 8.8 Hz), 5.90 (t, 1H, J = 5.4 Hz), 3.19 (q, 2H), 2.63 (q, 2H), 2.51 (s, 4H), 1.71 (s, 4H). LCMS m/z = 451 (m + H+). Calcd C, 63.98; H, 5.14; N, 12.42. Found C, 63.87; H, 5.18; N, 12.37.

### Example L4

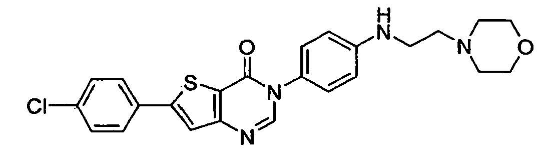

### 6-(4-chlorophenyl)-3-(4-{[2-(4-morpholinyl)ethyl]amino}phenyl)thieno[3,2-d]pyrimidin-4(3H)-one.

¹H NMR (DMSO-D₆) δ 8.34 (s, 1H), 7.95 (m, 3H), 7.59 (d, 2H, J = 8.6 Hz), 7.20 (d, 2H, J = 8.7 Hz), 6.68 (d, 2H, J = 8.8 Hz), 5.87 (t, 1H, J = 5.4 Hz), 3.60 (m, 4H), 3.19 (q, 2H), 2.52 (m, 2H), 2.50 (m, 4H). LCMS m/z = 467 (m + H+). Calcd C, 61.73; H, 4.96; N, 12.00. Found C, 61.80; H, 4.96; N, 11.93.

### Example L5

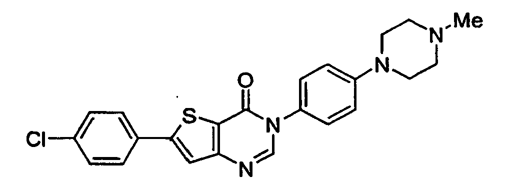

### 6-(4-chlorophenyl)-3-[4-(4-methyl-1-piperazinyl)phenyl]thieno[3,2-d]pyrimidin-4(3H)-one

¹H NMR (DMSO-D₆) δ 8.38 (s, 1H), 7.98 (m, 3H), 7.60 (d, 2H, J = 8.5 Hz), 7.35 (d, 2H, J = 8.7 Hz), 7.09 (d, 2H, J = 9.0 Hz), 3.22 (m, 4H), 2.50 (m, 4H, 2.24 (s, 3H). LCMS m/z = 437 (m + H+). Calcd (0.1 H₂O) C, 62.96; H, 4.87; N, 12.77. Found C, 62.75; H, 4.82; N, 12.55.

### Example M1

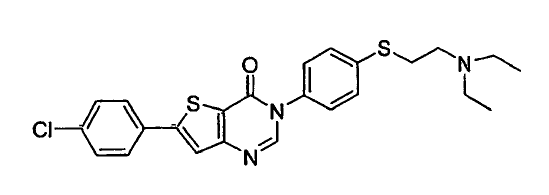

### 6-(4-chlorophenyl)-3-(4-{[2-(diethylamino)ethyl]sulfanyl}phenyl)thieno[3,2-d]pyrimidin-4(3H)-one

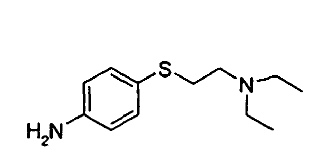

### 4-{[2-(diethylamino)ethyl]sulfanyl}aniline

To a solution of 4-aminothiophenol (23.0 mmol, 2.88 g) in DMF (23 mL) was added 2-(diethylamino)ethyl chloride hydrochloride (11.5 mmol, 1.98 g) and cesium carbonate (34.5 mmol, 11.2 g). The resulting mixture was heated to 60°C for 3 hours. The solvent was removed by rotary evaporation. The residue was partitioned between dichloromethane and water. The organic layer was washed with brine, dried over sodium sulfate and concentrated. The residue was purified by flash chromatography eluting with 5% methanol in dichloromethane with 1% triethylamine, giving the title compound (1.26 g, 49%). ¹H NMR (CDCl₃): δ 0.98 (6H, t, J = 7.0 Hz), 2.51 (4H, q, J = 7.1 Hz), 2.65 (2H, t, J = 8.1 Hz), 2.85 (2H, t, J = 8.3 Hz), 6.61 (2H, d, J = 8.6 Hz), 7.25 (2H, d, J = 8.6 Hz). LCMS m/z = 225 (m + H⁺).

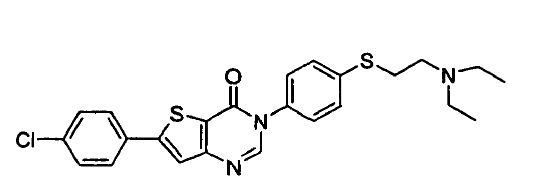

### 6-(4-chlorophenyl)-3-(4-{[2-(diethylamino)ethyl]sulfanyl}phenyl)thieno[3,2-d]pyrimidin-4(3H)-one

The mixture of 4-{[2-(diethylamino)ethyl]sulfanyl}aniline (1.0 mmol, 271 mg), amidine (methyl 5-(4-chlorophenyl)-3-{[(dimethylamino)methylidene]amino}-2-thiophenecarboxylate, 1.0 mmol, 322 mg, the preparation of which may be found in the Example J13) and phenol (350 mg) was heated to 190°C for 20 minutes. The mixture was cooled to room temperature and then washed with methanol. The crude solid product was collected by filtration and then was dissolved in minimum amount of dichlomethane. Adding methanol slowly and let sitting overnight, the title compound was precipitated out as white solid (180 mg, 39%). ¹H NMR (CDCl₃): δ 1.05 (6H, t, J = 7.2 Hz), 2.61 (4H, q, J = 7.1 Hz), 2.78 (2H, t, J = 7.6 Hz), 3.10 (2H, t, J = 7.6 Hz), 7.34 (2H, d, J = 8.6 Hz), 7.45 (4H, m, overlapping), 7.53 (1 H, s), 7.65 (2H, d, J = 8.5 Hz), 8.12 (1 H, s). LCMS m/z = 470 (m + H⁺).

### Example M2

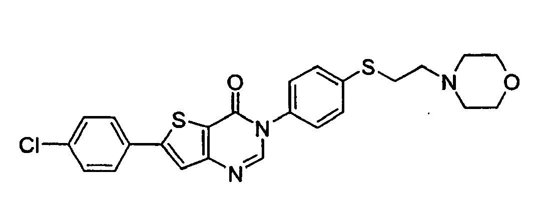

### 6-(4-chlorophenyl)-3-(4-{[2-(4-morpholinyl)ethyl]sulfanyl}phenyl)thieno[3,2-d]pyrimidin-4(3H)-one

Example M2 was prepared according to the procedures described in Example M1.
¹H NMR (CDCl₃): δ 2.50 (4H, t, J = 4.5 Hz), 2.68 (2H, m), 3.12 (2H, m), 3.72 (2H, t, J = 4.6 Hz), 7.34 (2H, d, J = 8.7 Hz), 7.44 (4H, m, overlapping), 7.52 (1 H, s), 7.64 (2H, d, J = 8.56Hz), 8.10(1 H, s). LCMS m/z = 484 (m + H⁺).

### Example N1

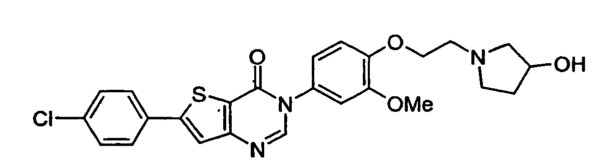

### 6-(4-chlorophenyl)-3-(4-[2-(3-hydroxypyrrolidin-1-yl)ethoxy]-3-methoxyphenyl}thieno[3,2-d]pyrimidin-4(3H)-one

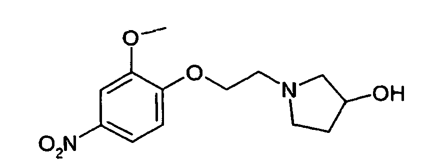

### 1-[2-(2-methoxy-4-nitrophenoxy)ethyl]pyrrolidin-3-ol

1-(2-Bromoethoxy)-2-methoxy-4-nitrobenzene (0.756 g, 2.7395 mmol) 3-pyrrolidinol (0.477 g, 5.479 mmol) and triethylamine (0.554 g, 5.479 mmol) were combined in DMF (10 mL) and heated to 80°C. The reaction was stirred for 2 h. Cooled to RT and diluted with EtOAc (100 mL) and washed with water (2 x 100 mL). The organics were dried over MgSO₄, filtered and concentrated to afford 0.3967 g (1.407 mmol, 51%) of the desired product as a dark brown oil. ¹H NMR (CDCl₃) δ 7.90 (d, 1H, J = 9.0 Hz), 7.75 (s, 1H), 6.93 (d, 1H, J = 9.0 Hz), 4.38 (m, 1 H), 4.24 (t, 2H, J = 6.2 Hz), 3.95 (s, 3H), 3.2 - 3.0 (m, 4H), 3.0 - 2.90 (m, 1 H), 2.8 -2.70 (m, 1 H), 2.60 - 2.50 (m, 1 H), 2.30 - 2.20 (m, 1 H), 1.85 -1.75 (m, 1 H).

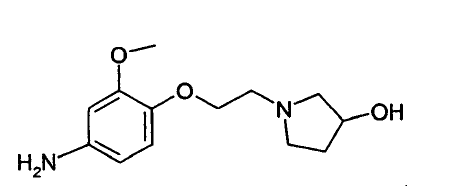

### 1-[2-(4-amino-2-methoxyphenoxy)ethyl]pyrrolidin-3-ol

1-[2-(2-methoxy-4-nitrophenoxy)ethyl] pyrrolidin-3-ol (0.169 g, 0.583 mmol) was taken up in EtOAc (5 mL) and 10% Pd/C (0.016 g) was added. The hydrogen gas was bubbled through the reaction and the reaction then placed under a hydrogen blanket. Stirred over night. Filtered through celite and concentrated to give 0.118 g (0.470 mmol, 81 %) of the desired product. ¹H NMR (CDCl₃) δ 6.74 (d, 1H, J = 8.5 Hz), 6.28 (s, 1H), 6.19 (d, 1H, J = 8.5 hz), 4.34 (m, 1H), 4.05 (t, 2H, J = 6.2 Hz), 3.79 (s, 3H), 3.03 - 2.83 (m, 5H), 2.63 - 2.59 (m, 1 H), 2.42 - 2.38 (m, 1 H), 2.23 - 2.14 (m, 1 H), 1.75 (m, 1 H).

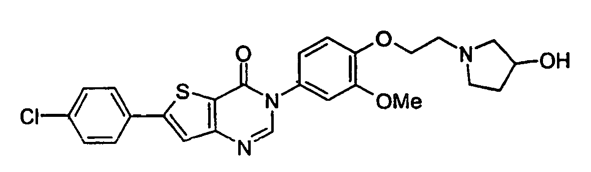

**6-(4-chlorophenyl)-3-{4-[2-(3-hydroxypyrrolidin-1-yl)ethoxy]-3-methoxyphenyl}thieno[3,2-*d*]pyrimidin-4(3*H*)-one:** Methyl 3-amino-5-(4-chlorophenyl)thiophene-2-carboxylate (0.126 g, 0.470 mmol) was dissolved in a mixture of DMF (1 mL) and N,N-dimethylformamide dimethyl acetal (1 mL) and stirred at 110°C for 2 h. The mixture was then concentrated to dryness. 1-[2-(4-amino-2-methoxyphenoxy)ethyl]pyrrolidin-3-ol (0.118 g, 0.470 mmol, descibed in the preceding step) in anhydrous ethanol (2 mL) was added and the reaction heated to reflux in an oil bath. The mixture was stirred for 18 h and was then cooled to RT and the precipitate collected and washed with cold ethanol (2 x 5 mL) to give 0.039 g (0.078 mmol, 17%) of the title compound as an off-white solid.
¹H NMR (CDCl₃) δ 8.14 (s, 2H), 7.66 (d, 2H, J = 8.4Hz), 7.53 (s, 1H), 7.45 (d, 2H, J = 8.4 Hz), 7.02 (d, 1H, 8.3 Hz), 6.93 (m, 2H), 4.39 (m, 1H), 4.24 (t, 2H, J = 6.0 Hz), 3.95 (s, 3H), 3.2 - 3.0 (m, 4H), 3.0 - 2.90 (m, 1 H), 2.8 -2.70 (m, 1 H), 2.60 - 2.50 (m, 1 H), 2.30 - 2.20 (m, 1 H), 1.85 -1.75 (m, 1 H). LCMS M + H 498

### Example N2

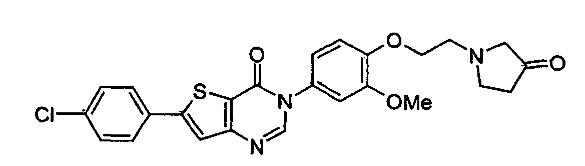

### 6-(4-chlorophenyl)-3-{3-methoxy-4-[2-(3-oxopyrrolidin-1-yl)ethoxy]phenyl}thieno[3,2-d]pyrimidin-4(3H)-one

Oxalyl chloride (0.042 g, 0.33 mmol) was charged to a flask with methylene chloride (2 mL). The reaction was cooled to -78°C. DMSO (0.052 g, 0.67 mmol) was added and the reaction was stirred for 10 min. 6-(4-chlorophenyl)-3-{4-[2-(3-hydroxypyrrolidin-1-yl)ethoxy]-3-methoxyphenyl}thieno[3,2-*d*]pyrimidin-4(3*H*)-one (0.064 g, 0.13 mmol) in methylene chloride (2 mL) was added to the reaction and stirred for 1 h at-78°C. Triethylamine (0.13 g, 1.29 mmol) was added and the reaction warmed to room temperature. The mix was diluted with methylene chloride (10 mL) and washed with water (2 x 50 mL). The organics were dried over MgSO₄, filtered and concentrated. The resultant residue was purified on a chromatatron (90:10 CH₂Cl₂:MeOH) to give 0.019 g (0.038 mmol, 30%) of the desired product as a light yellow solid.
¹H NMR (CDCl₃) δ 8.14 (s, 2H), 7.65 (d, 2H, J = 8.5Hz), 7.53 (s, 1H), 7.44 (d, 2H, J = 8.4 Hz), 7.02 (d, 1H, 8.4 Hz), 6.93 (m, 2H), 4.22 (t, 2H, J = 6.5 Hz), 3.88 (s, 3H), 3.15 (s, 2H), 3.09 - 3.05 (m, 4H), 2.43 (t, 2H, J = 7.0 Hz).LCMS M + H 497.

### Example O1

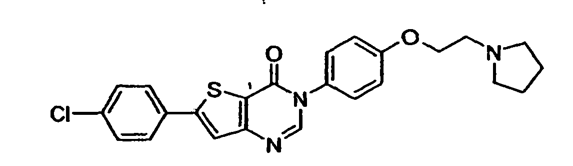

### 6-(4-chlorophenyl)-3-[4-(2-pyrrolidin-1-ylethoxy)phenyl]thieno[3,2-d]pyrimidin-4(3H)-one

A solution of 1-(2-hydroxyethyl)pyrrolidine in THF was added dropwise to a slurry of NaH in THF under N₂ at 25°C over a 5 min period. The mixture was stirred at 25°C for 30 min, during which time it turned to a dark amber solution. A solution of 4-nitrofluorobenzene in THF was added dropwise and the resultant mixture was stirred at 25°C for 48 h. The dark brown solution was carefully quenched by the addition of saturated aqueous NaHCO₃ and was diluted with EtOAc. The aqueous layer was extracted with EtOAc (3x) and the combined organic extracts were washed with saturated aqueous NaHCO₃, brine (2x) and were dried over MgSO₄ and concentrated *in vacuo* to give a pale amber oil that was used without further purification. A solution of 1-[2-(4-nitrophenoxy)ethyl]pyrrolidine in EtOH under N₂ was treated with 10% Pd/C and placed under an atmosphere of H₂ via Parr apparatus. The mixture was agitated for 3 h at̃ ~310275 Nm⁻² (45 psi) of hydrogen and was then purged with N₂, and filtered through a plug of Celite (EtOAc/EtOH wash) under N₂ to give a dark brown oil. Purification of the oil by flash chromatography (12 g pre-packed silica gel ISCO column, 0 - 15% CH₃OH/CH₂Cl₂) afforded 4-(2-pyrrolidin-1-ylethoxy)aniline as a brown oil. A solution of Methyl 5-(4-chlorophenyl)-3-{[(*E*)-(dimethylamino)methylidene]amino}-2-thiophenecarboxylate (Example J13) and aniline in EtOH under N₂ was stirred and heated at reflux for 48 h. The mixture was filtered and the white solid was washed with EtOH and dried *in vacuo.* Purification by reverse phase chromatography (Gilson, 10 - 95% acetonitrile/water) and concentration via lyophilization afforded the desired product as a TFA salt. ¹H NMR (DMSO_{d6}) δ 9.80 (br s, 1H), 8.37 (s, 1 H), 7.96 (s, 1H), 7.91 (d, J = 8.8 Hz, 2 H), 7.56 (d, J = 8.8 Hz, 2H), 7.50 (d, J = 9 Hz, 2H), 7.16 (d, J = 9 Hz, 2H), 4.36 (m, 2H), 3.61 (m, 4H), 3.14 (m, 2H), 2.03 (m, 2H), 1.88 (m, 2H). LCMS m/z = 452 (M⁺ + H).

### Examples O2-O6 were prepared through procedures analogous to those described in Example O1.

### Example O2

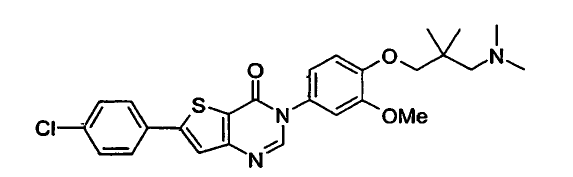

### 6-(4-Chlorophenyl)-3-{4-[3-(dimethylamino)-2,2-dimethylpropoxy]-3-methoxyphenyl}thieno[3,2-d]pyrimidin-4(3H)-one

¹H NMR (300 MHz, DMSO-d₆) δ 8.39 (s, 1 H), 7.97 (s, 1H), 7.92 (d, J = 8.7 Hz, 2H), 7.57 (d, J = 8.7 Hz, 2H), 7.17 (d, J = 2.4 Hz, 1H), 7.09 (d, J = 8.6 Hz, 1H), 7.03 (dd, J = 8.6, 2.4 Hz, 1H), 3.77 (s, 3H), 3.73 (s, 2H), 2.25 (m, 2H), 2.20 (m, 4H), 0.95 (s, 6H). LCMS m/z 499 (M + H⁺).

### Example O3

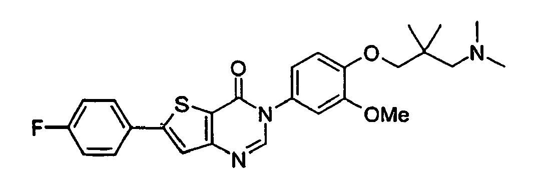

### 6-(4-Fluorophenyl)-3-{4-[3-(dimethylamino)-2,2-dimethylpropoxy]-3-methoxyphenyl}thieno[3,2-d]pyrimidin-4(3H)-one

Characterized as the HCl salt: ¹H NMR (300 MHz, DMSO-d₆) δ 9.23 (br s, 1H), 8.38 (s, 1 H), 7.95 (dd, J = 8.8, 5.1 Hz, 2H), 7.92 (s, 1 H), 7.36 (apparent t, J = 8.8 Hz, 2H), 7.23(d, J = 2.3 Hz, 1 H), 7.15 (d, J = 8.6 Hz, 1 H), 7.07 (dd, J = 8.6, 2.3 Hz, 1 H), 3.94 (s, 3H), 3.80 (br s, 1 H), 3.22 (s, 2H), 2.87 (s, 2H), 1.15 (s, 6H). LCMS m/z 482 (M + H⁺).

### Example O4

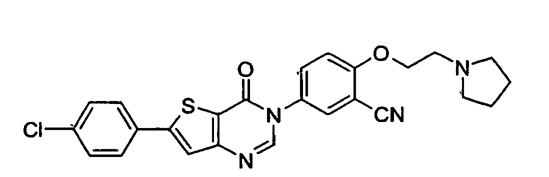

### 5-[6-(4-Chlorophenyl)-4-oxothieno[3,2-d]pyrimidin-3(4H)-yl]-2-(2-pyrrolidin-1-ylethoxy)benzonitrile

Characterized as the HCl salt: ¹H NMR (300 MHz, DMSO-d₆) δ 10.65 (br s, 1H), 8.44 (s, 1 H), 8.09 (d, J = 2.6 Hz, 1H), 8.00 (s, 1 H), 7.93 (obscured d, J = 9.2, 1H), 7.57 (d, J = 8.7 Hz, 2H), 7.50 (d, J = 9.2 Hz, 1H), 4.60 (t, J = 4.6 Hz, 2H), 3.71-3.65 (m, 4H), 3.17 (m, 2H), 2.04 (m, 2H), 1.89 (m, 2H). LCMS m/z 477 (M + H⁺).

### Example O5

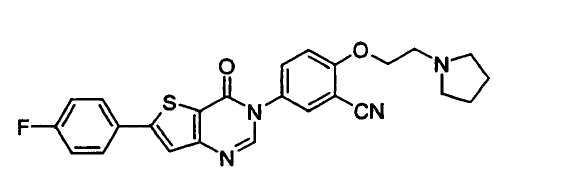

### 5-[6-(4-Fluorophenyl)-4-oxothieno[3,2-d]pyrimidin-3(4H)-yl]-2-(2-pyrrolidin-1-ylethoxy)benzonitrile

Characterized as the HCl salt: ¹H NMR (300 MHz, DMSO-d₆) δ 10.59 (br s, 1H), 8.43 (s, 1H), 8.10 (d, J = 2.6 Hz, 1 H), 7.96-7.92 (m, 4H), 7.51 (d, J = 9.2 Hz, 1H), 7.36 (apparent t, J = 8.8 Hz, 2H), 4.60 (t, J = 4.6 Hz, 2H), 3.73-3.60 (m, 4H), 3.17 (m, 2H), 2.04 (m, 2H), 1.88 (m, 2H). LCMS m/z 461 (M + H⁺).

### Example O6

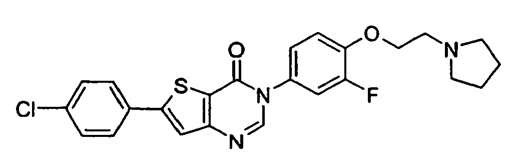

### 6-(4-Chlorophenyl)-3-[3-fluoro-4-(2-pyrrolidin-1-ylethoxy)phenyl]thieno[3,2-d]pyrimidin-4(3H)-one

¹H NMR (300 MHz, DMSO-d₆) δ 8.40 (s, 1 H), 7.97 (s, 1 H), 7.92 (d, J = 8.5 Hz, 2H), 7.57 (d, J = 8.5 Hz, 2H), 7.55 (m, 1 H), 7.36-7.32 (m, 2H), 4.22 (t, J = 5.7 Hz, 2H), 2.84 (m, 2H), 2.51 (br s, 4H), 1.86 (m, 4H). LCMS m/z 471 (M + H⁺). The activity of the compounds used in this invention may be assessed in a functional assay of MCHR1 as follows:

### Materials

Black, 96-well, tissue culture-treated plates (#3904) were obtained from Coming Costar, (Cambridge, MA), LucPlus^{™} Luciferase Reporter Gene Assay Kit (# 6016969) was from Packard (Meriden, CT), plate seals (#097-05-00006) were from Beckman/Sagian (Fullerton, CA). DMEM/F12 medium (#11039-021), fetal bovine serum (# 16140-071), L-glutamine (#25030-081), 0.05% trypsin (# 25300-054), G418 (#10131-035) and dPBS (#4190-144) were obtained from Gibco BRL (Gaithersburg, MD). Thrombin (T7009) was obtained from Sigma Chemical Co (St. Louis, MO), MCH peptide (H-1482) was obtained from BaChem California (Torrance, CA). Chinese hamster ovary (CHO-K1) cells were obtained from the American Type Culture Collection (Rockville, MD).

### Methods

CHO cells, stably expressing an elkgal4-luc⁺ reporter gene (host) were transfected by electroporation with the human melanin-concentrating hormone one receptor. A stable clone was selected using G418 for functional antagonist assays. MCH1 R-elkgal4-luc⁺ CHO cells were propagated in complete medium (DMEM/F12, 5% FBS, 2 mM l-glutamine) in T225 flasks. Forty-eight hours prior to assay, cells were harvested with 2 mL of 0.05% trypsin, washed with complete medium and plated at a concentration of 10,000 cells/well in complete medium in black 96-well plates. Eighteen hours prior to the assay, the medium was removed from the cells by aspiration and replaced with 90 µl/well of serum-free DMEM/F12. At the time of the assay, antagonists (1 µL, 100% DMSO) as 10-point concentration curves were pipetted into the medium and plates were incubated for forty-five minutes at 37°C in a cell culture incubator. Following this incubation, 10 uL of an EC₈₀ concentration of MCH was added to the medium and plates were incubated for five hours at 37°C in a cell culture incubator. The medium was aspirated by vacuum followed by the addition of 50 µl of a 1:1 mixture of LucPlus^{™} and dPBS/1 mM CaCl₂/1 mM MgCl₂. The aspiration step was performed in order to avoid potential assay interference by compounds which could inhibit or stimulate luciferase activity or could inhibit light signal. Plates were sealed and subjected to dark adaptation at room temperature for 10 minutes before luciferase activity was quantitated on a TopCount^{™} microplate scintillation counter (Packard) using 3 seconds/well count time. The ability of the antagonist to inhibit the MCH EC₈₀ response was quantified by non-linear regression analysis using a curve-fitting program based in Microsoft ExCel. Specificity of the MCHR1 response was determined using the same protocol by measuring the ability of said antagonists to inhibit an EC₈₀ thrombin response (endogenous) in the host cells.

The compounds described in Examples have a plC₅₀ value of greater than 7. For example, the compounds of Examples H1, J1, and l3 have the respective MCHR1 plC₅₀ values shown below.

| Example | MCHR1 plC₅₀ |
|---|---|
| H1 | 7.1 |
| J1 | 7.2 |
| 13 | 9.1 |

## Claims

1. A compound of formula (Ia) comprising: 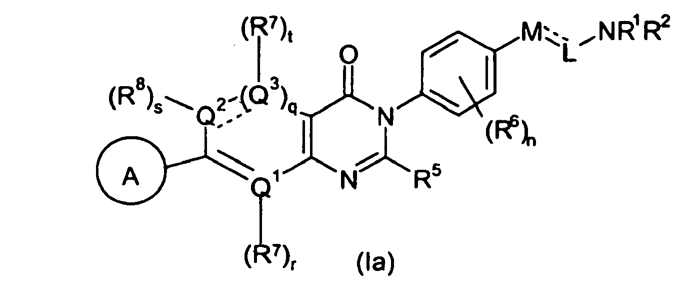 a pharmaceutically acceptable salt or solvate thereof, wherein:
Ⓐ is aryl or heteroaryl, optionally substituted by one to four C₁₋₆ straight or branched alkyl, alkenyl, halo, amino, alkylamino, dialkylamino, hydroxy, C₁₋₆ alkoxy, cyano, or alkylthio groups;
when q is 1, the dashed line is a double bond;
Q¹, Q², and Q³ are C; and q, r, s, and t are 1;
or
Q¹ is N; Q² is S and q, r, and s are 0;
or
Q¹ is C; Q² is S; q and s are 0; and r is 1.
each corresponding R⁷ is independently selected from the group consisting of hydrogen, C₁₋₆ straight or branched alkyl, C₃₋₆ cycloalkyl, C₁₋₆ alkoxy, amino, C₁₋₆ alkylamino, C₁₋₆ dialkylamino, hydroxy, cyano, C₁₋₆ alkylthio, and halo;
R⁵ is selected from the group consisting of hydrogen, C₁₋₆ straight or branched alkyl, C₃-₆ cycloalkyl and C₁₋₃ alkylthio;
R⁶ is selected from the group consisting of hydrogen and methoxy and n is 1;
M is selected from the group consisting of O, S, S(O)₂, S(O)₂NR, N-R, C(O), C(R)₂, N-C(O)R, and N-S(O)₂R ;
wherein R is selected from the group consisting of hydrogen, phenyl, heteroaryl, C₁-₆ straight or branched alkyl, and C₃₋₆ cycloalkyl;
L is C₂₋₃ alkyl, C₂₋₃ alkenyl, or -C(O)(CH₂)-;
(i) R¹ and R² each independently are selected from the group consisting of hydrogen, C₁₋₆ straight or branched alkyl, C₃₋₆ cycloalkyl, and a 5- or 6- membered heterocycle wherein said alkyl, said cycloalkyl and said heterocycle are optionally substituted by phenyl, one to four C₁₋₃ alkyl, hydroxy, oxo, alkoxy or halo;
or (ii) R¹ and R² may be selected from the group consisting of aryl and a 5- or 6-membered heteroaryl containing 1, 2, or 3 heteroatoms selected from N, O, and S, wherein said aryl and said heteroaryl are optionally substituted 1, 2, or 3 times with a substituent selected from halo, C₁₋₆ straight or branched alkyl, C₃₋₆ cycloalkyl, C₁₋₆ alkenyl, C₃₋₆ cycloalkenyl, hydroxy, C₁₋₆ alkoxy, oxo, amino, C₁₋₆ alkylamino, C₁₋₆ dialkylamino, C₁₋₆ alkylthio, C₁₋₆ alkylsulfinyl, and phenyl;
or (iii) R¹ and R² together with the nitrogen atom to which they are bonded form a 4-8 membered heterocyclic ring or a 7-11 membered bicyclic heterocyclic ring, each of said 4-8 membered heterocyclic ring and said 7-11 membered bicyclic heterocyclic ring contain 1, 2 or 3 heteroatoms selected from the group consisting of N, O, and S, and wherein either said heterocyclic ring or said bicyclic heterocyclic ring may be optionally substituted by phenyl, one to four C₁₋₃ alkyl, hydroxy, C₁₋₃ alkoxy, oxo, or halo;
or (iv) R¹ and R² may be independently linked either to the group L or linked to the group M when M is selected from the group consisting of S(O)₂NR, N-R, C(R)₂, N-C(O)R, and N-S(O)₂R, and wherein R is C₁₋₆ straight or branched alkyl, to form a 3-7 membered cyclic group which may be optionally substituted by phenyl, one to four C₁₋₃ alkyl, hydroxy, alkoxy, oxo, or halo.

2. The compound according to Claim 1 wherein said
Ⓐ is an aryl substituted with a group selected from the group consisting of halo, C₁₋₃ alkyl, and C₁₋₃ alkoxy.

3. The compound according to Claim 2 wherein said aryl is substituted with a group selected from the group consisting of fluoro, chloro, and methoxy.

4. The compound according to Claim 3 wherein said aryl is substituted with a halo group; q is 0; Q¹ is C; and R⁷ is hydrogen or halo.

5. The compound according to Claim 4 wherein said aryl is 4-chlorophenyl; R⁵ and R⁷ are each hydrogen.

6. The compound according to Claims 1-5 wherein L is C₂₋₃ alkyl or C₂₋₃ alkenyl.

7. The compound according to Claim 6 wherein L is C₂₋₃ alkyl.

8. The compound according to Claims 1-7, wherein M is selected from the group consisting of O, S, S(O)₂NR, N-R, N-C(O)R, and N-S(O)₂R- wherein R is selected from the group consisting of hydrogen, phenyl, heteroaryl, . C₁₋₆ straight or branched alkyl and C₃₋₆ cycloalkyl.

9. The compound according to Claim 8 wherein R is selected from the group consisting of hydrogen, phenyl, C₁₋₆ straight or branched alkyl, and C₃₋₆ cycloalkyl.

10. The compound according to Claim 9 wherein R is selected from the group consisting of hydrogen, C₁₋₆ straight or branched alkyl, and C₃₋₆ cycloalkyl.

11. The compound according to Claims 1-10 wherein R⁵ is hydrogen or C₁₋₃ alkyl.

12. The compound according to Claim 11 wherein R⁵ is hydrogen or methyl.

13. The compound according to Claims 1-12 wherein in (i) above R¹ and R² are selected from the group consisting of hydrogen, C₁₋₆ straight or branched alkyl, and C₃₋₆ cycloalkyl.

14. The compound according to Claim 13 wherein R¹ and R² in (i) are selected from the group consisting of hydrogen, C₁₋₃ alkyl, and C₃₋₆ cycloalkyl.

15. The compound according to Claims 1-12 wherein in (iii) above R¹ and R² together with the nitrogen atom to which they are bonded form a 5- or 6- membered heterocyclic ring or an 8- to 11-membered bicyclic heterocyclic ring having 1 or 2 heteroatoms selected from group N, O, and S, wherein said heterocyclic ring and said bicyclic heterocyclic ring may be optionally substituted up to two times with a substituent selected from the group consisting of oxo and halo.

16. The compound according to Claims 1-12 wherein in (iv) R¹ and R² may be independently linked to the group M when M is selected from the group consisting of S(O)₂NR, N-R, C(R)₂, N-C(O)R, and N-S(O)₂R, and wherein R is C₁₋₆ straight or branched alkyl, to form a 5-7 membered cyclic group which may be optionally substituted by phenyl, one to four C₁₋₃ alkyl, hydroxy, alkoxy, oxo, or halo.

17. The compound according to Claim 1 wherein L is C₂-C₃ alkyl or C₂-C₃ alkenyl;
in (i) R¹ and R² are selected from the group consisting of hydrogen, C₁-C₃ straight or branched alkyl, C₃-C₆ cycloalkyl substituted with a substituent selected from the group consisting of halo, alkyl, hydroxy, oxo, and alkoxy; or
in (iii) R¹ and R² together with the nitrogen atom to which they are bonded form a 4-6 membered heterocyclic ring wherein said heterocyclic ring is optionally substituted with a substituent selected from the group consisting of one to four C₁-C₃ alkyl, hydroxy, alkoxy, oxo, and halo.

18. The compound according to Claim 17 wherein L is a C₂-C₃ alkyl;
in (i) R¹ and R² are each independently selected from the group consisting of hydrogen and C₃-C₆ cycloalkyl substituted with a substituent selected from the group consisting of oxo and halo;
or in (iii) R¹ and R² together with the nitrogen atom to which they are bonded form a 5 or 6 membered heterocyclic wherein said heterocyclic ring is optionally substituted with a substituent selected from the group consisting of one to two oxo and halo.

19. The compound according to Claim 18 wherein L is CH₂CH₂ and in (iii) R¹ and R² together with the nitrogen atom to which they are bonded form a pyrrolidine ring substituted at the 3-position with a fluorine atom.

20. The compound according to Claim 1 wherein M is O, N-R or N-C(O)R where R is hydrogen or C₁-C₆ straight or branched alkyl and R⁶ is selected from the group consisting of hydrogen, C₁-C₆ straight or branched alkyl, C₁-C₃ alkoxy, trihaloalkyl, trihaloalkoxy, cyano, and halo.

21. The compound according to Claim 20 wherein M is O or N-R where R is hydrogen and R⁶ is selected from the group consisting of hydrogen, C₁-C₂ straight or branched alkyl, C₁-C₂ alkoxy, or halo.

22. The compound according to Claim 21 wherein M is O and R⁶ is methoxy.

23. The compound according to Claim 1 wherein the compound is selected from the group consisting of 6-(4-chlorophenyl)-3-{3-methoxy-4-[2-(3-oxopyrrolidin-1-yl)ethoxy]phenyl}thieno[3,2-*d*]pyrimidin-4(3*H*)-one and 6-(4-chlorophenyl)-3-{4-[2-(3-fluoropyrrolidin-1-yl)ethoxy]-3-methoxyphenyl}thieno[3,2-*d*]pyrimidin-4(3*H*)-one.

24. A process for preparing a compound according to claim 1 comprising reacting an aniline of formula (II) 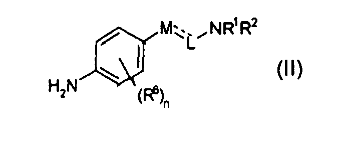 with a compound of formula (III) 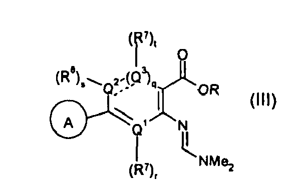 while heating in a solvent; wherein Ⓐ, R⁸, R⁷, R⁶, R⁵, R², R¹, M, L, Q¹, Q², Q³, q, r, s, t, and n are as defined in formula (la); and Me is methyl.

25. A process for preparing a compound according to claims 1-23 comprising coupling an amino acid of formula (IV) 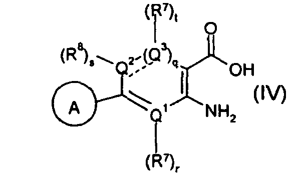 with an aniline of formula (II) 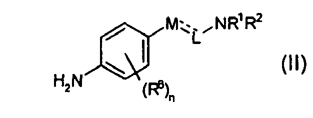 in a solvent in the presence of at least one coupling agent to produce a compound of formula (V) 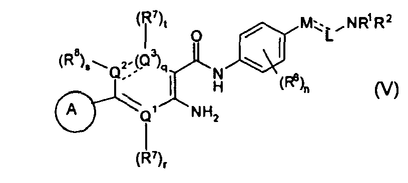 and cyclizing said compound of formula (V) to form a compound of formula (1a) and wherein Ⓐ, R⁸, R⁷, R⁶, R⁵, R², R¹, M, L, Q¹, Q², Q³, q, r, s, t, and n are as defined in formula (1a).

26. A process for preparing a compound according to claims 1-23 comprising reaction of a compound of formula (Va) 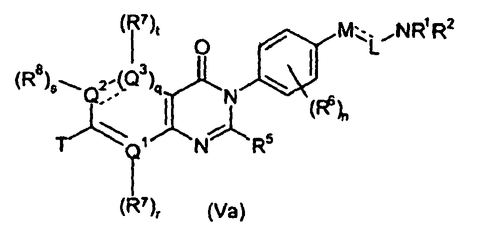 with a compound capable of introducing the group Ⓐ, and wherein Ⓐ, R⁸, R⁷, R⁶, R⁵, R², R¹, M, L, Q¹, Q², Q³, q, r, s, t, and n are as defined in formula (la) and T is a leaving group.

27. A process for preparing a compound according to claims 1-23 comprising reaction of a compound of formula (Va) 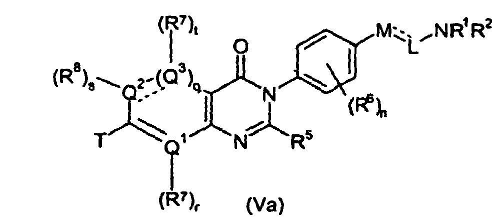 with a boronic acid and a palladium catalyst using a Suzuki coupling reaction or with an organostannane reagent and a palladium catalyst using a Stille coupling reaction and wherein Ⓐ, R⁸, R⁷, R⁶, R⁵, R², R¹, M, L, Q¹, Q², Q³, q, r, s, t, and n are as defined in formula (1a) and T is a leaving group.

28. A process for preparing a compound according to claim 1 wherein R⁵ is hydrogen comprising reacting a sulfur-containing compound of formula (VI) 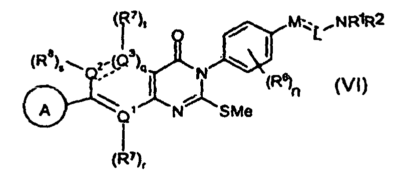 with a Raney nickel reductant in the presence of a solvent and wherein Ⓐ, R⁸, R⁷, R⁶, R⁵, R², R¹, M, L, Q¹, Q², Q³, q, r, s, t, and n are as defined in formula (la).

29. A process for preparing a compound according to claims 1-23 comprising the alkylation of an amine of formula (XV)
H-NR¹R² (XV)
with an alkylating agent of formula (XIV) 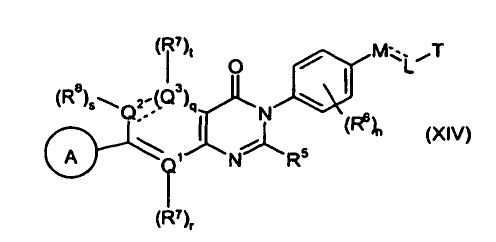 wherein M is O, T is a leaving group, and wherein Ⓐ, R⁸, R⁷, R⁸, R⁵, R², R¹, M, L, Q¹, Q², Q³, q, r, s, t, and n are as defined in formula (Ia).

30. A process for preparing a compound according to claim 1 wherein M is N(CO)R comprising acylation of aniline of formula (XVI) 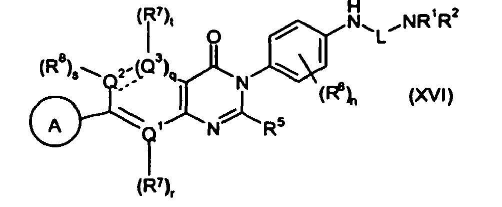 with an acylating agent of formula (XVII) 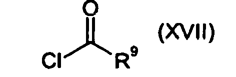 and wherein Ⓐ, R⁸, R⁷, R⁶, R⁵, R², R¹, M, L, Q¹, Q², Q³, q, r, s, t, and n are as defined in formula (1a) and R⁹ is selected from the group consisting of hydrogen, phenyl, heteroaryl, C₁₋₆ straight or branched alkyl, and C₃₋₆ cycloalkyl.

31. A process for preparing a compound according to claim 1 wherein M is N comprising reductive alkylation of an aniline of formula (XIX) 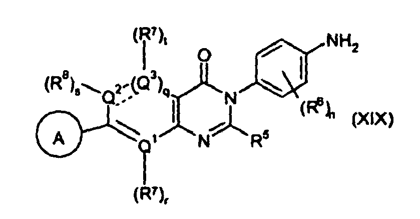 by an aldehyde of formula (XVIII) 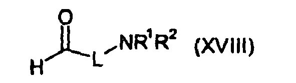 in the presence of a borohydride reducing agent or hydrogen and a catalyst and in which the L of formula (XVIII) is CH₂ or CH₂CH₂, and wherein Ⓐ, R⁸, R⁷, R⁶, R⁵, R², R¹, M, Q¹, Q², Q³, q, r, s, t, and n are as defined in formula (1a).

32. A process for preparing a compound according to claim 1 wherein M is O comprising alkylation of a phenol of formula (XX) 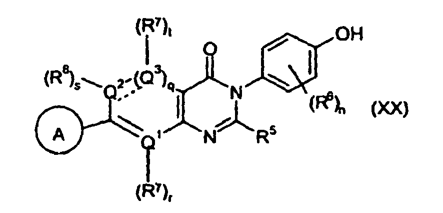 with an alkylating agent of formula (XXI)
T-L-NR¹R² (XXI)
in which T is a leaving group and wherein Ⓐ, R⁸, R⁷, R⁶, R⁵, R², R¹, M, L, Q¹, Q², Q³_{.} q, r, s, t, and n are as defined in formula (1a).

33. A process for preparing a compound according to claim 1 wherein M is O comprising reductive amination of an aldehyde of formula (XXII) 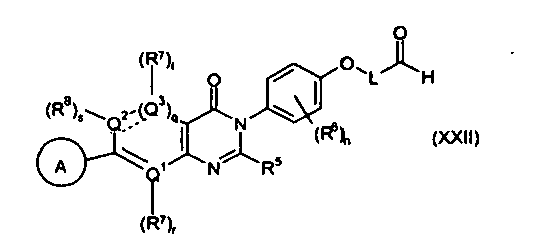 by an amine of formula (XV)
H-NR¹R² (XV)
in the presence of a reducing agent and a catalyst and wherein L is CH₂ or CH₂CH₂ and Ⓐ, R⁸, R⁷, R⁶, R⁵, R², R¹, M, Q¹, Q², Q³, q, r, s, t, and n are as defined in formula (Ia).

34. A process for preparing a compound according to claim 1 wherein M is O comprising reductive alkylation of an amine of formula (XXV) 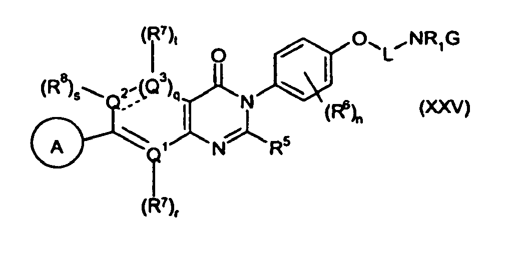 with an aldehyde and wherein for formula (XXV) G is H and Ⓐ, R⁸, R⁷, R⁶, R⁵, R², R¹, M, L, Q¹, Q², Q³, q, r, s, t, and n are as defined in formula (Ia).

35. A process for preparing a compound according to claim 1 in which L is -C(O)CH₂- comprising reaction of an amine of formula (XV)
H-NR¹ R² (XV)
with an alkylating agent of formula (XXVIII) 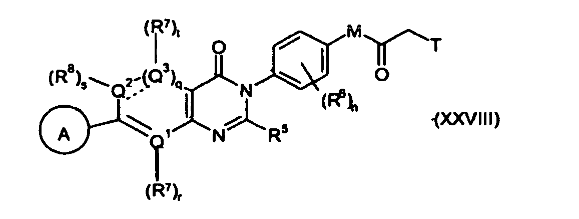 in which T is a leaving group, and wherein Ⓐ, R⁸, R⁷, R⁶, R⁵, R², R¹, M, L, Q¹, Q², Q³, q, r, s, t, and n are as defined in formula (Ia).

36. Use of a compound according to Claims 1-23 or a salt or solvate thereof for the manufacture of a medicament for the treatment of obesity, diabetes, depression, or anxiety in a mammal.

37. Use according to Claim 36 wherein said mammal is a human.

38. The compound according to Claim 1-23, a salt or solvate thereof in combination with at least one species selected from the group; consisting of an agent for treating diabetes, an agent for treating hypertension, and an agent for treating arteriosclerosis.

39. A pharmaceutical composition comprising a compound of Claim 1-23 or a salt or solvate thereof.

40. A pharmaceutical composition according to Claim 39 further comprising at least one pharmaceutically acceptable component selected from the group consisting of a carrier, a diluent, an excipient, and a mixture thereof.

41. A compound according to Claims 1-23 or a salt or solvate thereof for use in treating diabetes and for obesity.

## Patentansprüche

1. Verbindung der Formel (Ia), umfassend: 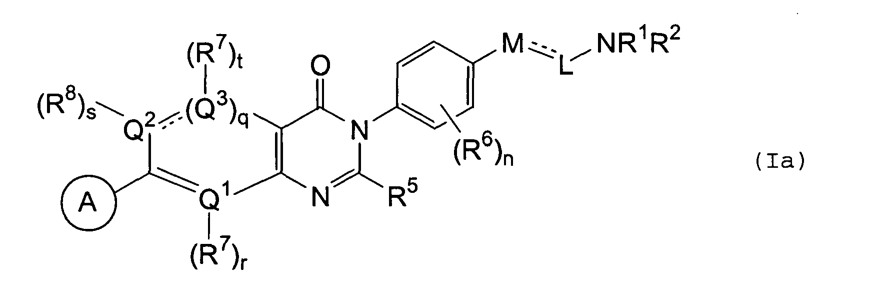 ein pharmazeutisch akzeptables Salz oder Solvat davon, worin:
Ⓐ Aryl oder Heteroaryl ist, gegebenenfalls substituiert mit 1 bis 4 linearen oder verzweigten C₁₋₆-Alkyl-, Alkenyl-, Halogen-, Amino-, Alkylamino-, Dialkylamino-, Hydroxy-, C₁-₆-Alkoxy-, Cyano- oder Alkylthio-Gruppen;
wenn q 1 ist, die gestrichelte Linie eine Doppelbindung ist;
Q¹, Q² und Q³ C sind; und q, r, s und t 1 sind;
oder
Q¹ N ist; Q² S ist und q, r und s 0 sind;
oder
Q¹ C ist; Q² S ist; q und s 0 sind; und r 1 ist;
jedes entsprechende R⁷ unabhängig aus der Gruppe ausgewählt ist, die aus Wasserstoff, linearem oder verzweigtem C₁-₆-Alkyl, C₃-₆-Cycloalkyl, C₁-₆-Alkoxy, Amino, C₁-₆-Alkylamino, C₁-₆-Dialkylamino, Hydroxy, Cyano, C₁-₆-Alkylthio und Halogen besteht;
R⁵ aus der Gruppe ausgewählt ist, die aus Wasserstoff, linearem oder verzweigtem C₁-₆-Alkyl, C₃-₆-Cycloalkyl und C₁-₃-Alkylthio besteht;
R⁶ aus der Gruppe ausgewählt ist, die aus Wasserstoff und Methoxy besteht, und n 1 ist;
M aus der Gruppe ausgewählt ist, die aus O, S, S(O)₂, S(O)₂NR, N-R, C(O), C(R)₂, N-C(O)R und N-S(O)₂R besteht;
worin R aus der Gruppe ausgewählt ist, die aus Wasserstoff, Phenyl, Heteroaryl, linearem oder verzweigtem C₁-₆-Alkyl und C₃-₆-Cycloalkyl besteht;
L C₂-₃-Alkyl, C₂-₃-Alkenyl oder -C(O)(CH₂)- ist;
(i) R¹ und R² jeweils unabhängig aus der Gruppe ausgewählt sind, die aus Wasserstoff, linearem oder verzweigtem C₁-₆-Alkyl, C₃-₆-Cycloalkyl und einem 5- oder 6-gliedrigen Heterocyclus besteht, worin das Alkyl, das Cycloalkyl und der Heterocyclus gegebenenfalls mit Phenyl, 1 bis 4 C₁-₃-Alkyl, Hydroxy, Oxo, Alkoxy oder Halogen substituiert sind;
oder (ii) R¹ und R² aus der Gruppe ausgewählt sein können, die aus Aryl und einem 5- oder 6-gliedrigen Heteroaryl, das 1, 2 oder 3 Heteroatome enthält, die aus N, O und S ausgewählt sind, besteht, worin das Aryl und das Heteroaryl gegebenenfalls 1-, 2- oder 3-mal mit einem Substituenten substituiert sind, der aus Halogen, linearem oder verzweigtem C₁-₆-Alkyl, C₃-₆-Cycloalkyl, C₁₋₆-Alkenyl, C₃-₆-Cycloalkenyl, Hydroxy, C₁-₆-Alkoxy, Oxo, Amino, C₁-₆-Alkylmino, C₁-₆-Dialkylamino, C₁-₆-Alkylthio, C₁-₆-Alkylsulfinyl und Phenyl ausgewählt ist;
oder (iii) R¹ und R² zusammen mit dem Stickstoffatom, an das sie gebunden sind, einen 4- bis 8-gliedrigen heterocyclischen Ring oder einen 7- bis 11-gliedrigen bicyclischen heterocyclischen Ring bilden, wobei jeder aus dem 4-8-gliedrigen heterocyclischen Ring und dem 7- bis 11-gliedrigen bicyclischen heterocyclischen Ring 1, 2 oder 3 Heteroatome enthält, die aus der Gruppe ausgewählt sind, die aus N, O und S besteht, und worin entweder der heterocyclische Ring oder der bicyclische heterocyclische Ring gegebenenfalls mit Phenyl, 1 bis 4 C₁-₃-Alkyl, Hydroxy, C₁-₃-Alkoxy, Oxo oder Halogen substituiert sein kann;
oder (iv) R¹ und R² unabhängig entweder an die Gruppe L gebunden sein können oder an die Gruppe M gebunden sein können, worin M aus der Gruppe ausgewählt ist, die aus S(O)₂NR, N-R, C(R)₂, N-C(O)R und N-S(O)₂R besteht, und worin R lineares oder verzweigtes C₁-₆-Alkyl ist, um eine 3- bis 7-gliedrige cyclische Gruppe zu bilden, die gegebenenfalls mit Phenyl, 1 bis 4 C₁-₃-Alkyl, Hydroxy, Alkoxy, Oxo oder Halogen substituiert sein kann.

2. Verbindung gemäß Anspruch 1, worin das Ⓐ ein Aryl ist, das mit einer Gruppe substituiert ist, die aus der Gruppe ausgewählt ist, die aus Halogen, C₁-₃-Alkyl und C₁-₃-Alkoxy besteht.

3. Verbindung gemäß Anspruch 2, worin das Aryl mit einer Gruppe substituiert ist, die aus der Gruppe ausgewählt ist, die aus Fluor, Chlor und Methoxy besteht.

4. Verbindung gemäß Anspruch 3, worin das Aryl mit einer Halogengruppe substituiert ist; q 0 ist; Q¹ C ist; und R⁷ Wasserstoff oder Halogen ist.

5. Verbindung gemäß Anspruch 4, worin das Aryl 4-Chlorphenyl ist; und R⁵ und R⁷ jeweils Wasserstoff sind.

6. Verbindung gemäß Ansprüchen 1 bis 5, worin L C₂-₃-Alkyl oder C₂-₃-Alkenyl ist.

7. Verbindung gemäß Anspruch 6, worin L C₂-₃-Alkyl ist.

8. Verbindung gemäß Ansprüchen 1 bis 7, worin M aus der Gruppe ausgewählt ist, die aus O, S, S(O)₂NR, N-R, N-C(O)R und N-S(O)₂R- besteht, worin R aus der Gruppe ausgewählt ist, die aus Wasserstoff, Phenyl, Heteroaryl, linearem oder verzweigtem C₁-₆-Alkyl und C₃-₆-Cycloalkyl besteht.

9. Verbindung gemäß Anspruch 8, worin R aus der Gruppe ausgewählt ist, die aus Wasserstoff, Phenyl, linearem oder verzweigtem C₁-₆-Alkyl und C₃-₆-Cycloalkyl besteht.

10. Verbindung gemäß Anspruch 9, worin R aus der Gruppe ausgewählt ist, die aus Wasserstoff, linearem oder verzweigtem C₁-₆-Alkyl und C₃-₆-Cycloalkyl besteht.

11. Verbindung gemäß Ansprüchen 1 bis 10, worin R⁵ Wasserstoff oder C₁-₃-Alkyl ist.

12. Verbindung gemäß Anspruch 11, worin R⁵ Wasserstoff oder Methyl ist.

13. Verbindung gemäß Ansprüchen 1 bis 12, worin in (i) oben R¹ und R² aus der Gruppe ausgewählt sind, die aus Wasserstoff, linearem oder verzweigtem C₁-₆-Alkyl und C₃-₆-Cycloalkyl besteht.

14. Verbindung gemäß Anspruch 13, worin R¹ und R² in (i) aus der Gruppe ausgewählt sind, die aus Wasserstoff, C₁-₃-Alkyl und C₃-₆-Cycloalkyl besteht.

15. Verbindung gemäß Ansprüchen 1 bis 12, worin in (iii) oben R¹ und R² zusammen mit dem Stickstoffatom, an das sie gebunden sind, einen 5- oder 6-gliedrigen heterocyclischen Ring oder einen 8- bis 11-gliedrigen bicyclischen heterocyclischen Ring mit 1 oder 2 Heteroatomen bilden, die aus der Gruppe N, O und S ausgewählt sind, worin der heterocyclische Ring und der bicyclische heterocyclische Ring gegebenenfalls bis zu 2-mal mit einem Substituenten substituiert sein können, der aus der Gruppe ausgewählt ist, die aus Oxo und Halogen besteht.

16. Verbindung gemäß Ansprüchen 1 bis 12, worin in (iv) R¹ und R² unabhängig an die Gruppe M gebunden sein können, wenn M aus der Gruppe ausgewählt ist, die aus S(O)₂NR, N-R, C(R)₂, N-C(O)R und N-S(O)₂R besteht, und worin R lineares oder verzweigtes C₁-₆-Alkyl ist, um eine 5- bis 7-gliedrige cyclische Gruppe zu bilden, die gegebenenfalls mit Phenyl, 1 bis 4 C₁-₃-Alkyl, Hydroxy, Alkoxy, Oxo oder Halogen substituiert sein kann.

17. Verbindung gemäß Anspruch 1, worin L C₂-₃-Alkyl oder C₂-₃-Alkenyl ist;
in (i) R¹ und R² aus der Gruppe ausgewählt sind, die aus Wasserstoff, linearem oder verzweigtem C₁-₃-Alkyl und C₃-₆-Cycloalkyl, das mit einem Substituenten substituiert ist, der aus der Gruppe ausgewählt ist, die aus Halogen, Alkyl, Hydroxy, Oxo und Alkoxy besteht, besteht; oder in (iii) R¹ und R² zusammen mit dem Stickstoffatom, an das sie gebunden sind, einen 4- bis 6-gliedrigen heterocyclischen Ring bilden, worin der heterocyclische Ring gegebenenfalls mit einem Substituenten substituiert ist, der aus der Gruppe ausgewählt ist, die aus 1 bis 4 C₁-₃-Alkyl, Hydroxy, Alkoxy, Oxo und Halogen besteht.

18. Verbindung gemäß Anspruch 17, worin L C₂-₃-Alkyl ist;
in (i) R¹ und R² jeweils unabhängig aus der Gruppe ausgewählt sind, die aus Wasserstoff und C₃-₆-Cycloalkyl, das mit einem Substituenten substituiert ist, der aus der Gruppe ausgewählt ist, die aus Oxo und Halogen besteht, besteht;
oder in (iii) R¹ und R² zusammen mit dem Stickstoffatom, an das sie gebunden sind, einen 5- oder 6-gliedrigen heterocyclischen Ring bilden, worin der heterocyclische Ring gegebenenfalls mit einem Substituenten substituiert ist, der aus der Gruppe ausgewählt ist, die aus 1 bis 2 Oxo und Halogen besteht.

19. Verbindung gemäß Anspruch 18, worin L CH₂CH₂ ist und in (iii) R¹ und R² zusammen mit dem Stickstoffatom, an das sie gebunden sind, einen Pyrrolidinring bilden, der in der 3-Stellung mit einem Fluoratom substituiert ist.

20. Verbindung gemäß Anspruch 1, worin M O, N-R oder N-C(O)R ist, worin R Wasserstoff oder lineares oder verzweigtes C₁-₆-Alkyl ist und R⁶ aus der Gruppe ausgewählt ist, die aus Wasserstoff, linearem oder verzweigtem C₁-₆-Alkyl, C₁-₃-Alkoxy, Trihalogenalkyl, Trihalogenalkoxy, Cyano und Halogen besteht.

21. Verbindung gemäß Anspruch 20, worin M O oder N-R ist,
worin R Wasserstoff ist und R⁶ aus der Gruppe ausgewählt ist, die aus Wasserstoff, linearem oder verzweigtem C₁-₂-Alkyl, C₁-₂-Alkoxy oder Halogen besteht.

22. Verbindung gemäß Anspruch 21, worin M O ist und R⁶ Methoxy ist.

23. Verbindung gemäß Anspruch 1, worin die Verbindung aus der Gruppe ausgewählt ist, die aus 6-(4-Chlorphenyl)-3-(3-methoxy-4-[2-(3-oxopyrrolidin-1-yl)ethoxy]phenyl}thieno[3,2-d]pyrimidin-4(3H)-on und 6-(4-Chlorphenyl)-3-{4-[2-(3-fluorpyrrolidin-1-yl)ethoxy]-3-methoxyphenyl}thieno[3,2-d]pyrimidin-4(3H)-on besteht.

24. Verfahren zur Herstellung einer Verbindung gemäß Anspruch 1, umfassend das Umsetzen eines Anilins der Formel (II): 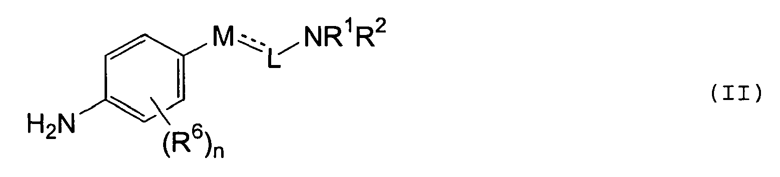 mit einer Verbindung der Formel (III): 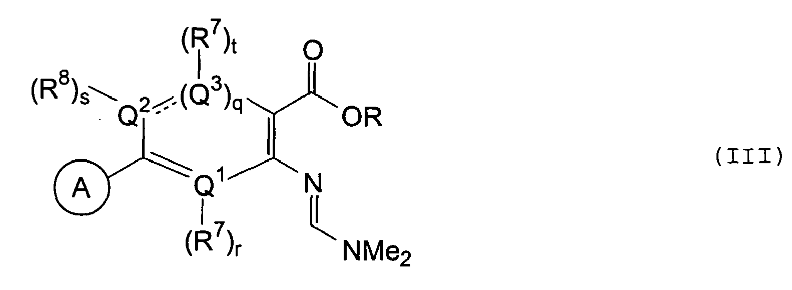 unter Erwärmen in einem Lösungsmittel; worin Ⓐ, R⁸, R⁷, R⁶, R⁵, R², R¹, M, L, Q¹, Q², Q³, q, r, s, t und n wie in Formel (Ia) definiert sind; und Me Methyl ist.

25. Verfahren zur Herstellung einer Verbindung gemäß Ansprüchen 1 bis 23 umfassend das Kuppeln einer Aminosäure der Formel (IV): 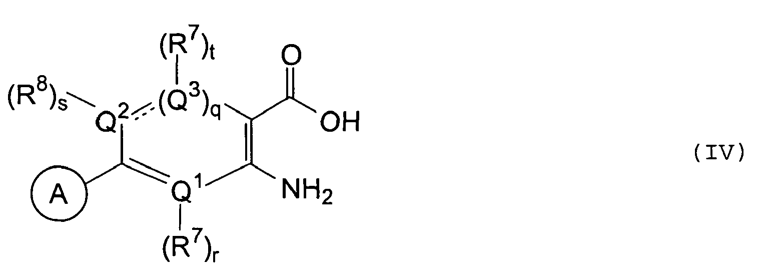 mit einem Anilin der Formel (II): 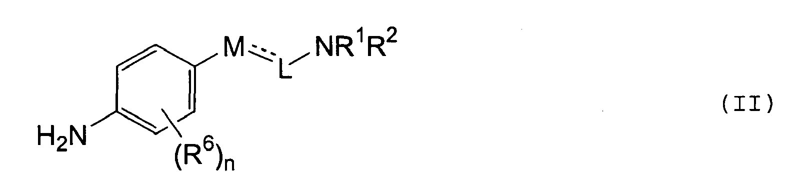 in einem Lösungsmittel in Gegenwart wenigstens eines Kupplungsmittels zur Herstellung einer Verbindung der Formel (V): 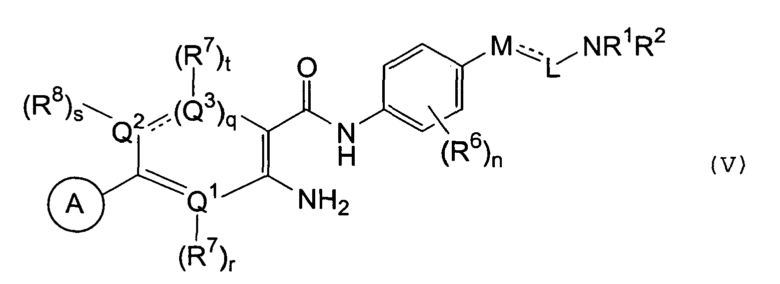 und Cyclisieren der Verbindung der Formel (V) unter Bildung einer Verbindung der Formel (Ia),
worin Ⓐ, R⁸, R⁷, R⁶, R⁵, R², R¹, M, L, Q¹, Q², Q³, q, r, s, t und n wie in Formel (Ia) definiert sind.

26. Verfahren zur Herstellung einer Verbindung gemäß Ansprüchen 1 bis 23, umfassend die Reaktion einer Verbindung der Formel (Va): 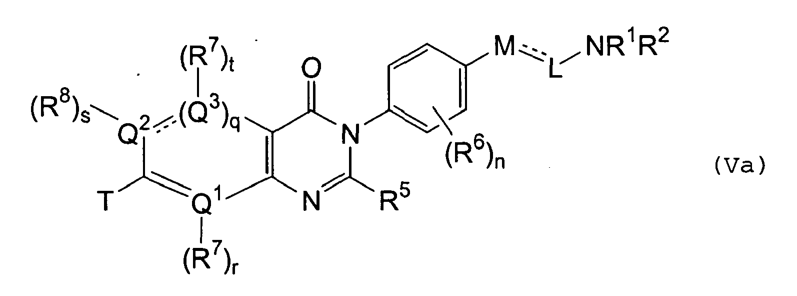 mit einer Verbindung, die die Gruppe Ⓐ einführen kann,
worin Ⓐ, R⁸, R⁷, R⁶, R⁵, R², R¹, M, L, Q¹, Q², Q³, q, r, s, t und n wie in Formel (Ia) definiert sind und T eine Abgangsgruppe ist.

27. Verfahren zur Herstellung einer Verbindung gemäß Ansprüchen 1 bis 23, umfassend die Reaktion einer Verbindung der Formel (Va): 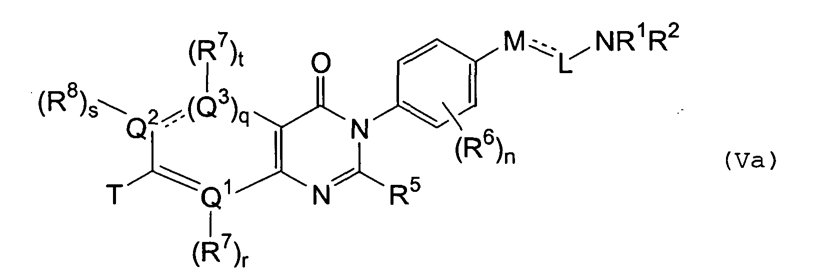 mit einer Boronsäure und einem Palladiumkatalysator unter Verwendung einer Suzuki-Kupplungsreaktion oder mit einem Organostannanreagens und einem Palladiumkatalysator unter Verwendung einer Stille-Kupplungsreaktion, worin Ⓐ, R⁸, R⁷, R⁶, R⁵, R², R¹, M, L, Q¹, Q², Q³, q, r, s, t und n wie in Formel (Ia) definiert sind und T eine Abgangsgruppe ist.

28. Verfahren zur Herstellung einer Verbindung gemäß Anspruch 1, worin R⁵ Wasserstoff ist, umfassend das Umsetzen einer schwefelhaltigen Verbindung der Formel 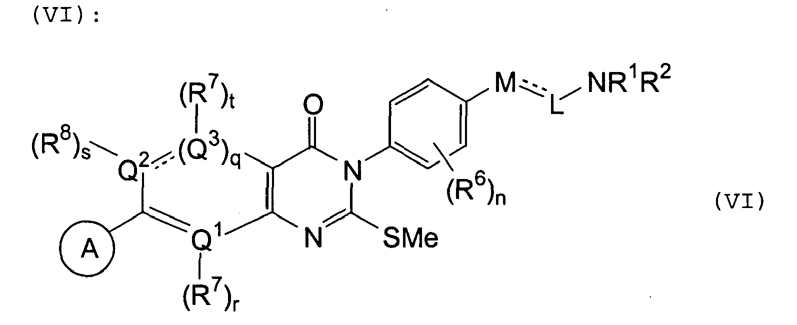 mit einem Raney-Nickel-Reduktionsmittel in Gegenwart eines Lösungsmittels, worin Ⓐ, R⁸, R⁷, R⁶, R⁵, R², R¹, M, L, Q¹, Q², Q³, q, r, s, t und n wie in Formel (Ia) definiert sind.

29. Verfahren zur Herstellung einer Verbindung gemäß Ansprüchen 1 bis 23, umfassend die Alkylierung eines Amins der Formel (XV) :
H-NR¹R² (XV)
mit einem Alkylierungsmittel der Formel (XIV): 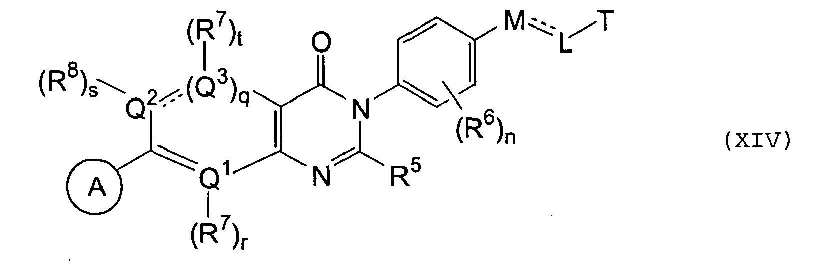 worin M O ist, T eine Abgangsgruppe ist und Ⓐ, R⁸, R⁷, R⁶, R⁵, R², R¹, M, L, Q¹, Q², Q³, q, r, s, t und n wi in Formel (Ia) definiert sind.

30. Verfahren zur Herstellung einer Verbindung gemäß Anspruch 1, worin M N(CO)R ist, umfassend die Acylierung eines Anilins der Formel (XVI): 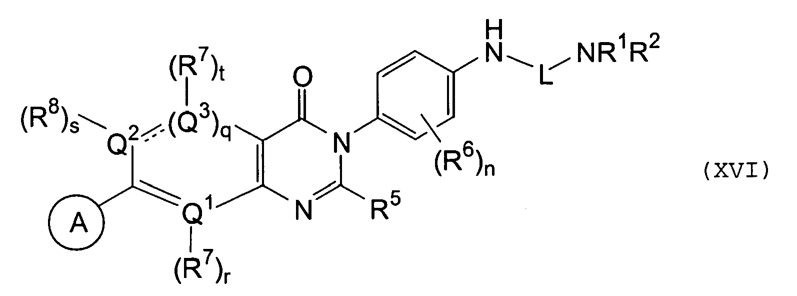 mit einem Acylierungsmittel der Formel (XVII): 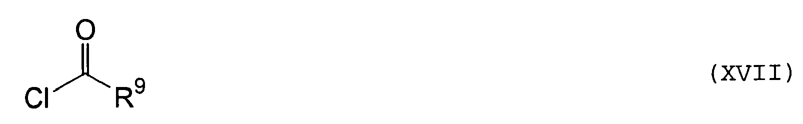 worin Ⓐ, R⁸, R⁷, R⁶, R⁵, R², R¹, M, L, Q¹, Q², Q³, q, r, s, t und n wie in Formel (Ia) definiert sind und R⁹ aus der Gruppe ausgewählt ist, die aus Wasserstoff, Phenyl, Heteroaryl, linearem oder verzweigtem C₁-₆-Alkyl und C₃-₆-Cycloalkyl besteht.

31. Verfahren zur Herstellung einer Verbindung gemäß Anspruch 1, worin M N ist, umfassend die reduktive Alkylierung eines Anilins der Formel (XIX): 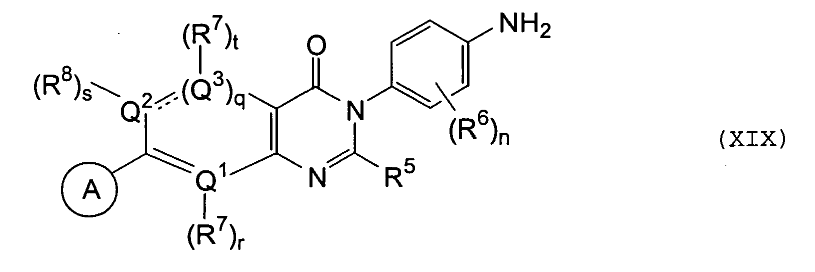 mit einem Aldehyd der Formel (XVIII): 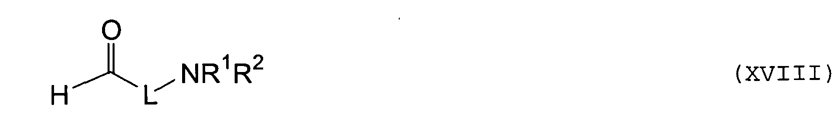 in Gegenwart eines Borhydrid-Reduktionsmittels oder von Wasserstoff und eines Katalysators, worin das L der Formel (XVIII) CH₂ oder CH₂CH₂ ist und worin Ⓐ, R⁸, R⁷, R⁶, R⁵, R², R¹, M, Q¹, Q², Q³, q, r, s, t und n wie in Formel (Ia) definiert sind.

32. Verfahren zur Herstellung einer Verbindung gemäß Anspruch 1, worin M O ist, umfassend die Alkylierung eines Phenols der Formel (XX): 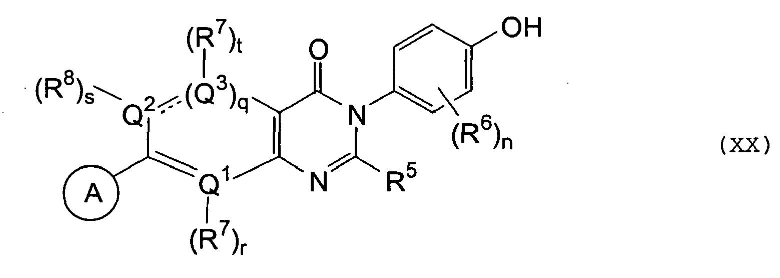 mit einem Alkylierungsmittel der Formel (XXI): 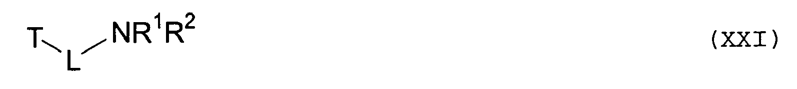 worin T eine Abgangsgruppe ist und Ⓐ, R⁸, R⁷, R⁶, R⁵, R², R¹, M, L, Q¹, Q², Q³, q, r, s, t und n wie in Formel (Ia) definiert sind.

33. Verfahren zur Herstellung einer Verbindung gemäß Anspruch 1, worin M O ist, umfassend die reduktive Aminierung eines Aldehyds der Formel (XXII): 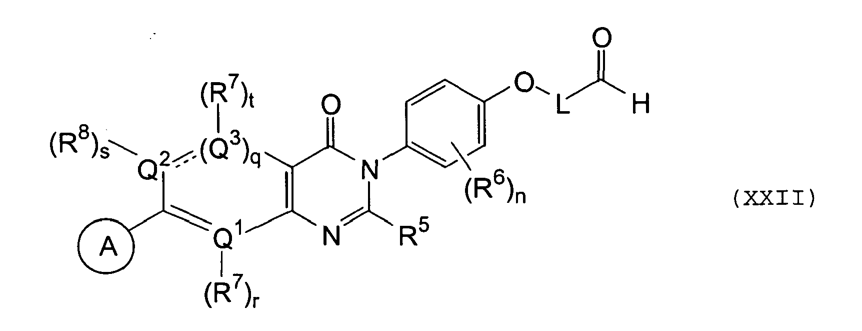 durch ein Amin der Formel (XV):
H-NR¹R² (XV)
in Gegenwart eines Reduktionsmittels und eines Katalysators, worin L CH₂ oder CH₂CH₂ ist und Ⓐ, R⁸, R⁷, R⁶, R⁵, R², R¹, M, Q¹, Q², Q³, q, r, s, t und n wie in Formel (Ia) definiert sind.

34. Verfahren zur Herstellung einer Verbindung gemäß Anspruch 1, worin M O ist, umfassend die reduktive Alkylierung eines Amins der Formel (XXV) : 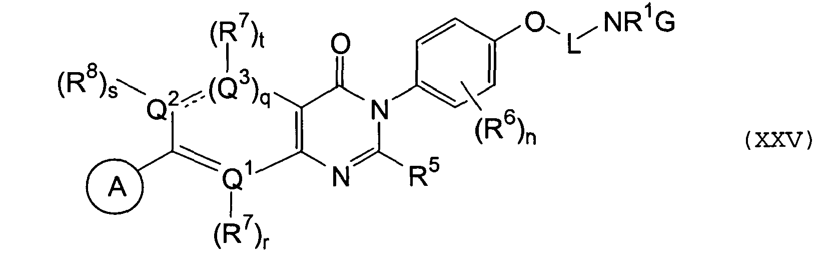 mit einem Aldehyd, worin für Formel (XXV) G H ist und Ⓐ, R⁸, R⁷, R⁶, R⁵, R², R¹, M, L, Q¹, Q², Q³, q, r, s, t und n wie in Formel (Ia) definiert sind.

35. Verfahren zur Herstellung einer Verbindung gemäß Anspruch 1, worin L -C(O)CH₂- ist, umfassend die Reaktion eines Amins der Formel (XV):
H-NR¹R² (XV)
mit einem Alkylierungsmittel der Formel (XXVIII): 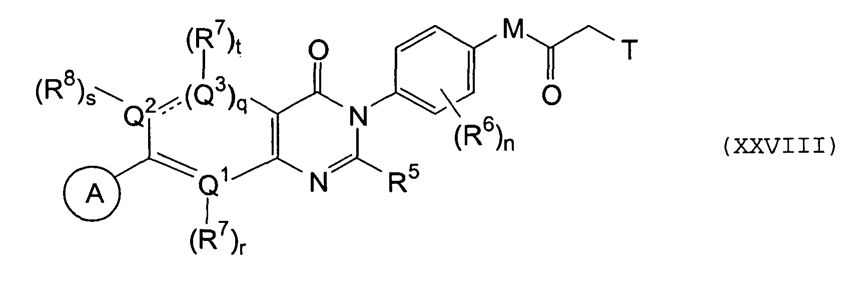 worin T eine Abgangsgruppe ist und Ⓐ, R⁸, R⁷, R⁶, R⁵, R², R¹, M, L, Q¹, Q², Q³, q, r, s, t und n wie in Formel (Ia) definiert sind.

36. Verwendung einer Verbindung gemäß Ansprüchen 1 bis 23 oder eines Salzes oder Solvats davon zur Herstellung eines Medikaments zur Behandlung von Fettsucht, Diabetes, Depression oder Angstzustand in einem Säugetier.

37. Verwendung gemäß Anspruch 36, worin das Säugetier ein Mensch ist.

38. Verbindung gemäß Ansprüchen 1 bis 23, Salz oder Solvat davon in Kombination mit wenigstens einer Spezies, die aus der Gruppe ausgewählt ist, die aus einem Mittel zur Behandlung von Diabetes, einem Mittel zur Behandlung von Hypertonie und einem Mittel zur Behandlung von Arteriosklerose besteht.

39. Pharmazeutische Zusammensetzung, die eine Verbindung gemäß Ansprüchen 1 bis 23 oder ein Salz oder Solvat davon umfaßt.

40. Pharmazeutische Zusammensetzung gemäß Anspruch 39, die ferner wenigstens eine pharmazeutisch akzeptable Komponente umfaßt, die aus der Gruppe ausgewählt ist, die aus einem Träger, einem Verdünnungsmittel, einem Exzipienten und einer Mischung daraus besteht.

41. Verbindung gemäß Ansprüchen 1 bis 23 oder Salz oder Solvat davon zur Verwendung in der Behandlung von Diabetes und Fettsucht.

## Revendications

1. Composé de formule (la) comprenant : 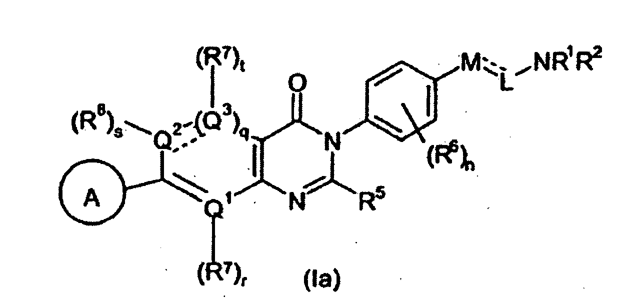 un sel ou solvate pharmaceutiquement acceptable de celui-ci, dans lequel :
Ⓐ est un aryle ou un hétéroaryle, facultativement substitué par un à quatre groupes alkyle linéaire ou ramifié en C₁₋₆, alcényle, halogéno, amino, alkylamino, dialkylamino, hydroxy, alcoxy en C₁₋₆, cyano ou alkylthio ;
lorsque q est 1, le trait tireté est une double liaison;
Q¹, Q², et Q³ sont C ; et q, r, s, et t sont 1 ;
ou
Q¹ est N ; Q² est S et q, r, et s sont 0 ;
ou
Q¹ est C ; Q² est S ; q et s sont 0 ; et r est 1 ;
chaque R⁷ correspondant est indépendamment choisi dans le groupe constitué de l'hydrogène, un alkyle linéaire ou ramifié en C₁₋₆, un cycloalkyle en C₃₋₆, un alcoxy en C₁₋₆, un amino, un alkylamino en C₁₋₆, un dialkylamino en C₁₋₆, un hydroxy, un cyano, un alkylthio en C₁₋₆, et un halogéno ;
R⁵ est choisi dans le groupe constitué de l'hydrogène, un alkyle linéaire ou ramifié en C₁₋₆, un cycloalkyle en C₃₋₆ et un alkylthio en C₁₋₃ ;
R⁶ est choisi dans le groupe constitué de l'hydrogène et le méthoxy et n est 1 ;
M est choisi dans le groupe constitué de O, S, S(O)₂, S(O)₂NR, N-R, C(O), C(R)₂, N-C(O)R, et N-S(O)₂R ;
dans lequel R est choisi dans le groupe constitué de l'hydrogène, le phényle, un hétéroaryle, un alkyle linéaire ou ramifié en C₁₋₆ et un cycloalkyle en C₃₋₆ ;
L est un alkyle en C₂₋₃, un alcényle en C₂₋₃, ou -C(O)(CH₂)- ;
(i) R¹ et R² chacun indépendamment sont choisis dans le groupe constitué de l'hydrogène, un alkyle linéaire ou ramifié en C₁₋₆, un cycloalkyle en C₃₋₆, et un hétérocycle de 5 ou 6 chaînons dans lequel ledit alkyle, ledit cycloalkyle et ledit hétérocycle sont facultativement substitués par un phényle, un à quatre alkyle en C₁₋₃, hydroxy, oxo, alcoxy ou halogéno ;
ou (ii) R¹ et R² peuvent être choisis dans le groupe constitué d'un aryle et d'un hétéroaryle de 5 ou 6 chaînons contenant 1, 2 ou 3 hétéroatomes choisis parmi N, O, et S, dans lequel ledit aryle et ledit hétéroaryle sont facultativement substitués 1, 2, ou 3 fois avec un substituant choisi parmi un halogéno, un alkyle linéaire ou ramifié en C₁₋₆, un cycloalkyle en C₃₋₆, un alcényle en C₁₋₆, un cycloalcényle en C₃₋₆, un hydroxy, un alcoxy en C₁₋₆, l'oxo, l'amino, un alkylamino en C₁₋₆, un dialkylamino en C₁₋₆, un alkylthio en C₁₋₆, un alkylsulfinyle en C₁₋₆, et un phényle ;
ou (iii) R¹ et R² conjointement avec l'atome d'azote auquel ils sont liés forment un anneau hétérocyclique de 4 à 8 chaînons ou un anneau hétérocyclique bicyclique de 7 à 11 chaînons, chacun parmi ledit anneau hétérocyclique de 4 à 8 chaînons et ledit anneau hétérocyclique bicyclique de 7 à 11 chaînons contiennent 1, 2 ou 3 hétéroatomes choisis dans le groupe constitué de N, O, et S, et dans lequel ledit anneau hétérocyclique ou ledit anneau hétérocyclique bicyclique peuvent être facultativement substitués par un phényle, un à quatre alkyle en C₁₋₃, hydroxy, alcoxy en C₁₋₃, oxo ou halogéno ;
ou (iv) R¹ et R² peuvent être indépendamment liés au groupe L ou liés au groupe M lorsque M est choisi dans le groupe constitué de S(O)₂NR, N-R, C(R)₂, N-C(O)R, et N-S(O)₂R, et dans lequel R est un alkyle linéaire ou ramifié en C₁₋₆, pour former un groupe cyclique de 3 à 7 chaînons qui peut être facultativement substitué par un phényle, un à quatre alkyle en C₁₋₃, hydroxy, alcoxy, oxo, ou halogéno.

2. Composé selon la revendication 1 dans lequel ledit Ⓐ est un aryle substitué avec un groupe choisi dans le groupe constitué d'un halogéno, un alkyle en C₁₋₃, et un alcoxy en C₁₋₃.

3. Composé selon la revendication 2, dans lequel ledit aryle est substitué avec un groupe choisi dans le groupe constitué du fluoro, du chloro et du méthoxy.

4. Composé selon la revendication 3, dans lequel ledit aryle est substitué avec un groupe halogéno ; q est 0 ; Q¹ est C ; et R⁷ est l'hydrogène ou un halogéno.

5. Composé selon la revendication 4, dans lequel ledit aryle est le 4-chlorophényle ; R⁵ et R⁷ sont chacun l'hydrogène.

6. Composé selon les revendications 1 à 5, dans lequel L est un alkyle en C₂₋₃ ou un alcényle en C₂₋₃.

7. Composé selon la revendication 6, dans lequel L est un alkyle en C₂₋₃.

8. Composé selon les revendications 1 à 7, dans lequel M est choisi dans le groupe constitué de O, S, S(O)₂NR, N-R, N-C(O)R, et N-S(O)₂R- dans lequel R est choisi dans le groupe constitué de l'hydrogène, le phényle, un hétéroaryle, un alkyle linéaire ou ramifié en C₁₋₆ et un cycloalkyle en C₃₋₆.

9. Composé selon la revendication 8, dans lequel R est choisi dans le groupe constitué de l'hydrogène, le phényle, un alkyle linéaire ou ramifié en C₁₋₆ et un cycloalkyle en C₃₋₆.

10. Composé selon la revendication 9, dans lequel R est choisi dans le groupe constitué de l'hydrogène, un alkyle linéaire ou ramifié en C₁₋₆ et un cycloalkyle en C₃₋₆.

11. Composé selon les revendications 1 à 10, dans lequel R⁵ est l'hydrogène ou un alkyle en C₁₋₃.

12. Composé selon la revendication 11, dans lequel R⁵ est l'hydrogène ou le méthyle.

13. Composé selon les revendications 1 à 12, dans lequel dans (i) ci-dessus R¹ et R² sont choisis dans le groupe constitué de l'hydrogène, un alkyle linéaire ou ramifié en C₁₋₆, et un cycloalkyle en C₃₋₆.

14. Composé selon la revendication 13, dans lequel R¹ et R² dans (i) sont choisis dans le groupe constitué de l'hydrogène, un alkyle en C₁₋₃ et un cycloalkyle en C₃₋₆.

15. Composé selon les revendications 1 à 12, dans lequel dans (iii) ci-dessus R¹ et R² conjointement avec l'atome d'azote auquel ils sont liés forment un anneau hétérocyclique de 5
ou 6 chaînons ou un anneau hétérocyclique bicyclique de 8 à 11 chaînons ayant 1 ou 2 hétéroatomes choisis dans le groupe constitué de N, O, et S, dans lequel ledit anneau hétérocyclique et ledit anneau bicyclique hétérocyclique peuvent être facultativement substitués jusqu'à deux fois avec un substituant choisi dans le groupe constitué de l'oxo et d'un halogéno.

16. Composé selon les revendications 1 à 12, dans lequel dans (iv) R¹ et R² peuvent être indépendamment liés au groupe M lorsque M est choisi dans le groupe constitué de S(O)₂NR, N-R, C(R)₂, N-C(O)R, et N-S(O)₂R, et dans lequel R est un alkyle linéaire ou ramifié en C₁₋₆, pour former un groupe cyclique de 5 à 7 chaînons qui peut être facultativement substitué par un phényle, un à quatre alkyle en C₁₋₃, hydroxy, alcoxy, oxo, ou halogéno.

17. Composé selon la revendication 1, dans lequel L est un alkyle en C₂₋₃ ou un alcényle en C₂₋₃ ;
dans (i) R¹ et R² sont choisis dans le groupe constitué de l'hydrogène, un alkyle linéaire ou ramifié en C₁₋₃, un cycloalkyle en C₃₋₆ substitué avec un substituant choisi dans le groupe constitué d'un halogéno, un alkyle, un hydroxy, un oxo, et un alcoxy ; ou
dans (iii) R¹ et R² conjointement avec l'atome d'azote auquel ils sont liés forment un anneau hétérocyclique de 4 à 6 chaînons dans lequel ledit anneau hétérocyclique est facultativement substitué avec un substituant choisi dans le groupe constitué d'un à quatre alkyle en C₁₋₃, hydroxy, alcoxy, oxo et halogéno.

18. Composé selon la revendication 17, dans lequel L est un alkyle en C₂₋₃;
dans (i) R¹ et R² sont chacun indépendamment choisis dans le groupe constitué de l'hydrogène et d'un cycloalkyle en C₃₋₆ substitué avec un substituant choisi dans le groupe constitué de l'oxo et d'un halogéno ;
ou dans (iii) R¹ et R² conjointement avec l'atome d'azote auquel ils sont liés forment un anneau hétérocyclique de 5 ou 6 chaînons dans lequel ledit anneau hétérocyclique est facultativement substitué avec un substituant choisi dans le groupe constitué d'un à deux oxo et halogéno.

19. Composé selon la revendication 18, dans lequel L est CH₂CH₂ et dans iii) R¹ et R² conjointement avec l'atome d'azote auquel ils sont liés forment un anneau pyrrolidine substitué à la position 3 avec un atome de fluor.

20. Composé selon la revendication 1, dans lequel M est O, N-R ou N-C(O)R où R est l'hydrogène ou un alkyle linéaire ou ramifié en C₁₋₆ et R⁶ est choisi dans le groupe constitué de l'hydrogène, un alkyle linéaire ou ramifié en C₁₋₆, un alcoxy en C₁₋₃, un trihalogénoalkyle, un trihalogénoalcoxy, le cyano, et un halogéno.

21. Composé selon la revendication 20, dans lequel M est O ou N-R où R est l'hydrogène et R⁶ est choisi dans le groupe constitué de l'hydrogène, un alkyle linéaire ou ramifié en C₁₋₂, un alcoxy en C₁₋₂, ou un halogéno.

22. Composé selon la revendication 21, dans lequel M est O et R⁶ est le méthoxy.

23. Composé selon la revendication 1, dans lequel le composé est choisi dans le groupe constitué de la 6-(4-chlorophényl)-3-{3-méthoxy-4-[2-(3-oxopyrrolidin-1-yl)éthoxy]phényl}thiéno[3,2-*d*]pyrimidin-4(3*H*)-one et la 6-(4-chlorophényl)-3-{4-[2-(3-fluoropyrrolidin-1-yl)éthoxy]-3-méthoxyphényl}thiéno[3,2-d]pyrimidin-4(3H)-one.

24. Procédé pour préparer un composé selon la revendication 1 comprenant la réaction d'une aniline de formule (II) 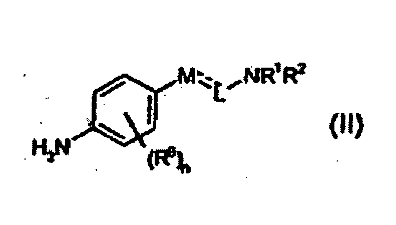 avec un composé de formule (III) 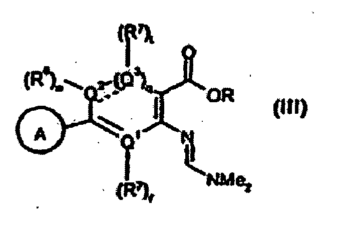 sous chauffage dans un solvant ; dans lequel Ⓐ, R⁸, R⁷, R⁶, R⁵, R², R¹, M, L, Q¹, Q², Q³, q, r, s, t, et n sont comme définis dans la formule (Ia); et Me est le méthyle.

25. Procédé pour préparer un composé selon les revendications 1 à 23 comprenant le couplage d'un aminoacide de formule (IV) 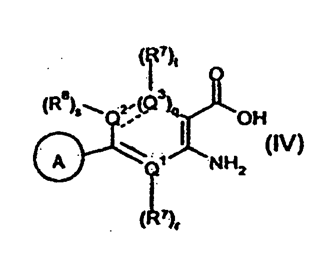 avec une aniline de formule (II) 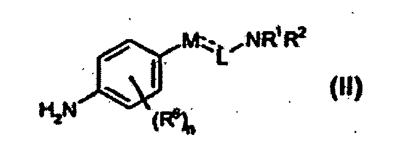 dans un solvant en présence d'au moins un agent de couplage pour produire un composé de formule (V) 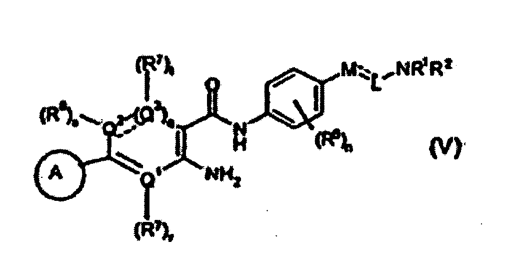 et la cyclisation dudit composé de formule (V) pour former un composé de formule (Ia) et dans lequel Ⓐ, R⁸ R⁷, R⁶, R⁵, R², R¹, M, L, Q¹, Q², Q³, q, r, s, t, et n sont comme définis dans la formule (Ia).

26. Procédé pour préparer un composé selon les revendications 1 à 23 comprenant la réaction d'un composé de formule (Va) 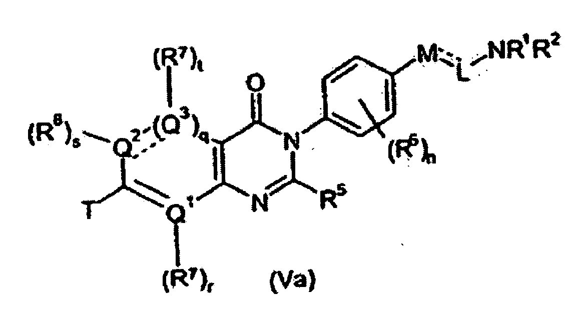 avec un composé capable d'introduire le groupe Ⓐ, et dans lequel Ⓐ, R⁸, R⁷, R⁶, R⁵, R², R¹, M, L, Q¹, Q², Q³, q, r, s, t, et n sont comme définis dans la formule (Ia) et T est un groupe labile.

27. Procédé pour préparer un composé selon les revendications 1 à 23 comprenant la réaction d'un composé de formule (Va) 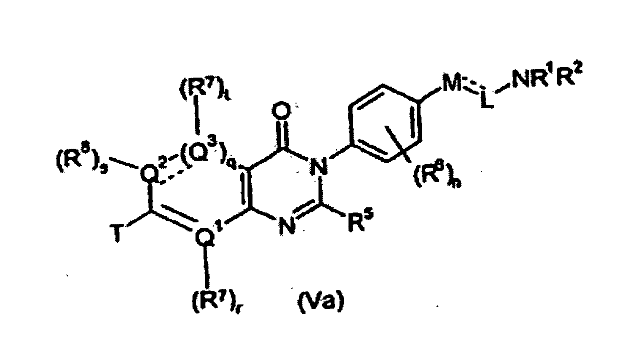 avec un acide borique et un catalyseur au palladium en utilisant une réaction de couplage de Suzuki ou avec un réactif d'organostannane et un catalyseur au palladium en utilisant une réaction de couplage de Stille et dans lequel Ⓐ, R⁸, R⁷, R⁶, R⁵, R², R¹, M, L, Q¹, Q², Q³, q, r, s, t, et n sont comme définis dans la formule (Ia) et T est un groupe labile.

28. Procédé pour préparer un composé selon la revendication 1, dans lequel R⁵ est l'hydrogène comprenant la réaction d'un composé soufré de formule (VI) 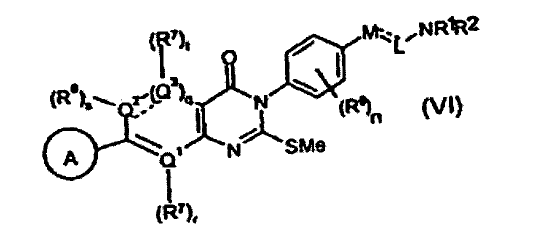 avec un réducteur au nickel de Raney en présence d'un solvant et dans lequel Ⓐ, R⁸, R⁷, R⁶, R⁵, R², R¹, M, L, Q¹, Q², Q³, q, r, s, t, et n sont comme définis dans la formule (Ia).

29. Procédé pour préparer un composé selon les revendications 1 à 23 comprenant l'alkylation d'une amine de formule (XV)
H-NR¹R² (XV)
avec un agent d'alkylation de formule (XIV) 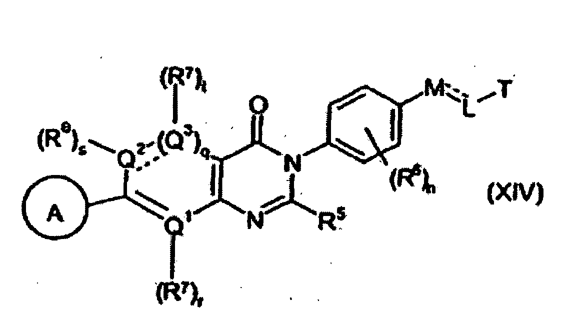 dans lequel M est O, T est un groupe labile, et dans lequel Ⓐ, R⁸, R⁷, R⁶, R⁵, R², R¹, M, L, Q¹, Q², Q³, q, r, s, t, et n sont comme définis dans la formule (Ia).

30. Procédé pour préparer un composé selon la revendication 1, dans lequel M est N(CO)R comprenant l'acylation d'une aniline de formule (XVI) 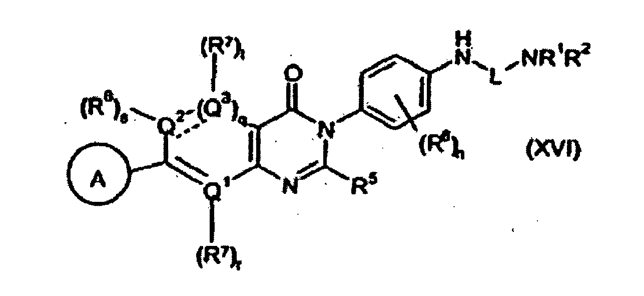 avec un agent d'acylation de formule (XVII) 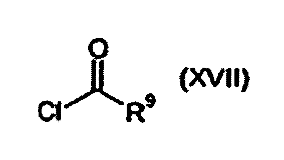 et dans lequel Ⓐ, R⁸, R⁷, R⁶, R⁵, R², R¹, M, L, Q¹, Q², Q³, q, r, s, t, et n sont comme définis dans la formule (Ia) et R⁹ est choisi dans le groupe constitué de l'hydrogène, le phényle, un hétéroaryle, un alkyle linéaire ou ramifié en C₁₋₆, et un cycloalkyle en C₃₋₆.

31. Procédé pour préparer un composé selon la revendication 1 dans lequel M est N comprenant l'alkylation réductrice d'une aniline de formule (XIX) 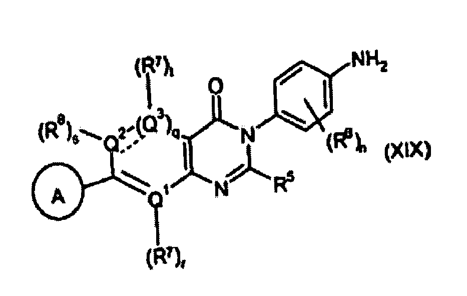 par un aldéhyde de formule (XVIII) 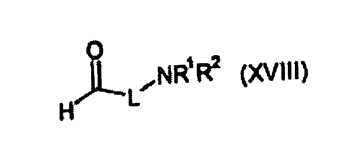 en présence d'un agent réducteur au borohydrure ou l'hydrogène et un catalyseur et dans lequel le L de la formule (XVIII) est CH₂ ou CH₂CH₂, et dans lequel Ⓐ, R⁸, R⁷, R⁶, R⁵, R², R¹, M, L, Q¹, Q², Q³, q, r, s, t, et n sont comme définis dans la formule (Ia).

32. Procédé pour préparer un composé selon la revendication 1, dans lequel M est O comprenant l'alkylation d'un phénol de formule (XX) 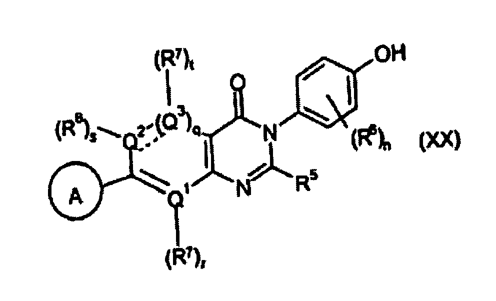 avec un agent d'alkylation de formule (XXI) 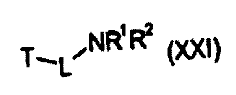 dans lequel T est un groupe labile et dans lequel Ⓐ, R⁸, R⁷, R⁶, R⁵, R², R¹, M, L, Q¹, Q², Q³, q, r, s, t, et n sont comme définis dans la formule (Ia).

33. Procédé pour préparer un composé selon la revendication 1, dans lequel M est O comprenant l'amination réductrice d'un aldéhyde de formule (XXII) 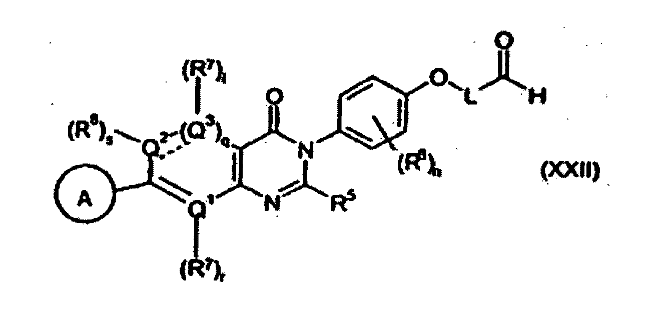 par une amine de formule (XV)
H-NR¹R² (XV)
en présence d'un agent réducteur et d'un catalyseur et dans lequel L est CH₂ ou CH₂CH₂ et Ⓐ, R⁸, R⁷, R⁶, R⁵, R², R¹, M, L, Q¹, Q², Q³, q, r, s, t, et n sont comme définis dans la formule (Ia).

34. Procédé pour préparer un composé selon la revendication 1, dans lequel M est O comprenant l'alkylation réductrice d'une amine de formule (XXV) 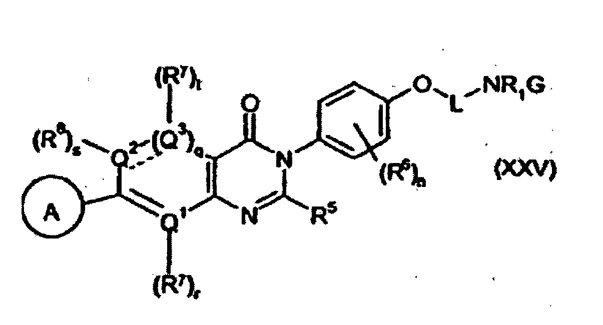 avec un aldéhyde et dans lequel pour la formule (XXV) G est H et Ⓐ, R⁸, R⁷, R⁶, R⁵, R², R¹, M, L, Q¹, Q², Q³, q, r, s, t, et n sont comme définis dans la formule (Ia).

35. Procédé pour préparer un composé selon la revendication 1, dans lequel L est -C(O)CH₂- comprenant la réaction d'une amine de formule (XV)
H-NR¹ R² (XV)
avec un agent d'alkylation de formule (XXVIII) 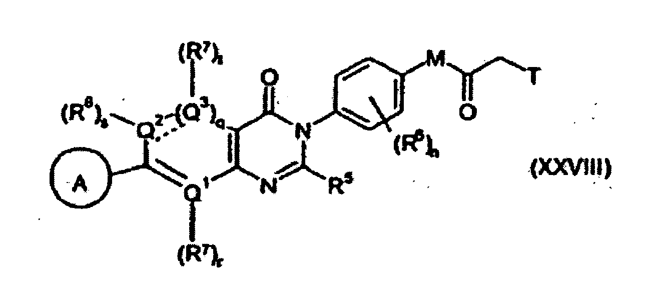 dans lequel T est un groupe labile, et dans lequel Ⓐ, R⁸, R⁷, R⁶, R⁵, R², R¹, M, L, Q¹, Q², Q³, q, r, s, t, et n sont comme définis dans la formule (Ia).

36. Utilisation d'un composé selon les revendications 1 à 23 ou d'un sel ou solvate de celui-ci pour la fabrication d'un médicament pour le traitement de l'obésité, le diabète, la dépression, ou l'anxiété chez un mammifère.

37. Utilisation selon la revendication 36, dans laquelle ledit mammifère est un humain.

38. Composé selon les revendications 1 à 23, sel ou solvate de celui-ci en combinaison avec au moins une espèce choisie dans le groupe constitué d'un agent pour traiter le diabète, un agent pour traiter l'hypertension, et un agent pour traiter l'artériosclérose.

39. Composition pharmaceutique comprenant un composé des revendications 1 à 23 ou un sel ou solvate de celui-ci.

40. Composition pharmaceutique selon la revendication 39 comprenant en outre au moins un composant pharmaceutiquement acceptable choisi dans le groupe constitué d'un véhicule, un diluant, un excipient, et un mélange de ceux-ci.

41. Composé selon les revendications 1 à 23 ou sel ou solvate de celui-ci pour utilisation dans le traitement du diabète et pour l'obésité.
